(19) Europäisches Patentamt
European Patent Office
Office européen des brevets

(11) EP 2 914 583 B1

(12) **EUROPEAN PATENT SPECIFICATION**

(45) Date of publication and mention of the grant of the patent:
**27.02.2019 Bulletin 2019/09**

(21) Application number: **12810437.9**

(22) Date of filing: **30.10.2012**

(51) Int Cl.:
***C07D 233/84*** *(2006.01)* ***C07D 239/38*** *(2006.01)*
***C07D 277/74*** *(2006.01)* ***C07D 513/04*** *(2006.01)*
***C07D 295/096*** *(2006.01)* ***C07C 311/29*** *(2006.01)*
***C07C 311/39*** *(2006.01)* ***C07C 317/14*** *(2006.01)*
***C07C 317/18*** *(2006.01)* ***C07C 317/36*** *(2006.01)*
***C07C 323/67*** *(2006.01)* ***A61K 31/18*** *(2006.01)*
***A61K 31/33*** *(2006.01)* ***A61P 25/00*** *(2006.01)*
***A61P 27/06*** *(2006.01)*

(86) International application number:
**PCT/LT2012/000007**

(87) International publication number:
**WO 2014/062044 (24.04.2014 Gazette 2014/17)**

(54) **FLUORINATED BENZENESULFONAMIDES AS INHIBITORS OF CARBONIC ANHYDRASE**
FLUORIERTE BENZOLSULFONAMIDE ALS CARBOANHYDRASEHEMMER
BENZÈNESULFONAMIDES FLUORÉS INHIBITEURS D'ANHYDRASE CARBONIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.10.2012 LT 2012092**

(43) Date of publication of application:
**09.09.2015 Bulletin 2015/37**

(73) Proprietor: **Vilnius University**
**01513 Vilnius (LT)**

(72) Inventors:
- **MATULIS, Daumantas**
  **LT-06230 Vilnius (LT)**
- **DUDUTIENE, Virginija**
  **LT-06145 Vilnius (LT)**
- **ZUBRIENE, Asta**
  **LT-08226 Vilnius (LT)**

(74) Representative: **Petniunaite, Jurga**
**AAA Law**
**P.O. Box 33**
**A. Gostauto street 40B**
**03163 Vilnius (LT)**

(56) References cited:
**EP-A1- 2 147 915** **WO-A1-2009/118292**
**WO-A1-2011/029842** **WO-A1-2011/071565**
**WO-A2-2008/017932** **GB-A- 1 025 314**
**GB-A- 1 031 082** **US-A1- 2009 163 586**

- **VIRGINIJA DUDUTIENE ET AL: "4-Substituted-2,3,5,6-tetrafluorobenzenes ulfonamides as inhibitors of carbonic anhydrases I, II, VII, XII, and XIII", BIOORGANIC & MEDICINAL CHEMISTRY, 1 January 2013 (2013-01-01), XP055055325, ISSN: 0968-0896, DOI: 10.1016/j.bmc.2013.01.008**
- **XAVIER DE LEVAL ET AL: "Carbonic Anhydrase Inhibitors: Synthesis and Topical Intraocular Pressure Lowering Effects of Fluorine-Containing Inhibitors Devoid of Enhanced Reactivity", JOURNAL OF MEDICINAL CHEMISTRY, vol. 47, no. 11, 1 May 2004 (2004-05-01), pages 2796-2804, XP055055369, ISSN: 0022-2623, DOI: 10.1021/jm031116j**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Field of the Invention

The present invention describes novel aromatic sulfonamide derivatives, potentially useful in biomedicine as active ingredients of pharmaceutical preparations because of their ability to inhibit enzymes participating in disease progression.

The enzymes in this description of the invention include different metal (mostly zinc) ion-possessing proteins, such as carbonic anhydrases and metalloproteinases.

### Background of the Invention

Carbonic anhydrases are zinc-containing enzymes which catalyze reversible reaction of carbon dioxide hydration. These enzymes participate in essential physiological processes related to respiration, $CO_2$/bicarbonate transport between lungs and metabolizing tissues, pH and $CO_2$ homeostasis, electrolyte secretion in many tissues/organs, etc. To date there are 15 carbonic anhydrase (CA) isozymes identified in humans - 12 catalytically active and 3 inactive, so called carbonic anhydrase related proteins. The 12 active isoforms have different subcellular localization - 5 of them are cytosolic, 4 - membrane bound, 2 mitochondrial and 1 - secreted. The major class of carbonic anhydrase inhibitors is aromatic and heterocyclic inhibitors possessing sulfonamide group. Sulfonamide-class carbonic anhydrase inhibitors are widely used as therapeutic agents for treatment of various diseases, since the 15 carbonic anhydrase isozymes are widely distributed in most of the cells, tissues and organs where they are responsible for essential physiological functions. Another similar protein class is metalloproteinases, proteolytic enzymes, which are characterized by increased expression during various steps of cancer progression. Sulfonamide inhibitors have a great potential for the inhibition of metalloproteinases.

Carbonic anhydrases participate in many essential physiological processes, therefore the increased activity or expression of different CA isoforms results in significant pathological outcomes. Therefore the regulation of CA catalytic activity by means of inhibition or activation proposes a therapeutic perspective.

There are several diseases with the characteristic disbalance of the interconversion between carbonic dioxide and bicarbonate resulting in pH alteration, disturbance of ion transport, fluid secretion, etc. CA activators may have pharmacological applications in pathologies in which learning and memory are impaired, such as Alzheimer's disease or aging (Temperini, C. et al. (2009), Drug Design of Zinc-Enzyme Inhibitors: Functional, Structural, and Disease Applications; Eds.: Supuran, C. T. and Winum, J.-Y., Wiley: Hoboken, NJ, p 473). While most often carbonic anhydrase inhibitors are used as antiglaucoma agents, they are also employed for treatment of another diseases: retinal and cerebral edema (inhibitors of CA I) (Gao, B.B. et al. (2007), Nat. Med. 13, 181), altitude sickness (inhibitors of CA II) (Basnyat, B. et al. (2003), High Alt. Med. Biol. 4, 45), epilepsy (inhibitors of CA II, CA VII, CA XIV) (Hen, N. et al. (2011), J. Med. Chem. 54, 3977). Few novel synthesized inhibitors of CA VA, CA VB, CA XII and CA IX are undergoing clinical investigation as antiobesity and antitumor drugs or diagnostic tools (De Simone, G. et al. (2008), Curr. Pharm. Des. 14, 655; Guler, O. O. et al. (2010), Curr. Med. Chem. 17, 1516). It was identified that CA inhibitors suppress the growth of leukemia, melanoma, lung, ovarian, colon, kidney, prostate, breast, and CNS cancer cells (Supuran, C. T. et al. (2000), Eur. J. Med. Chem. 35, 867; Guler, O.O. et al. (2010), Curr. Med. Chem. 17, 1516; De Simone, G. et al. (2010), Biochim. Biophys. Acta, 1804, 404; Battke, C. et al. (2011), Cancer Immunol. Immunother. 60, 649). Namely, the carbonic anhydrases IX and XII are directly related to cancer development. The use of CA IX-specific inhibitor set for detection and treatment of pre-cancer and neoplastic state is described (WO 2004/048544). There are a few reports about CA XIII involvement in the sperm mobility processes (probably together with CA XIV). Inhibition of these two CAs may be used in obtaining contraceptive agents (Lehtonen, I. et al. (2004), J. Biol. Chem. 279, 2791). It was established that CA inhibitors are useful diuretics for the treatment of patients which suffer from edema and heart deficiency. It is supposed that inhibition of the CA II activity could be useful for the diminishment of the bone resorption. It was shown in prokaryotes that the carbonic anhydrases are essential for respiration, carbon dioxide transport and photosynthesis. Therefore it was hypothesized that carbonic anhydrase inhibitors could be used as antibiotics. Ethoxzolamide was even used for the treatment of meningitis. It was noticed that carbonic anhydrase inhibitors possess an antimalarial activity. (Merlin, C. et al. (2003), J. Bacteriol. 185, 6415; Pastorekova, S. et al. (2004), J. Enzyme Inhib. Med. Chem. 19, 199; WO 2005/107470).

Introduction of fluorine atom as substituent in various positions of the benzene ring of benzenesulfonamides was investigated from the point of view of CA inhibition. Pentafluorobenzenesulfonamide was described as bCAII inhibitor (Olander, J. et al. (1973), JACS, 95, 1616; Krishnamurthy, V. M. et al. (2007), Chem. Asian J. 2, 94). As far as known to the authors of this invention, there are no mention in the literature about 4-substituted-2,3,5,6-tetrafluorobenzenesulfonamides activity towards CA inhibition. Such compounds bearing cycloalkylamino or alkylamino groups in 4 -position were described as anticonvulsants (GB 1031082, BE 659230(original)). The compound 4-methoxy-2,3,5,6-tetrafluorobenzenesulfonamide was described as anticonvulsant too (GB 1025314, BE 664831). The compounds 4-piperonyl-2,3,5,6-tetrafluorobenzenesulfonamide and 4-(cyclohexylamino)-2,3,5,6-tetrafluorobenzenesulfonamide were mentioned in an article about detection of sulfonamido groups (Bradshaw, L. R. A. (1969), Journal of Chromatography, 44, 422). The synthesis of 4-methoxy- and 4-amino-2,3,5,6-tetrafluorobenzenesulfonamides was described (Robson, P. et al. (1963), J. Chem. Soc. 3692). The synthesis of non substituted 2,3,5,6-tetrafluorobenzenesulfonamide was described in the same article. As far as known to the authors of this invention, there are no mention in the literature about 2,4-disubstituted-3,5,6-trifluorobenzenesulfonamides and 3,4-disubstituted-2,5,6-trifluorobenzenesulfonamides. The compounds 2-substituted-3,5,6- trifluorobenzenesulfonamides have not been investigated as CA inhibitors according to authors of this invention. It is known only that compound 2-cyclopropylamino-3,5,6-trifluorobenzenesulfonamide was used for preparation of benzothiadiazine derivatives, which were used as AMPA receptor modulators (WO 2010004139). The synthesis of non substituted 2,3,5-trifluorobenzenesulfonamide, 2,3,4-trifluorobenzenesulfonamide, 2,3,6-trifluorobenzenesulfonamide and 2,4,6-trifluorobenzenesulfonamide and their use for preparation of benzothiadiazine derivatives was described in the same patent. Preparation of pyrazolylbenzothiazoles bearing fragment of 2,3,4-trifluorobenzenesulfonamide and their use as inhibitors of integrin-linked kinase was described (WO 2004011460). The synthesis of 2,3,4-trifluorobenzenesulfonamide and use as intermediate compound for preparation of 2,3-difluorobenzenesulfonamide derivatives was described in another patent (WO 2008017932). These derivatives were investigated as CA inhibitors. As far as known to the authors of this invention, there are no mention in the literature about 3,4,5-trisubstituted-2,6-difluorobenzenesulfonamides. Other substituted difluorobenzenesulfonamides are investigated vaguely as CA inhibitors. Derivatives of 2,3-difluorobenzensulfonamide were described in patent (WO 2008017932). Such fluorinated benzenesulfonamides as non substituted 2,6-difluorobenzenesulfonamide and 3,5-difluorobenzenesulfonamide were investigated for the same reason (Krishnamurthy, V. M. et al. (2007), Chem. Asian J. 2, 94). The compound 5-(aminosulfonyl)-2,3-difluorobenzoic acid was described as intermediate compound for preparation of mono fluorinated substituted benzenesulfonamides as CA inhibitors (Vernier, W. et. al. (2010), Biorg. Med. Chem. 18, 3307). But there are a lot of data about substituted difluorobenzenesulfonamides bearing fluorine atoms in different positions and their use for different purposes than CA inhibition. 4-Substituted-2,3-difluorobenzenesulfonamides were intermediates for preparation of benzothiadiazine derivatives, which were used as AMPA receptor modulators (WO 2010004139). Substituted 2,3-difluorobenzenesulfonamides were investigated as prostaglandin E synthase-1 inhibitors (US 20090163586), matrix metalloprotease inhibitors (WO 2009118292). Substituted 2,4-difluorobenzenesulfonamides were intermediates for preparation of benzothiadiazine derivatives, which were used as AMPA receptor modulators (WO 2010004139). Substituted 2,4-difluorobenzenesulfonamides were investigated as mGluR2 antagonists (WO 2007110337, WO 2006099972), TRPV1 inhibitors (US 20080146637), CCR5 antagonists (WO 2004054974), prostaglandin E synthase-1 inhibitors (US 20090163586). Substituted 2,5-difluorobenzenesulfonamides were intermediates for preparation of benzothiadiazine derivatives, which were used as AMPA receptor modulators (WO 2010004139). Substituted 2,5-difluorobenzenesulfonamides were investigated as dipeptidyl peptidase IV inhibitors (CN 101418001). Substituted 2,6-difluorobenzenesulfonamides were intermediates for preparation of benzothiadiazine derivatives, which were used as AMPA receptor modulators (WO 2010004139). Substituted 3,4-difluorobenzenesulfonamides were intermediates for preparation of benzothiadiazine derivatives, which were used as ATP-Sensitive Potassium Channel Openers (de Tullio, P. et. al. (2005), J. Med. Chem. 48, 4990). Substituted 3,4-difluorobenzenesulfonamides were intermediates for preparation of N-(phenylsulfonyl)benzamides and N-(3-pyridylsulfonyl)benzamides as apoptosis-inducing agents for the treatment of cancer and immune diseases and autoimmune diseases (US 20110124628). Substituted 3,4-difluorobenzenesulfonamides were investigated as prostaglandin E synthase-1 inhibitors (US 20090163586), CCR5 antagonists (WO 2004054974). Substituted 3,5-difluorobenzenesulfonamides were intermediates for preparation of N-(phenylsulfonyl)benzamides and N-(3-pyridylsulfonyl)benzamides as apoptosis-inducing agents for the treatment of cancer and immune diseases and autoimmune diseases (US 20110124628). Substituted 3,5-difluorobenzenesulfonamides were investigated as prostaglandin E synthase-1 inhibitors (US 20090163586), TGR5 agonists (WO 2010093845, WO 2011071565), for treatment of cancer (WO 2011029842). Substituted 3,6-difluorobenzenesulfonamides were investigated for preparation of substituted imidazolidine-2,4-diones (WO 2008017381).

Despite the fact that a large number of different sulfonamides have been synthesized to date, the available pharmaceutical agents created on the basis of these sulfonamides have a number of shortcomings. One of the main shortcomings is the non-selective inhibition of all carbonic anhydrases throughout the whole human body. This results in various unexpected side effects, mostly because of non-specific inhibition of all CA isoforms and their toxicity.

Presently clinically used CA inhibitors, when acting non-specifically, cause a number of side-effects. Especially toxic are systemic inhibitors. They cause electrolyte disbalance, drowsiness, head-ache, depression, apathy, malaise, irritability, nervousness, fatigue, gut irritability, anorexia, nausea, thirst, obstruction, muscle weakness, tremor, hyper- and hypoglycemia, kidney pain, disuria, bone marrow depression, metabolic acidosis and other.

Therefore, the creation of isoform-specific or organ-selective sulfonamide inhibitors is still an important task.

Invented compounds show great possibility to synthesize fluorinated benzenesulfonamides bearing different substitutes in o, m, p positions according to sulfonamide group. Presence of fluorine atoms in such compounds exerts

an acidifying effect on the sulfonamide protons, which correlates with an increase in the CA inhibitory properties. These features enable good possibilities to create isoform-specific sulfonamide inhibitors.

Summary of the Invention

This invention describes new sulfonamides with general structural formula (I)

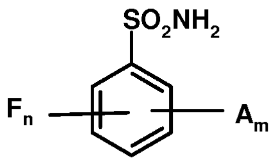

(I)

where benzenesulfonamide ring can be tetra- or penta- substituted,
where

$n>1$, $n<5$
$m\geq1$, (1-3 A groups are identical or different from each other, at least one $A\neq H$, $A=H$ no more than once),
A is H, $R^1$, OH, $OR^1$, SH, $SR^1$, $S(O)R^1$, $SO_2R^1$, $C(O)R^1$, $C(O)OR^1$, $OC(O)R^1$, $NHR^1$, $N(R^1)_2$, $C(O)NHR^1$, $C(O)N(R^1)_2$, $NHC(O)R^1$, $NR^1C(O)R^1$, $NHC(O)OR^1$, $NR^1C(O)OR^1$, $NHC(O)NH_2$, $NHC(O)NHR^1$, $NHC(O)N(R^1)_2$, $NR^1C(O)NHR^1$, $NR^1C(O)N(R^1)_2$, $SO_2NHR^1$, $SO_2N(R^1)_2$, $NR^1SO_2R^1$, $NHSO_2NHR^1$, $NHSO_2N(R^1)_2$, $NR^1SO_2NHR^1$, $NR^1SO_2N(R^1)_2$, C(O)NHNOH, $C(O)NHNOR^1$, $C(O)NHSO_2R^1$, $C(NH)NH_2$, $C(NH)NHR^1$, $C(NH)N(R^1)_2$, $NHSO_2NHR^1$, $NHSO_2N(CH_3)R^1$ $N(CH_3)SO_2N(CH_3)R^1$ Cl, Br, I, CN, $NO_2$, $N_3$, C(O)H, CHNOH, $CH(NOCH_3)$, $CF_3$, $CF_2CF_3$, $OCF_3$, $OCF_2CF_3$, C(O)OH, $C(O)NH_2$,
$R^1$ is $R^2$, $R^3$, $R^4$, $R^5$, $R^6$, $R^7$,
$R^2$ is phenyl, which is unfused or fused with benzene, heteroarene, cycloalkane or heterocycloalkane,
$R^3$ is heteroaryl, which is unfused or fused with benzene, heteroarene, cycloalkane or heterocycloalkane,
$R^4$ is cycloalkyl, cycloalkenyl, cycloalkynyl, heterocycloalkyl, heterocycloakenyl or heterocycloakynyl, each of which is unfused or fused with benzene, heteroarene,
$R^5$ is alkyl, alkenyl or alkynyl each of which is unsubstituted or substituted by one or more identical or different groups selected from $R^8$, OH, $OR^8$, SH, $SR^8$, $S(O)R^8$, $SO_2R^8$, $C(O)R^8$, $C(O)OR^8$, $OC(O)R^8$, $NHR^8$, $N(R^8)_2$, $C(O)NHR^8$, $C(O)N(R^8)_2$, $NHC(O)R^8$, $NR^8C(O)R^8$, $NHC(O)OR^8$, $NR^8C(O)OR^8$, $NHC(O)NH_2$, $NHC(O)NHR^8$, $NHC(O)N(R^8)_2$, $NR^8C(O)NHR^8$, $NR^8C(O)N(R^8)_2$, $SO_2NHR^8$, $SO_2N(R^8)_2$, $NR^8SO_2R^8$, $NHSO_2NHR^8$, $NHSO_2N(R^8)_2$, $NR^8SO_2NHR^8$, $NR^8SO_2N(R^8)_2$, C(O)NHNOH, $C(O)NHNOR^8$, $C(O)NHSO_2R^8$, $C(NH)NH_2$, $C(NH)NHR^8$, $C(NH)N(R^8)_2$, $NHSO_2NHR^8$, $NHSO_2N(CH_3)R^8$, $N(CH_3)SO_2N(CH_3)R^8$, F, Cl, Br, I, CN, $NO_2$, $N_3$, C(O)H, CHNOH, $CH(NOCH_3)$, $CF_3$, $CF_2CF_3$, $OCF_3$, $OCF_2CF_3$, C(O)OH, $C(O)NH_2$,
$R^8$ is $R^9$ , $R^{10}$, $R^{11}$, $R^{12}$, $R^{13}$, $R^{14}$,
$R^9$ is phenyl, which is unfused or fused with benzene, heteroarene, cycloalkane or heterocycloalkane,
$R^{10}$ is heteroaryl, which is unfused or fused with benzene, heteroarene, cycloalkane or heterocycloalkane,
$R^{11}$ is cycloalkyl, cycloalkenyl, cycloalkynyl, heterocycloalkyl, heterocycloakenyl or heterocycloakynyl, each of which is unfused or fused with benzene, heteroarene,
$R^{12}$ is alkyl, alkenyl or alkynyl each of which is unsubstituted or substituted by one or more identical or different groups selected from
$NH_2$, $NHCH_3$, $N(CH_3)_2$, SH, SMe, $C(O)NH_2$, C(O)NHOH, $CF_3$, $CF_2CF_3$, $OCF_3$, $OCF_2CF_3$, C(O)H, C(O)OH, $C(O)OC_2H_5$, OH, $OCH_3$, $OC_2H_5$, $CH_3$, $C_2H_5$, $CH(CH_3)_2$, CN, $N_3$, $NO_2$, F, Cl, Br, I,
$R^{13}$ is phenyl which is unsubstituted or substituted by one or more identical or different groups selected from
$NH_2$, $NHCH_3$, $N(CH_3)_2$, SH, SMe, $C(O)NH_2$, C(O)NHOH, $CF_3$, $CF_2CF_3$, $OCF_3$, $OCF_2CF_3$, C(O)H, C(O)OH, $C(O)OC_2H_5$, OH, $OCH_3$, $OC_2H_5$, $CH_3$, $C_2H_5$, $CH(CH_3)_2$, CN, $N_3$, $NO_2$, F, Cl, Br, I,
$R^{14}$ is heteroaryl, which is unsubstituted or substituted by one or more identical or different groups selected from
$NH_2$, $NHCH_3$, $N(CH_3)_2$, SH, SMe, $C(O)NH_2$, C(O)NHOH, $CF_3$, $CF_2CF_3$, $OCF_3$, $OCF_2CF_3$, C(O)H, C(O)OH, $C(O)OC_2H_5$, OH, $OCH_3$, $OC_2H_5$, $CH_3$, $C_2H_5$, $CH(CH_3)_2$, CN, $N_3$, $NO_2$, F, Cl, Br, I,
$R^6$ is phenyl which is unsubstituted or substituted by one or more identical or different groups selected from $R^{15}$, OH, $OR^{15}$, SH, $SR^{15}$, $S(O)R^{15}$, $SO_2R^{15}$, $C(O)R^{15}$, $C(O)OR^{15}$, $OC(O)R^{15}$, $NHR^{15}$, $N(R^{15})_2$, $C(O)NHR^{15}$, $C(O)N(R^{15})_2$, $NHC(O)R^{15}$, $NR^{15}C(O)R^{15}$, $NHC(O)OR^{15}$, $NR^{15}C(O)OR^{15}$, $NHC(O)NH_2$, $NHC(O)NHR^{15}$, $NHC(O)N(R^{15})_2$, $NR^{15}C(O)NHR^{15}$, $NR^{15}C(O)N(R^{15})_2$, $SO_2NHR^{15}$, $SO_2N(R^{15})_2$, $NR^{15}SO_2R^{15}$, $NHSO_2NHR^{15}$, $NHSO_2N(R^{15})_2$, $NR^{15}SO_2NHR^{15}$, $NR^{15}SO_2N(R^{15})_2$, C(O)NHNOH, $C(O)NHNOR^{15}$, $C(O)NHSO_2R^{15}$, $C(NH)NH_2$, $C(NH)NHR^{15}$, $C(NH)N(R^{15})_2$, $NHSO_2NHR^{15}$ $NHSO_2N(CH_3)R^{15}$, $N(CH_3)SO_2N(CH_3)R^{15}$, F, Cl, Br, I, CN, $NO_2$, $N_3$, C(O)H, CHNOH,

$CH(NOCH_3)$, $CF_3$, $CF_2CF_3$, $OCF_3$, $OCF_2CF_3$, C(O)OH, $C(O)NH_2$,

$R^{15}$ is $R^{16}$, $R^{17}$, $R^{18}$, $R^{19}$, $R^{20}$, $R^{21}$,

$R^{16}$ is phenyl, which is unfused or fused with benzene, heteroarene, cycloalkane or heterocycloalkane,

$R^{17}$ is heteroaryl, which is unfused or fused with benzene, heteroarene, cycloalkane or heterocycloalkane,

$R^{18}$ is cycloalkyl, cycloalkenyl, cycloalkynyl, heterocycloalkyl, heterocycloakenyl or heterocycloakynyl, each of which is unfused or fused with benzene, heteroarene,

$R^{19}$ is alkyl, alkenyl or alkynyl each of which is unsubstituted or substituted by one or more identical or different groups selected from

$NH_2$, $NHCH_3$, $N(CH_3)_2$, SH, SMe, $C(O)NH_2$, C(O)NHOH, $CF_3$, $CF_2CF_3$, $OCF_3$, $OCF_2CF_3$, C(O)H, C(O)OH, $C(O)OC_2H_5$, OH, $OCH_3$, $OC_2H_5$, $CH_3$, $C_2H_5$, $CH(CH_3)_2$, CN, $N_3$, $NO_2$, F, Cl, Br, I,

$R^{20}$ is phenyl which is unsubstituted or substituted by one or more identical or different groups selected from

$NH_2$, $NHCH_3$, $N(CH_3)_2$, SH, SMe, $C(O)NH_2$, C(O)NHOH, $CF_3$, $CF_2CF_3$, $OCF_3$, $OCF_2CF_3$, C(O)H, C(O)OH, $C(O)OC_2H_5$, OH, $OCH_3$, $OC_2H_5$, $CH_3$, $C_2H_5$, $CH(CH_3)_2$, CN, $N_3$, $NO_2$, F, Cl, Br, I,

$R^{21}$ is heteroaryl, which is unsubstituted or substituted by one or more identical or different groups selected from

$NH_2$, $NHCH_3$, $N(CH_3)_2$, SH, SMe, $C(O)NH_2$, C(O)NHOH, $CF_3$, $CF_2CF_3$, $OCF_3$, $OCF_2CF_3$, C(O)H, C(O)OH, $C(O)OC_2H_5$, OH, $OCH_3$, $OC_2H_5$, $CH_3$, $C_2H_5$, $CH(CH_3)_2$, CN, $N_3$, $NO_2$, F, Cl, Br, I,

$R^7$ is heteroaryl, which is unsubstituted or substituted by one or more identical or different groups selected from

$R^{22}$, OH, $OR^{22}$, SH, $SR^{22}$, $S(O)R^{22}$, $SO_2R^{22}$, $C(O)R^{22}$, $C(O)OR^{22}$, $OC(O)R^{22}$, $NHR^{22}$, $N(R^{22})_2$, $C(O)NHR^{22}$, $C(O)N(R^{22})_2$, $NHC(O)R^{22}$, $NR^{22}C(O)R^{22}$, $NHC(O)OR^{22}$, $NR^{22}C(O)OR^{22}$, $NHC(O)NH_2$, $NHC(O)NHR^{22}$, $NHC(O)N(R^{22})_2$, $NR^{22}C(O)NHR^{22}$, $NR^{22}C(O)N(R^{22})_2$, $SO_2NHR^{22}$, $SO_2N(R^{22})_2$, $NR^{22}SO_2R^{22}$, $NHSO_2NHR^{22}$, $NHSO_2N(R^{22})_2$, $NR^{22}SO_2NHR^{22}$, $NR^{22}SO_2N(R^{22})_2$, C(O)NHNOH, $C(O)NHNOR^{22}$, $C(O)NHSO_2R^{22}$ $C(NH)NH_2$, $C(NH)NHR^{22}$, $C(NH)N(R^{22})_2$, $NHSO_2NHR^{22}$, $NHSO_2N(CH_3)R^{22}$, $N(CH_3)SO_2N(CH_3)R^{22}$ , F, Cl, Br, I, CN, $NO_2$, $N_3$, C(O)H, CHNOH, $CH(NOCH_3)$, $CF_3$, $CF_2CF_3$, $OCF_3$, $OCF_2CF_3$, C(O)OH, $C(O)NH_2$,

$R^{22}$ is $R^{23}$, $R^{24}$, $R^{23}$, $R^{26}$, $R^{27}$, $R^{28}$,

$R^{23}$ is phenyl, which is unfused or fused with benzene, heteroarene, cycloalkane or heterocycloalkane,

$R^{24}$ is heteroaryl, which is unfused or fused with benzene, heteroarene, cycloalkane or heterocycloalkane,

$R^{25}$ is cycloalkyl, cycloalkenyl, cycloalkynyl, heterocycloalkyl, heterocycloakenyl or heterocycloakynyl, each of which is unfused or fused with benzene, heteroarene,

$R^{26}$ is alkyl, alkenyl or alkynyl each of which is unsubstituted or substituted by one or more identical or different groups selected from

$NH_2$, $NHCH_3$, $N(CH_3)_2$, SH, SMe, $C(O)NH_2$, C(O)NHOH, $CF_3$, $CF_2CF_3$, $OCF_3$, $OCF_2CF_3$, C(O)H, C(O)OH, $C(O)OC_2H_5$, OH, $OCH_3$, $OC_2H_5$, $CH_3$, $C_2H_5$, $CH(CH_3)_2$, CN, $N_3$, $NO_2$, F, Cl, Br, I,

$R^{27}$ is phenyl which is unsubstituted or substituted by one or more identical or different groups selected from

$NH_2$, $NHCH_3$, $N(CH_3)_2$, SH, SMe, $C(O)NH_2$, C(O)NHOH, $CF_3$, $CF_2CF_3$, $OCF_3$, $OCF_2CF_3$, C(O)H, C(O)OH, $C(O)OC_2H_5$, OH, $OCH_3$, $OC_2H_5$, $CH_3$, $C_2H_5$, $CH(CH_3)_2$, CN, $N_3$, $NO_2$, F, Cl, Br, I,

$R^{28}$ is heteroaryl, which is unsubstituted or substituted by one or more identical or different groups selected from

$NH_2$, $NHCH_3$, $N(CH_3)_2$, SH, SMe, $C(O)NH_2$, C(O)NHOH, $CF_3$, $CF_2CF_3$, $OCF_3$, $OCF_2CF_3$, C(O)H, C(O)OH, $C(O)OC_2H_5$, OH, $OCH_3$, $OC_2H_5$, $CH_3$, $C_2H_5$, $CH(CH_3)_2$, CN, $N_3$, $NO_2$, F, Cl, Br, I.

The objects of the invention are also the non-toxic, pharmaceutically acceptable salts of the sulfonamides of general formula (I). They include all salts which retain activity comparable to original compounds and do not attain any harmful and undesirable effects. Such salts are obtained from compounds with general structural formula (I), by mixing their solution with pharmacologically acceptable acids or bases.

Among the pharmaceutically acceptable acids there may be mentioned, without implying any limitation, hydrochloric acid, hydrobromic acid, sulphuric acid, phosphoric acid, acetic acid, trifluoroacetic acid, lactic acid, pyruvic acid, malonic acid, succinic acid, glutaric acid, fumaric acid, tartaric acid, maleic acid, citric acid, ascorbic acid, oxalic acid, methanesulphonic acid, benzenesulphonic acid, camphoric acid and other.

Among the pharmaceutically acceptable bases there may be mentioned, without implying any limitation, sodium hydroxide, potassium hydroxide, triethylamine and tert-butylamine.

The examples of the implementation of the invention are compounds: 4-substituted-2,3,5,6-tetrafluorobenzenesulfonamides, 2,4-disubstituted-3,5,6-trifluorobenzenesulfonamides, 2-substituted-3,5,6-trifluorobenzensulfonamides, 3,4-disubstituted-2,5,6-trifluorobenzenesulfonamides, 3,4,5-trisubstituted-2,6-difluorobenzenesulfonamides.

Examples of the invented compouds are selected compounds from the group comprising:

2,3,5,6-tetrafluoro-4-hydrazinobenzenesulfonamide;

4-(2-benzylidenehydrazino)-2,3,5,6-tetrafluorobenzenesulfonamide;

2,3,5,6-tetrafluoro-4[(2-hydroxyethyl)thio]benzenesulfonamide;

2,3,5,6-tetrafluoro-4[(2-hydroxyethyl)sulfonyl]benzenesulfonamide;

2-{[4-(aminosulfonyl)-2,3,5,6-tetrafluorophenyl]sulfonyl}ethyl acetate;
2,3,5,6-tetrafluoro-4-(propylthio)benzenesulfonamide;
{[4-(aminosulfonyl)-2,3,5,6-tetrafluorophenyl]thio}acetic acid;
3-{[4-(aminosulfonyl)-2,3,5,6-tetrafluorophenyl]thio}propanoic acid;
6-{[4-(aminosulfonyl)-2,3,5,6-tetrafluorophenyl]amino}hexanoic acid;
2,3,5,6-tetrafluoro-4-(phenylthio)benzenesulfonamide;
2,3,5,6-tetrafluoro-4-(phenylsulfonyl)benzenesulfonamide;
2,3,5,6-tetrafluoro-4-phenoxybenzenesulfonamide;
4-(benzylthio)-2,3,5,6-tetrafluorobenzenesulfonamide;
4-(benzylamino)-2,3,5,6-tetrafluorobenzenesulfonamide;
2,3,5,6-tetrafluoro-4-{[2-(4-hydroxyphenyl)ethyl]amino}benzenesulfonamide;
2,3,5,6-tetrafluoro-4-[(2-phenylethyl)thio]benzenesulfonamide;
2,3,5,6-tetrafluoro-4-[(2-phenylethyl)sulfonyl]benzenesulfonamide;
2,3,5,6-tetrafluoro-4-morpholin-4-ylbenzenesulfonamide;
2,3,5,6-tetrafluoro-4-[(mesitylmethyl)thio]benzenesulfonamide;
4-[(4,6-dimethylpyrimidin-2-yl)thio]-2,3,5,6-tetrafluorobenzenesulfonamide;
4-(1,3-benzothiazol-2-ylthio)-2,3,5,6-tetrafluorobenzenesulfonamide;
4-(1-adamantylamino)-2,3,5,6-tetrafluorobenzenesulfonamide;
3-{[4-(aminosulfonyl)-2,3,5,6-tetrafluorophenyl]thio}-[1,2,3]thiadiazolo[3,4-a]benzimidazole;
4-[(4,5-diphenyl-1*H*-imidazol-2-yl)thio]-2,3,5,6-tetrafluorobenzenesulfonamide;
2-(isopropylamino)-3,5,6-trifluoro-4-[(2-phenylethyl)thio]benzenesulfonamide;
2-(benzylamino)-3,5,6-trifluoro-4-[(2-phenylethyl)thio]benzenesulfonamide;
2-[(2-phenylethyl)amino]-3,5,6-trifluoro-4-[(2-phenylethyl)thio]benzenesulfonamide;
2-[(1-phenylethyl)amino]-3,5,6-trifluoro-4-[(2-phenylethyl)thio]benzenesulfonamide;
2-morpholin-4-yl-3,5,6-trifluoro-4-[(2-phenylethyl)thio]benzenesulfonamide;
2-(cyclohexylamino)-3,5,6-trifluoro-4-[(2-phenylethyl)thio]benzenesulfonamide;
2-(cycloheptylamino)-3,5,6-trifluoro-4-[(2-phenylethyl)thio]benzenesulfonamide;
2-(cyclooctylamino)-3,5,6-trifluoro-4-[(2-phenylethyl)thio]benzenesulfonamide;
2-(cyclododecylamino)-3,5,6-trifluoro-4-[(2-phenylethyl)thio]benzenesulfonamide;
2-[(2,6-dimethoxybenzyl)amino]-3,5,6-trifluoro-4-[(2-phenylethyl)thio]benzenesulfonamide;
2-[(3,4-dimethoxybenzyl)amino]-3,5,6-trifluoro-4-[(2-phenylethyl)thio]benzenesulfonamide;
2-(2,3-dihydro-1*H*-inden-2-ylamino)-3,5,6-trifluoro-4-[(2-phenylethyl)thio]benzenesulfonamide;
2-[(1S)-2,3-dihydro-1*H*-inden-1-ylamino]-3,5,6-trifluoro-4-[(2-phenylethyl)thio]benzenesulfonamide;
2-[(1S)-1,2,3,4-tetrahydronaphthalen-1-ylamino]-3,5,6-trifluoro-4-[(2-phenylethyl)thio]benzenesulfonamide;
2-{[(1S,2R)-2-hydroxy-1,2-diphenylethyl]amino}-3,5,6-trifluoro-4-[(2-phenylethyl)thio]benzenesulfonamide;
2-(cyclooctylamino)-3,5,6-trifluoro-4-[(2-hydroxyethyl)thio]benzenesulfonamide;
2-(cyclododecylamino)-3,5,6-trifluoro-4-[(2-hydroxyethyl)thio]benzenesulfonamide;
2-[(2,6-dimethoxybenzyl)amino]-3,5,6-trifluoro-4-[(2-hydroxyethyl)thio]benzenesulfonamide;
2-[(3,4-dimethoxybenzyl)amino]-3,5,6-trifluoro-4-[(2-hydroxyethyl)thio]benzenesulfonamide;
2-[(1S)-2,3-dihydro-1*H*-inden-1-ylamino]-3,5,6-trifluoro-4-[(2-hydroxyethyl)thio]benzenesulfonamide;
2-[(1S)-1,2,3,4-tetrahydronaphthalen-1-ylamino]-3,5,6-trifluoro-4-[(2-hydroxyethyl)thio]benzenesulfonamide;
2-{[(1S,2R)-2-hydroxy-1,2-diphenylethyl]amino}-3,5,6-trifluoro-4-[(2-hydroxyethyl)thio]benzenesulfonamide;
2-(cyclooctylamino)-3,5,6-trifluoro-4-(propylthio)benzenesulfonamide;
2-(cyclooctylamino)-3,5,6-trifluoro-4-{[2-(4-hydroxyphenyl)ethyl]amino}benzenesulfonamide;
2-(cyclooctylamino)-3,5,6-trifluorobenzenesulfonamide;
2-(cyclododecylamino)-3,5,6-trifluorobenzenesulfonamide;
2-[(2,6-dimethoxybenzyl)amino]-3,5,6-trifluorobenzenesulfonamide;
2-[(3,4-dimethoxybenzyl)amino]-3,5,6-trifluorobenzenesulfonamide;
2-[(1S)-2,3-dihydro-1*H*-inden-1-ylamino]-3,5,6-trifluorobenzenesulfonamide;
2-[(1S)-1,2,3,4-tetrahydronaphthalen-1-ylamino]-3,5,6-trifluorobenzenesulfonamide;
3-(methylamino)-2,5,6-trifluoro-4-[(2-phenylethyl)sulfonyl]benzenesulfonamide;
3-(*tert*-butylamino)-2,5,6-trifluoro-4-[(2-phenylethyl)sulfonyl]benzenesulfonamide;
3-(benzylamino)-2,5,6-trifluoro-4-[(2-phenylethyl)sulfonyl]benzenesulfonamide;
3-[(2-phenylethyl)amino]-2,5,6-trifluoro-4-[(2-phenylethyl)sulfonyl]benzenesulfonamide;
3-morpholin-4-yl-2,5,6-trifluoro-4-[(2-phenylethyl)sulfonyl]benzenesulfonamide;
3-(cyclooctylamino)-2,5,6-trifluoro-4-[(2-phenylethyl)sulfonyl]benzenesulfonamide;
3-(cyclododecylamino)-2,5,6-trifluoro-4-[(2-phenylethyl)sulfonyl]benzenesulfonamide;
3-[(2,6-dimethoxybenzyl)amino]-2,5,6-trifluoro-4-[(2-phenylethyl)sulfonyl]benzenesulfonamide;

3-[(3,4-dimethoxybenzyl)amino]-2,5,6-trifluoro-4-[(2-phenylethyl)sulfonyl]benzenesulfonamide;
3-(1-adamantylamino)-2,5,6-trifluoro-4-[(2-phenylethyl)sulfonyl]benzenesulfonamide;
3-(2,3-dihydro-1*H*-inden-2-ylamino)-2,5,6-trifluoro-4-[(2-phenylethyl)sulfonyl]benzenesulfonamide;
3-[(1S)-2,3-dihydro-1*H*-inden-1-ylamino ]-2,5,6-trifluoro-4-[(2-phenylethyl)sulfonyl]benzenesulfonamide;
3-[(1S)-1,2,3,4-tetrahydronaphthalen-1-ylamino]-2,5,6-trifluoro-4-[(2-phenylethyl)sulfonyl]benzenesulfonamide;
3-{[(1S,2R)-2-hydroxy-1,2-diphenylethyl]amino}-2,5,6-trifluoro-4-[(2-phenylethyl)sulfonyl]benzenesulfonamide;
3-{[(1R,2S)-2-hydroxy-1,2-diphenylethyl]amino}-2,5,6-trifluoro-4-[(2-phenylethyl)sulfonyl]benzenesulfonamide;
3-(benzylamino)-2,5,6-trifluoro-4-[(2-hydroxyethyl)sulfonyl]benzenesulfonamide;
3-(cyclooctylamino)-2,5,6-trifluoro-4-[(2-hydroxyethyl)sulfonyl]benzenesulfonamide;
3-(cyclododecylamino)-2,5,6-trifluoro-4-[(2-hydroxyethyl)sulfonyl]benzenesulfonamide;
3-[(2,6-dimethoxybenzyl)amino]-2,5,6-trifluoro-4-[(2-hydroxyethyl)sulfonyl]benzenesulfonamide;
3-[(3,4-dimethoxybenzyl)amino]-2,5,6-trifluoro-4-[(2-hydroxyethyl)sulfonyl]benzenesulfonamide;
3-[(1S)-2,3-dihydro-1*H*-inden-1-ylamino]-2,5,6-trifluoro-4-[(2-hydroxyethyl) sulfonyl]benzenesulfonamide;
3-[(1S)-1,2,3,4-tetrahydronaphthalen-1-ylamino]-2,5,6-trifluoro-4-[(2-hydroxyethyl)sulfonyl]benzenesulfonamide;
3-{[(1S,2R)-2-hydroxy-1,2-diphenylethyl]amino}-2,5,6-trifluoro-4-[(2-hydroxyethyl)sulfonyl]benzenesulfonamide;
3,5-bis(cyclooctylamino)-2,6-difluoro-4-[(2-phenylethyl)sulfonyl]benzenesulfonamide;
3,5-bis[(3,4-dimethoxybenzyl)amino]-2,6-difluoro-4-[(2-hydroxyethyl)sulfonyl]benzenesulfonamide;

with the proviso that the following compounds are excluded

2,3,5,6-tetrafluoro-4-methoxybenzenesulfonamide;
2,3,5,6-tetrafluoro-4-piperidin-l-ylbenzenesulfonamide;
2,3,5,6-tetrafluoro-4-(methylamino)benzenesulfonamide;
4-(dimethylamino)-2,3,5 ,6-tetrafluorobenzenesulfonamide;
4-(cyclohexylamino)-2,3,5,6-tetrafluorobenzenesulfonamide;
2,3,5,6-tetrafluoro-4-(isopropylamino)benzenesulfonamide;
4-(cyclopentylamino)-2,3,5,6-tetrafluorobenzenesulfonamide;
2,3,5,6-tetrafluoro-4-pyrrolidin-1-ylbenzenesulfonamide;
2,3,5,6-tetrafluoro-4-morpholin-4-ylbenzenesulfonamide;
2,3,5,6-tetrafluoro-4-(4-methylpiperazin-1-yl)benzenesulfonamide;
2,3,5,6-tetrafluoro-4-[(2-hydroxyethyl)amino]benzenesulfonamide;
4-(butylamino)-2,3,5,6-tetrafluorobenzenesulfonamide;
4-azepan-1-yl-2,3,5,6-tetrafluorobenzenesulfonamide;
2-(cyclopropylamino)-3,5,6-trifluorobenzenesulfonamide;
4-N-hexamethyleneimino-2,3,5,6-tetrafluorobenzenesulphonamide.

Demonstration, that all above listed compounds exhibit CA inhibitor properties.
The starting materials used are products that are known or that are prepared according to known operating procedures.
The structures of the compounds described in the Examples were determined according to the usual spectrophotometric techniques (infrared, NMR, mass spectrometry).

## Detailed Description of the Invention

### Abreviations used in the text

AZM - acetazolamide,
CA - carbonic anhydrase,
DMSO - dimethyl sulfoxide,
$Et_3N$ - triethylamine,
EZA - ethoxzolamide,
HRMS - high-resolution mass spectrometry,
ITC - isothermal titration calorimetry,
$K_d$- dissociation constant,
NMR - nuclear magnetic resonance,
Ph - phenyl,

TSA - fluorescent thermal shift assay.

New compounds of the invention are obtained according to general synthesis schemes A-J.

**Scheme A.** Synthesis of 4-substituted-2,3,5,6-tetrafluorobenzenesulfonamides (compounds **2a-x**). Sulfonamides **2a**, **c**, **f-j**, **l-p**, **r-x** were obtained from compound **1** by using appropriate nucleophile in ethanol, methanol or DMSO in the presence of $Et_3N$ or $K_2CO_3$ (excess of nucleophile was used in several cases instead of mentioned bases). The compounds **2d**, **e** were prepared by oxidation of **2c** with $CH_3COOH\backslash H_2O_2$. Oxidation of the compounds **2j**, **p** with $CrO_3$ gave **2k**, **q**. The reaction of **2a** with benzaldehyde leaded to formation of **2b**.

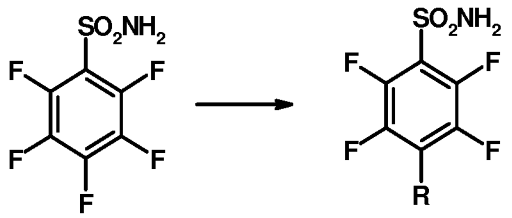

**1** **2 (a-x)**

**R=** $NHNH_2$(a), NHN=CHPh(b), $SCH_2CH_2OH$ (c), $SO_2CH_2CH_2OH$(d), $SO_2CH_2CH_2OCOCH_3$(e), $SCH_2CH_2CH_3$(f), $SCH_2COOH$(g), $SCH_2CH_2COOH$(h), $NH(CH_2)_5COOH$ (i), SPh(j), $SO_2Ph$(k), OPh(l), $SCH_2Ph$(m), $NHCH_2Ph$(n), $NHCH_2CH_2PhpOH$(o), $SCH_2CH_2Ph$(p), $SO_2CH_2CH_2Ph$(q),

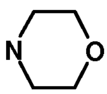 (r), 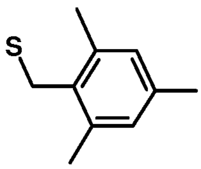 (s), 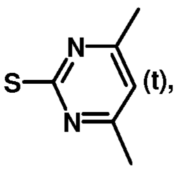 (t), 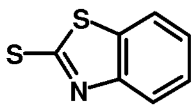 (u), 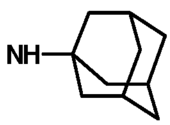 (v),

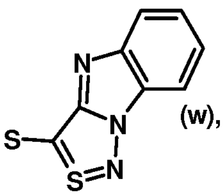 (w), 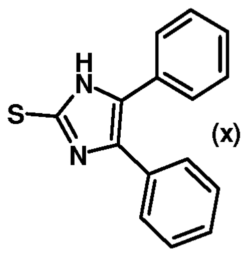 (x)

**Scheme B.** Synthesis of 2-substituted-3,5,6-trifluoro-4-[(2-phenylethyl)thio]benzenesulfonamides (compounds **3a-o**). Sulfonamides **3a-o** were obtained from compound **2p** by using appropriate nucleophile in DMSO in the presence of $Et_3N$.

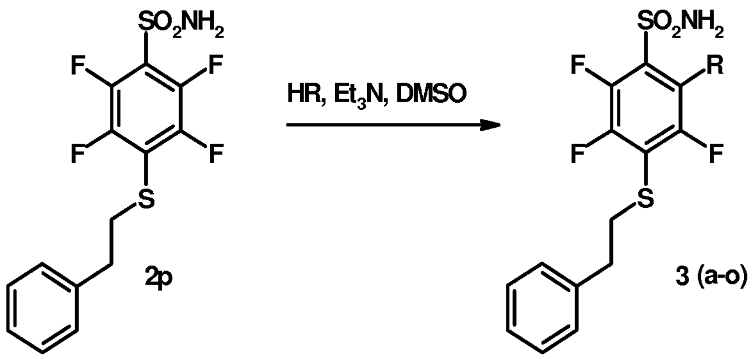


2p 3 (a-o)

R= $NHCH(CH_3)_2$(a), $NHCH_2Ph$(b), $NHCH_2CH_2Ph$(c), $NHCH(CH_3)Ph$(d), 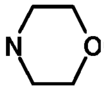(e), 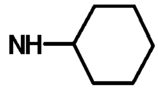 (f),

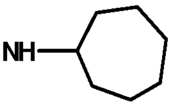 (g), 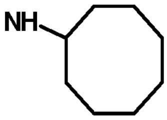 (h), 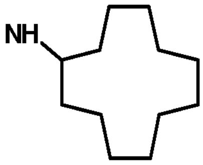 (i), 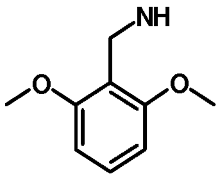 (j), 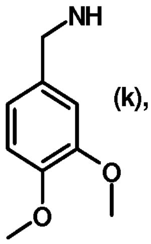 (k),

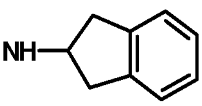 (l), 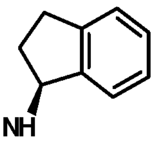 (m), 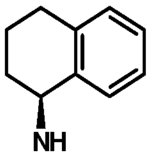 (n), 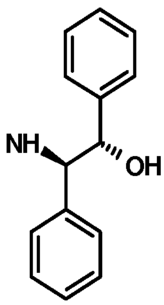 (o)

**Scheme C**. Synthesis of 2-substituted-3,5,6-trifluoro-4-[(2-hydroxyethyl)thio]benzenesulfonamides (compounds **4a-g**). Sulfonamides **4a-g** were obtained from compound **2c** by using appropriate nucleophile in DMSO in the presence of $Et_3N$.

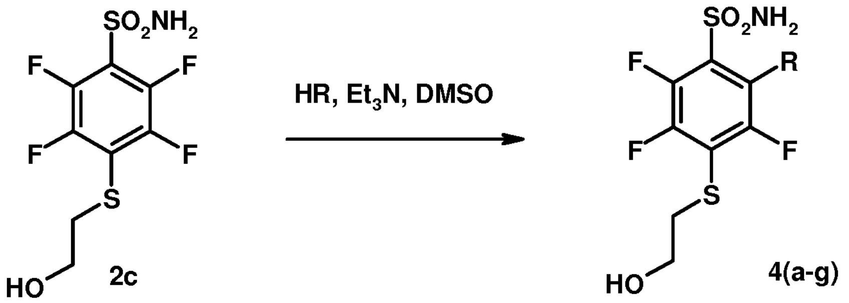


2c

4(a-g)

R=

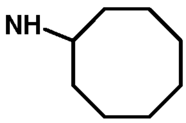
(a),

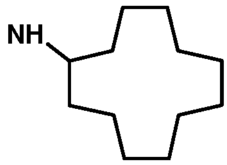
(b),

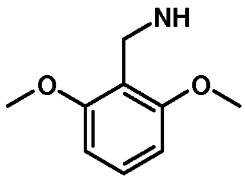
(c),

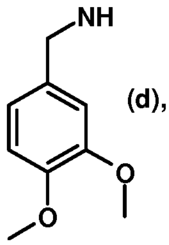
(d),

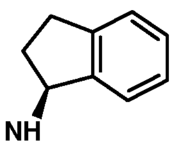
(e),

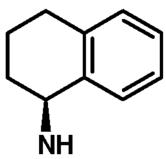
(f),

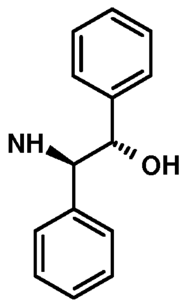
(g)

**Scheme D**. Synthesis of 2-(cyclooctylamino)-3,5,6-trifluoro-4-(propylthio)benzenesulfonamide (compound **5**). The compound **5** was obtained from compound **2f** by using cyclooctylamine in DMSO in the presence of $Et_3N$.

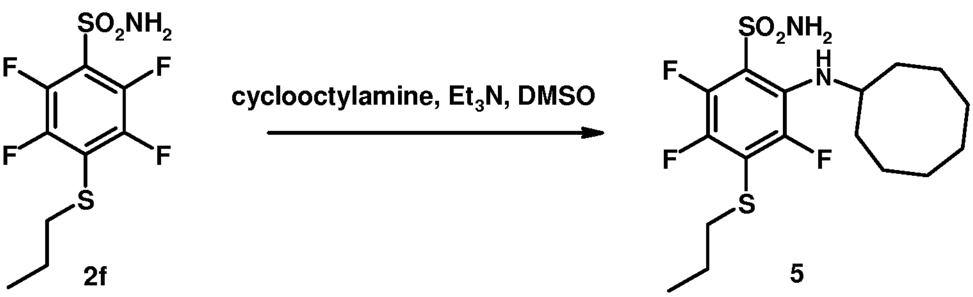


2f

5

**Scheme E**. Synthesis of 2-(cyclooctylamino)-3,5,6-trifluoro-4-{[2-(4-hydroxyphenyl)ethyl]amino}benzensulfonamide (compound **6**). The compound **6** was obtained from compound **2o** by using cyclooctylamine in DMSO in the presence of $Et_3N$.

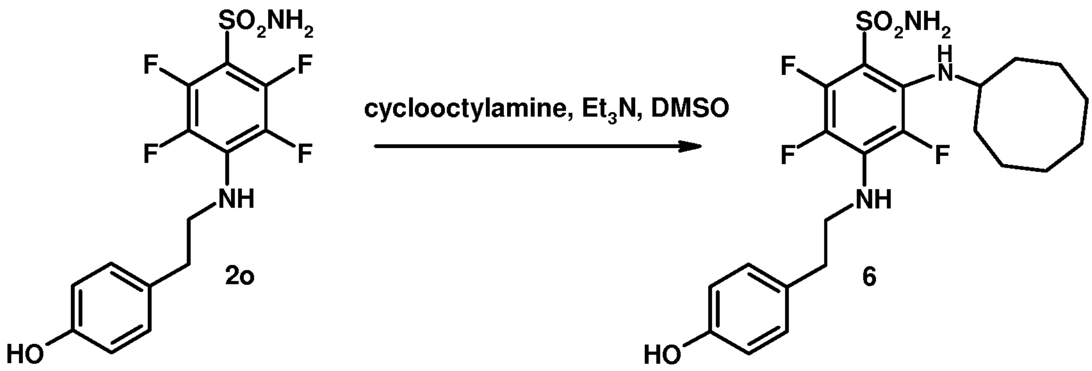


2o

6

**Scheme F**. Synthesis of 2-substituted-3,5,6-trifluorobenzensulfonamides (compounds **8a-f**). Sulfonamides **8a-f** were obtained from compound **7** by using appropriate nucleophile in DMSO in the presence of $Et_3N$.

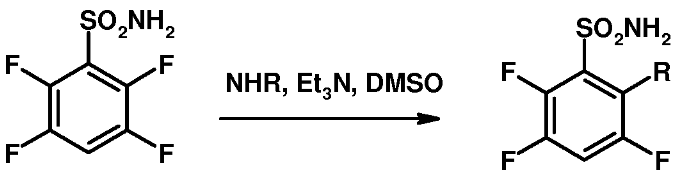


7

8(a-f)

R=

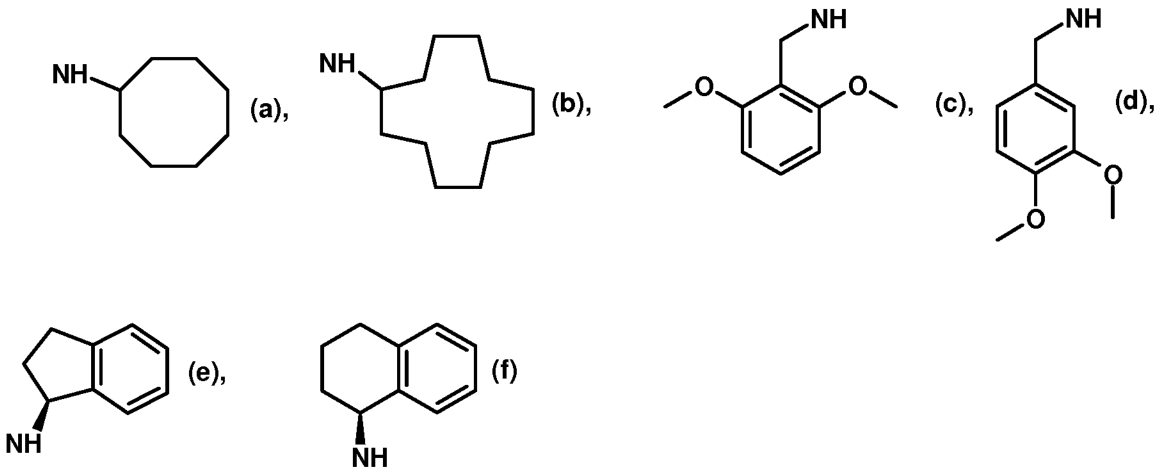

(a), (b), (c), (d), (e), (f)

**Scheme G**. Synthesis of 3-substituted-2,5,6-trifluoro-4-[(2-phenylethyl)sulfonyl]benzenesulfonamides (compounds **9a-o**). Sulfonamides **9d**, **f-o** were obtained from compound **2q** by using appropriate nucleophile in DMSO in presence of $Et_3N$. The compound **9a** was synthesized using excess of methylamine in MeOH. The compound **9c** was synthesized in MeOH in presence of $Et_3N$. Fuorinated derivatives **9b**, **9e** were obtained by using 2eq of nucleophile in DMSO.

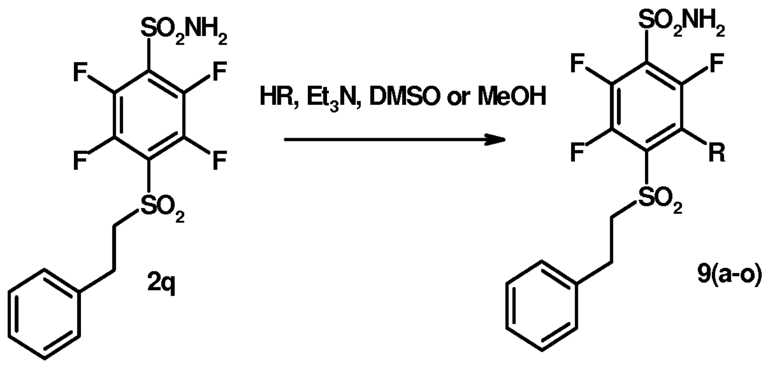

2q
9(a-o)

R= $NHCH_3$(a), $NHC(CH_3)_3$(b), $NHCH_2Ph$(c), $NHCH_2CH_2Ph$(d),

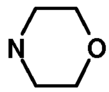
(e),

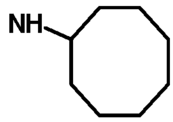
(f),

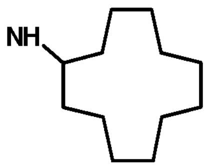
(g),

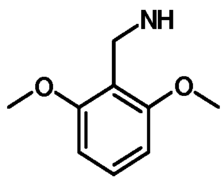
(h),

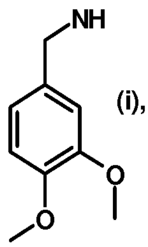
(i),

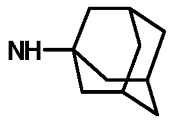
(j),

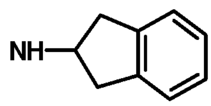
(k),

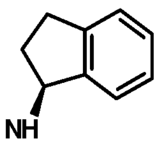
(l),

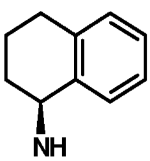
(m),

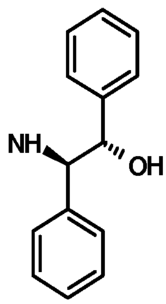
(n),

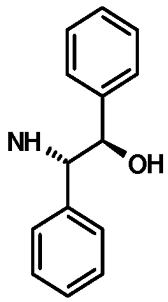
(o)

**Scheme H**. Synthesis of 3-substituted-2,5,6-trifluoro-4-[(2-hydroxyethyl)sulfonyl]benzenesulfonamides (compounds **10a-h**). Sulfonamides **10c-h** were obtained from compound **2d** by using 2eq of appropriate nucleophile in DMSO. The compounds **10a**, **b** were synthesized in MeOH.

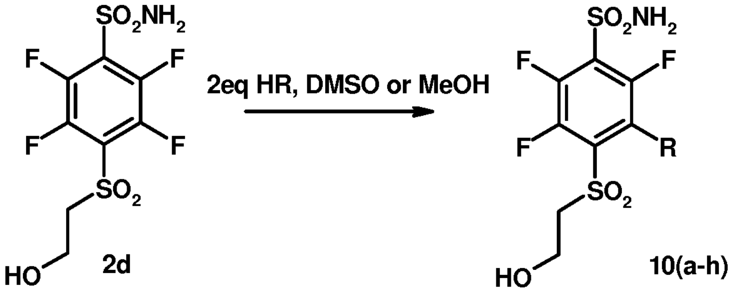


2d

10(a-h)

R= $NHCH_2Ph$(a), 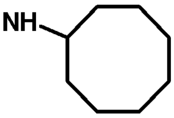 (b), 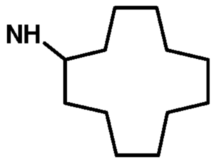 (c), 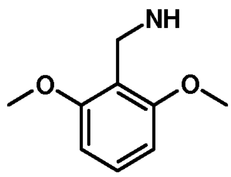 (d), 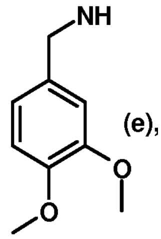 (e),

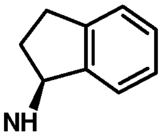 (f), 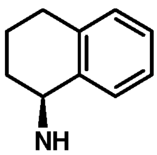 (g), 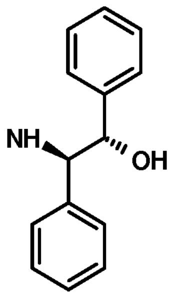 (h)

**Scheme I**. Synthesis of 3,5-bis(cyclooctylamino)-2,6-difluoro-4-[(2-phenylethyl)sulfonyl]benzenesulfonamide (compound **11**). The compound **11** was obtained from compound **2q** by using 2eq of cyclooctylamine in DMSO in the presence of $Et_3N$.

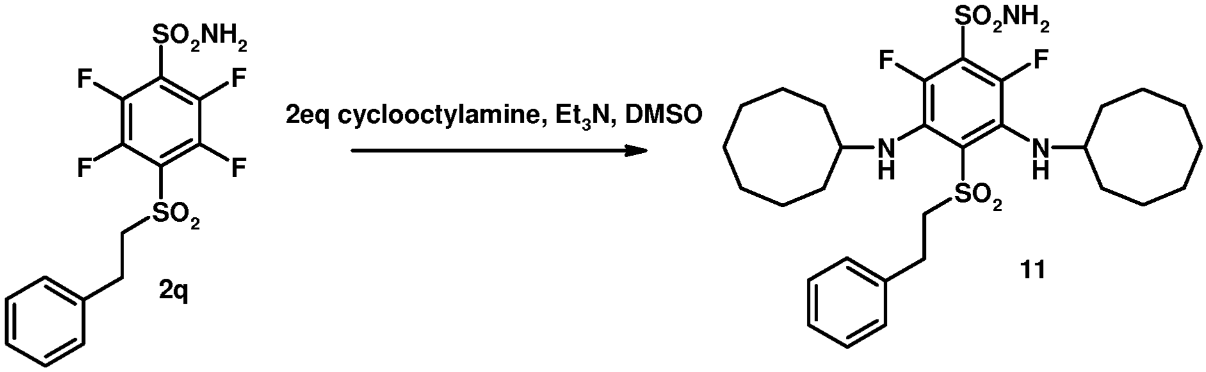


2q

11

**Scheme J**. Synthesis of 3,5-bis[(3,4-dimetoxybenzyl)amino-2,6-difluoro-4-[(2-hydroxyethyl)sulfonyl]benzenesulfonamide (compound **12**). The compound **12** was obtained from compound **2d** by using 4eq of 3,4-dimethoxybenzylamine in DMSO.

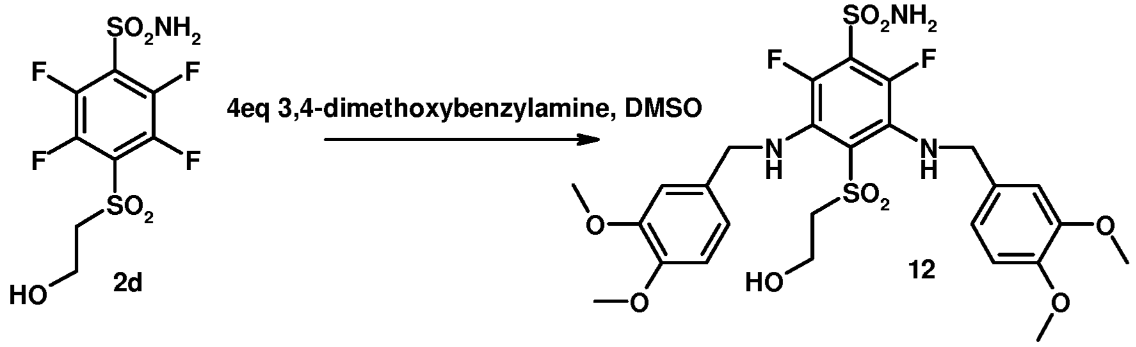


2d

12

Embodiments of the Invention

Represented below are specific examples of invention compounds synthesis. These examples are presented only for illustrative purpose of the invention; they do not limit the scope of the invention.

**Example 1.** Preparation of 2,3,5,6-tetrafluoro-4-hydrazinobenzenesulfonamide (compound **2a**).

The mixture of pentafluorobenzenesulfonamide (compound **1**) (0.32g, 1.295mmol), $NH_2NH_2 \times H_2O$ (0.126mL, 2.59mmol), and EtOH (10mL) was stirred at ambient temperature for 24h. EtOH was evaporated in vacuum and the resultant precipitate was filtered, washed with $H_2O$. Recrystallization was accomplished from $H_2O$. Yield: 0.2g, 60%, decomp. at 160-161°C.

$^1$H NMR (300 MHz, DMSO-$D_6$): 4.68(2H, s, $NH_2$), 7.75(1H, s, NH), 7.98(2H, s, $SO_2NH_2$).

$^{13}$C NMR (75 MHz, DMSO-$D_6$): 108.6(C1, t, J($^{19}$F-$^{13}$C)= 14Hz), 135(C4, t, J($^{19}$F-$^{13}$C)= 16Hz), 136(C3, C5, d, J($^{19}$F-$^{13}$C)= 240Hz), 144.1(C2, C6, d, J($^{19}$F-$^{13}$C)= 240 Hz).

$^{19}$F NMR (282 MHz, DMSO-$D_6$): -143.05(2F, d, J= 15.8Hz), -160.15(2F, d, J= 17.2Hz).

HRMS calcd. for $C_6H_5F_4N_3O_2S$ [$(M+H)^+$]: 335.9782, found: 335.9780.

**Example 2.** Preparation of 4-(2-benzylidenehydrazino)-2,3,5,6-tetrafluorobenzenesulfonamide (compound **2b**).

The mixture of 2,3,5,6-tetrafluoro-4-hydrazinobenzenesulfonamide (compound **2a**) (0.13g, 0.5mmol), benzaldehyde (0.051mL, 0.5mmol), and MeOH (10mL) was stirred at ambient temperature for 4h. MeOH was evaporated in vacuum. Recrystallization was accomplished from iPrOH. Yield: 0.14g, 82%, decomp. at 272-273°C.

$^1$H NMR (300 MHz, DMSO-$D_6$): 7.35-7.55(3H, m, Ph), 7.6-7.75(2H, m, Ph), 8.12(1H, s, NH), 8.25(2H, s, $SO_2NH_2$), 10.96(1H, s, CH).

$^{13}$C NMR (75 MHz, DMSO-$D_6$): 111.7(C1, t, J($^{19}$F-$^{13}$C)= 14Hz), 127.1(Ph), 128.5(C4, t, J($^{19}$F-$^{13}$C)= 13Hz), 129.5(Ph), 130.1(Ph), 135.1(Ph), 136.3(C3, C5, d, J($^{19}$F-$^{13}$C)= 244Hz), 144.7(CH), 144.5(C2, C6, d, J($^{19}$F-$^{13}$C)= 251Hz).

$^{19}$F NMR (282 MHz, DMSO-$D_6$): -141.6(2F, d, J= 16.4Hz), -156.6(2F, d, J= 18Hz).

HRMS calcd. for $C_{13}H_9F_4N_3O_2S$ [$(M+H)^+$]: 348.0424, found: 348.0433.

**Example 3.** Preparation of 2,3,5,6-tetrafluoro-4[(2-hydroxyethyl)sulfonyl] benzenesulfonamide (compound **2d**).

The mixture of 2,3,5,6-tetrafluoro-4[(2-hydroxyethyl)thio]benzenesulfonamide (compound **2c**) (0.1g, 0.33mmol), $CH_3COOH$ (2mL), $H_2O$ (1mL) was heated at 70ºC for 22h. $H_2O_2$ was added by portions (0.2mL) every 4h (overall amount 1mL). The progress of reaction was monitored by TLC. The solvent was then removed in vacuum and crude product was purified by crystallization from $H_2O$. Yield: 0.067g, 61%, mp 139-140°C.

$^1$H NMR (300 MHz, DMSO-$D_6$): 3.75(2H, t, J= 5.4Hz, $SO_2\underline{CH_2}CH_2$), 3.86(2H, t, J= 5.4Hz, $SO_2CH_2\underline{CH_2}$), 5.01(1H, br s, OH), 8.65(2H, s, $SO_2NH_2$).

$^{13}$C NMR (75 MHz, DMSO-$D_6$): 55.9($SO_2CH_2\underline{CH_2}$), 60.2($SO_2\underline{CH_2}$CH2), 123.4(C1 or C4, t, J($^{19}$F-$^{13}$C)= 15Hz),

128(C1 or C4, t, J($^{19}$F-$^{13}$C)= 16Hz), 143.5(C3, C5 or C2, C6, dd, $^1$J($^{19}$F-$^{13}$C)= 254Hz, $^2$J($^{19}$F-$^{13}$C)= 18Hz), 145(C3, C5 or C2, C6, dd, $^1$J($^{19}$F-$^{13}$C)= 248Hz, $^2$J($^{19}$F-$^{13}$C)= 15Hz).

$^{19}$F NMR (282 MHz, DMSO-$D_6$): -136.7(2F, dd, $^1$J= 26Hz, $^2$J= 12Hz), -137.6(2F, dd, $^1$J= 26Hz, $^2$J= 12Hz).

HRMS calcd. for $C_8H_7F_4NO_5S_2$ [(M-H)$^-$]: 335.9629, found: 335.9635.

**Example 4.** Preparation of 2-{[4-(aminosulfonyl)-2,3,5,6-tetrafluorophenyl]sulfonyl} ethyl acetate (compound **2e**).

The mixture of 2,3,5,6-tetrafluoro-4[(2-hydroxyethyl)thio]benzenesulfonamide (compound **2c**) (0.1g, 0.33mmol), $CH_3COOH$ (2mL) was heated at 70ºC for 24h. $H_2O_2$ was added by portions (0.1mL) every 8h (overall amount 0.3mL). The progress of reaction was monitored by TLC. The solvent was then removed in vacuum and crude product was purified by two times crystallization from $H_2O$. Yield: 0.04g, 36%, mp 154°C.

$^1$H NMR (300 MHz, DMSO-$D_6$): 1.85(3H, s, $CH_3$), 4.04(2H, t, J= 5.4Hz, $SO_2\underline{CH_2}CH_2$), 4.43(2H, t, J= 5.4Hz, $SO_2CH_2CH_2$), 8.7(2H, s, $SO_2NH_2$).

$^{13}$C NMR (75 MHz, DMSO-$D_6$): 20.8($CH_3$), 56.6($SO_2CH_2CH_2$), 58($SO_2CH_2CH_2$), 122(C1 or C4, t, J($^{19}$F-$^{13}$C)= 14Hz), 128.6(C1 or C4, t, J($^{19}$F-$^{13}$C)= 16Hz), 143.7(C3, C5 or C2, C6, d, J($^{19}$F-$^{13}$C)= 255Hz), 145(C3, C5 or C2, C6, d, J($^{19}$F-$^{13}$C)= 248Hz), 170.2(C=O).

$^{19}$F NMR (282 MHz, DMSO-$D_6$): -136.2(2F, dd, $^1$J= 26Hz, $^2$J= 12Hz), -137.1(2F, dd, $^1$J= 26Hz, $^2$J= 12Hz).

HRMS calcd. for $C_{10}H_9F_4NO_6S_2$ [(M-H)$^-$]: 377.9735, found: 377.9737.

**Example 5**. Preparation of 2,3,5,6-tetrafluoro-4[(2-hydroxyethyl)thio]benzenesulfonamide (compound **2c**), 2,3,5,6-tetrafluoro-4-(propylthio)benzenesulfonamide (compound **2f**), {[4-(aminosulfonyl)-2,3,5,6-tetrafluorophenyl]thio}acetic acid (compound **2g**), 3-{[4-(aminosulfonyl)-2,3,5,6-tetrafluorophenyl]thio}propanoic acid (compound **2h**), 6-{[4-(aminosulfonyl)-2,3,5,6-tetrafluorophenyl]amino}hexanoic acid (compound **2i**), 4-(benzylthio)-2,3,5,6-tetrafluorobenzenesulfonamide (compound **2m**), 4-(benzylamino)-2,3,5,6-tetrafluorobenzenesulfonamide (compound **2n**), 2,3,5,6-tetrafluoro-4-{[2-(4-hydroxyphenyl)ethyl]amino}benzenesulfonamide (compound **2o**), 2,3,5,6-tetrafluoro-4-[(2-phenylethyl)thio]benzenesulfonamide (compound **2p**), 2,3,5,6-tetrafluoro-4-[(mesitylmethyl)thio]benzenesulfonamide (compound **2s**), 3-{[4-(aminosulfonyl)-2,3,5,6-tetrafluorophenyl]thio}-[1,2,3]thiadiazolo[3,4-a]benzimidazole (compound **2w**).

The mixture of pentafluorobenzenesulfonamide (compound **1**) (0.25g, 1mmol), MeOH (10mL), $Et_3N$ (0.141mL, 1.01mmol) and appropriate nucleophile (1.1mmol) was refluxed. The compounds **2c**, **h**, **i**, **n**, **p**, **s** were obtained after 8h, the compound **2f** was obtained after 10h, the compounds **2g** and **2o** were obtained after 15h, the compound **2m** was obtained after 4h, the compound **2w** was obtained after 1h. MeOH was evaporated in vacuum and the resultant precipitate was filtered, washed with $H_2O$ (except **2g**, **h**, **i**).

The compound **2c**. Recrystallization was accomplished from $H_2O$. Yield: 0.22g, 71%, mp 111-112°C.

$^1$H NMR (300 MHz, DMSO-$D_6$): 3.16(2H, t, J= 6Hz, $S\underline{CH_2}CH_2$), 3.6(2H, k, J= 6Hz, $SCH_2\underline{CH_2}$), 4.97(1H, t, J= 3.6Hz, OH), 8.43(2H, s, $SO_2NH_2$).

$^{13}$C NMR (75 MHz, DMSO-$D_6$): 37.3($SCH_2CH_2$), 61.4($SCH_2CH_2$), 119.95(C1, t, J($^{19}$F-$^{13}$C)= 14Hz), 122.85(C4, t, J($^{19}$F-$^{13}$C)= 14.9Hz), 143.2(C2, C6, ddd, $^1$J($^{19}$F-$^{13}$C)= 254Hz, $^2$J($^{19}$F-$^{13}$C)= 17Hz, $^3$J($^{19}$F-$^{13}$C)= 4Hz), 147(C3, C5, ddd, $^1$J($^{19}$F-$^{13}$C)= 228Hz, $^2$J($^{19}$F-$^{13}$C)= 14Hz, $^3$J($^{19}$F-$^{13}$C)= 4Hz).

$^{19}$F NMR (282 MHz, DMSO-$D_6$): -133.54(2F, dd, $^1$J= 26.8Hz, $^2$J= 13.8Hz), -139.97(2F, dd, $^1$J= 26.2Hz, $^2$J= 12.7Hz).

HRMS calcd. for $C_8H_7F_4NO_3S_2$ [(M-H)$^-$]: 303.9731, found: 303.9729.

The compound **2**f. Recrystallization was accomplished from EtOH:$H_2O$ (2:1). Yield: 0.25g, 81%, mp 120°C.

$^1$H NMR (300 MHz, DMSO-$D_6$): 0.96(3H, t, J= 7.2Hz, $CH_3$), 1.55(2H, sex, J= 7.2Hz, $CH_2$), 3.04(2H, t, J= 7.2Hz, $CH_2$), 8.41(2H, s, $SO_2NH_2$).

$^{13}$C NMR (75 MHz, DMSO-$D_6$): 13.3($CH_3$), 23.5($CH_2$), 36.4($CH_2$), 119.2(C1, t, J($^{19}$F-$^{13}$C)= 20Hz), 123.1(C4, t, J($^{19}$F-$^{13}$C)= 16Hz), 143.2(C2, C6, dd, $^1$J($^{19}$F-$^{13}$C)= 254$H_z$, $^2$J($^{19}$F-$^{13}$C)= 17Hz), 147.2(C3, C5, dd, $^1$J($^{19}$F-$^{13}$C)= 244Hz, $^2$J($^{19}$F-$^{13}$C)= 17Hz).

$^{19}$F NMR (282 MHz, DMSO-$D_6$): -133.9(2F, dd, $^1$J= 25Hz, $^2$J= 11Hz), -139.6(2F, dd, $^1$J= 25Hz, $^2$J= 14Hz).

HRMS calcd. for $C_9H_9F_4NO_2S_2$ [(M-H)$^-$]: 301.9938, found: 301.9940.

The compound **2g**. The product was purified by chromatography on a column of silica gel (0.04-0.063mm) with ethyl acetate. Yield: 0.12g, 38%, mp 158-159°C.

$^1$H NMR (300 MHz, DMSO-$D_6$): 3.9(2H, s, $CH_2$), 8.44(2H, s, $SO_2NH_2$), 12.8(1H, br s, COOH).

$^{13}$C NMR (75 MHz, DMSO-$D_6$): 36($CH_2$), 118.8(C1, t, J($^{19}$F-$^{13}$C)= 20Hz), 123.3(C4, t, J($^{19}$F-$^{13}$C)= 16Hz), 143.1(C2, C6, dd, $^1$J($^{19}$F-$^{13}$C)= 254Hz, $^2$J($^{19}$F-$^{13}$C)= 16Hz), 147(C3, C5, d, J($^{19}$F-$^{13}$C)= 249Hz), 170.3(COOH).

$^{19}$F NMR (282 MHz, DMSO-$D_6$): -133.6(2F, dd, $^1$J= 25Hz, $^2$J= 10Hz), -139.8(2F, dd, $^1$J= 25Hz, $^2$J= 12Hz).

HRMS calcd. for $C_8H_5F_4NO_4S_2$ [(M-H)$^-$]: 317.9523, found: 317.9525.

The compound **2h**. The product was purified by chromatography on a column of silica gel (0.04-0.063mm) with

ethyl acetate. Yield: 0.2g, 59%, mp 168-169°C.

$^{1}$H NMR (300 MHz, DMSO-$D_6$): 2.59(2H, t, J= 6.9Hz, $CH_2$), 3.21(2H, t, J= 6.6Hz, $CH_2$), 8.42(2H, s, $SO_2NH_2$), 12.4(1H, br s, COOH).

$^{13}$C NMR (75 MHz, DMSO-$D_6$): 30.1($CH_2$), 35.4($CH_2$), 118.8(C1, t, J($^{19}$F-$^{13}$C)= 20Hz), 123.4(C4, t, J($^{19}$F-$^{13}$C)= 16Hz), 143.2(C2, C6, dd, $^{1}$J($^{19}$F-$^{13}$C)= 254Hz, $^{2}$J($^{19}$F-$^{13}$C)= 17Hz), 147.3(C3, C5, dd, $^{1}$J($^{19}$F-$^{13}$C)= 241Hz, $^{2}$J($^{19}$F-$^{13}$C)= 19Hz), 173.1(COOH).

$^{19}$F NMR (282 MHz, DMSO-$D_6$): -133.3(2F, dd, $^{1}$J= 25Hz, $^{2}$J= 10Hz), -139.6(2F, dd, $^{1}$J= 25Hz, $^{2}$J= 10Hz).

HRMS calcd. for $C_9H_7F_4NO_4S_2$ [(M-H)$^-$]: 331.968, found: 331.9683.

The compound **2i**. The product was purified by chromatography on a column of silica gel (0.04-0.063mm) with ethyl acetate. Yield: 0.15g, 42%, mp 142-144°C.

$^{1}$H NMR (300 MHz, DMSO-$D_6$): 1.2-1.6(8H, m, $(CH_2)_4$), 2.22(2H, t, J= 7.2Hz, $CH_2$), 6.7(1H, s, NH), 7.93(2H, s, $SO_2NH_2$), 12.01(1H, s, COOH).

$^{13}$C NMR (75 MHz, DMSO-$D_6$): 24.9($CH_2$), 26.3($CH_2$), 30.7($CH_2$), 34.3($CH_2$), 44.7($CH_2$), 108.2(C1, t, J($^{19}$F-$^{13}$C)= 15Hz), 132(C4, t, J($^{19}$F-$^{13}$C)= 16Hz), 136.4(C2, C6, d, J($^{19}$F-$^{13}$C)= 238Hz), 144.7(C3, C5, d, J($^{19}$F-$^{13}$C)= 247Hz), 175.1(COOH).

$^{19}$F NMR (282 MHz, DMSO-$D_6$): -142.7(2F, d, J= 16.6Hz), -161.69(2F, d, J= 18.3Hz).

HRMS calcd. for $C_{12}H_{14}F_4N_2O_4S$ [(M-H)$^-$]: 357.0538, found: 357.0542.

The compound **2m.** Recrystallization was accomplished from iPrOH. Yield: 0.23g, 64%, mp 184°C.

$^{1}$H NMR (300 MHz, DMSO-$D_6$): 4.3(2H, s, $CH_2$), 7.1-7.5(5H, m, ArH), 8.44(2H, s, $SO_2NH_2$).

$^{13}$C NMR (75 MHz, DMSO-$D_6$): 38.6($CH_2$), 118.5(C1, t, J($^{19}$F-$^{13}$C)= 21Hz), 123.6(C4, t, J($^{19}$F-$^{13}$C)= 16Hz), 128.4(Ar), 129.3(Ar), 129.5(Ar), 137.4(Ar), 143.1(C2, C6, dd, $^{1}$J($^{19}$F-$^{13}$C)= 254Hz, $^{2}$J($^{19}$F-$^{13}$C)= 17Hz), 147.3(C3, C5, dd, $^{1}$J($^{19}$F-$^{13}$C)= 248Hz, $^{2}$J($^{19}$F-$^{13}$C)= 17Hz).

$^{19}$F NMR (282 MHz, DMSO-$D_6$): -132.8(2F, dd, $^{1}$J= 27Hz, $^{2}$J= 11Hz), -139.8(2F, dd, $^{1}$J= 26Hz, $^{2}$J= 10Hz).

HRMS calcd. for $C_{13}H_9F_4NO_2S_2$ [(M-H)$^-$]: 349.9938, found: 349.9940.

The compound **2n.** Recrystallization was accomplished from $H_2O$:EtOH (1:1). Yield: 0.21g, 62%, mp132-133°C.

$^{1}$H NMR (300 MHz, DMSO-$D_6$): 4.56(2H, d, J= 6.3Hz, $CH_2$), 7.2-7.6(6H, m, NH, ArH), 7.98(2H, s, $SO_2NH_2$).

$^{13}$C NMR (75 MHz, DMSO-$D_6$): 47.9($CH_2$), 109(C1, t, J($^{19}$F-$^{13}$C)= 17Hz), 127.2(Ar), 127.7(Ar), 129(Ar), 131.8(C4, t, J($^{19}$F-$^{13}$C)= 16Hz), 136.7(C2, C6, d, J($^{19}$F-$^{13}$C)= 240Hz), 140.5(Ar), 144.3(C3, C5, d, J($^{19}$F-$^{13}$C)= 241Hz).

$^{19}$F NMR (282 MHz, DMSO-$D_6$): -142.51(2F, d, J= 18.3Hz), -160.38(2F, d, J= 18.3Hz).

HRMS calcd. for $C_{13}H_{10}F_4N_2O_2S$ [(M+H)$^+$]: 335.0472, found: 335.0472.

The compound **2o**. Recrystallization was accomplished from EtOH:$H_2O$ (1:2). Yield: 0.25g, 68%, decomp. at 100°C.

$^{1}$H NMR (300 MHz, DMSO-$D_6$): 2.74(2H, t, J= 7.8Hz, $CH_2$), 3.45-3.6(2H, m, $CH_2$), 6.7(2H, d, J= 8.4Hz, ArH), 7.01(2H, d, J= 8.4Hz, ArH), 7.96(2H, s, $SO_2NH_2$), 9.23(1H, s, NH).

$^{13}$C NMR (75 MHz, DMSO-$D_6$): 36.5($CH_2$), 46.8($CH_2$), 108.4(C1, t, J($^{19}$F-$^{13}$C)= 16Hz), 115.9(Ar), 129.4(Ar), 130.3(Ar), 131.9(C4, t, J($^{19}$F-$^{13}$C)= 16Hz), 136.5(C2, C6, d, J($^{19}$F-$^{13}$C)= 240Hz), 144.4(C3, C5, d, J($^{19}$F-$^{13}$C)= 240Hz), 156.5(Ar).

$^{19}$F NMR (282 MHz, DMSO-$D_6$): -142.6(2F, d, J= 19Hz), -161.4(2F, d, J= 18Hz).

HRMS calcd. for $C_{14}H_{12}F_4N_2O_3S$ [(M-H)$^-$]: 363.0432, found: 363.0435.

The compound **2p**. Recrystallization was accomplished from EtOH:$H_2O$ (2:1). Yield: 0.24g, 71%, mp 121°C

$^{1}$H NMR (300 MHz, DMSO-$D_6$): 2.91(2H, t, J= 7.2Hz, $SCH_2\underline{CH_2}$), 3.37(2H, t, J= 7.2Hz, $S\underline{CH_2}CH_2$), 7.1-7.4(5H, m, ArH), 8.41(2H, s, $SO_2NH_2$).

$^{13}$C NMR (75 MHz, DMSO-$D_6$): 35.4($SCH_2CH_2$), 36.6($SCH_2CH_2$), 119(C1, t, J($^{19}$F-$^{13}$C)= 20Hz), 122.9(C4, t, J($^{19}$F-$^{13}$C)= 16Hz), 127.2(Ar), 128.9(Ar), 129.3(Ar), 139.7(Ar), 143.2(C2, C6, dd, $^{1}$J($^{19}$F-$^{13}$C)= 257Hz, $^{2}$J($^{19}$F-$^{13}$C)= 17Hz), 147(C3, C5, dd, $^{1}$J($^{19}$F-$^{13}$C)= 249Hz, $^{2}$J($^{19}$F-$^{13}$C)- 17Hz).

$^{19}$F NMR (282 MHz, DMSO-$D_6$): -133.45(2F, dd, $^{1}$J= 26.5Hz, $^{2}$J= 13.5Hz), -139.8(2F, dd, $^{1}$J= 25.6Hz, $^{2}$J= 10.7Hz).

HRMS calcd. for $C_{14}H_{11}F_4NO_2S_2$ [(M-H)$^-$]: 364.0095, found: 364.0100.

The compound **2s**. Recrystallization was accomplished from EtOH. Yield: 0.29g, 73%, mp 213-214°C.

$^{1}$H NMR (300 MHz, DMSO-$D_6$): 2.23(3H, s, $CH_3$), 2.3(6H, s, $CH_3$), 4.31(2H, s, $CH_2$), 6.89(2H, s, ArH), 8.47(2H, s, $SO_2NH_2$).

$^{13}$C NMR (75 MHz, DMSO-$D_6$): 19.6($CH_3$), 21.3($CH_3$), 34.3($CH_2$), 119.2(C1, t, J($^{19}$F-$^{13}$C)= 21Hz), 123.6(C4, t, J($^{19}$F-$^{13}$C)= 18Hz), 129.4(Ar), 129.7(Ar), 137.8(Ar), 137.9(Ar), 143.3(C2, C6, d, J($^{19}$F-$^{13}$C)= 253Hz), 147.4(C3, C5, d, J($^{19}$F-$^{13}$C)= 243Hz).

$^{19}$F NMR (282 MHz, DMSO-$D_6$): -133.1(2F, dd, $^{1}$J= 25Hz, $^{2}$J= 10Hz), -139.5(2F, dd, $^{1}$J= 25Hz, $^{2}$J= 11Hz).

HRMS calcd. for $C_{16}H_{15}F_4NO_2S_2$ [(M-H)$^-$]: 392.0408, found: 392.0412.

The compound **2w**. The obtained compound was washed with MeOH. Yield: 0.13g, 30%, decomp. at 233-234°C.

$^{1}$H NMR (300 MHz, DMSO-$D_6$): 7.33(1H, t, J= 7.8Hz, ArH), 7.57(1H, t, J= 8.4Hz, ArH), 7.81(1H, d, J= 8.7Hz,

ArH), 8.2(1H, d, J= 8.1Hz, ArH), 8.55(2H, s, $SO_2NH_2$).

$^{13}C$ NMR (75 MHz, DMSO-$D_6$): 118.4(Ar), 120.2(C1, t, J($^{19}F$-$^{13}C$)= 15Hz), 125.9(Ar), 126(Ar), 127.7(C4, t, J($^{19}F$-$^{13}C$)= 16Hz), 130(Ar), 133.2(Ar), 133.4(Ar), 148(C2, C6, d, J($^{19}F$-$^{13}C$)= 254Hz), 152(C3, C5, d, J($^{19}F$-$^{13}C$)= 236Hz), 158.7(Ar), 158.8(Ar).

$^{19}F$ NMR (282MHz, DMSO-$D_6$): -127.24(2F, dd, $^1J$= 25.9Hz, $^2J$= 11.3Hz), -134(2F, dd, $^1J$= 25.9Hz, $^2J$= 10.7Hz).

HRMS calcd. for $C_{14}H_6F_4N_4O_2S_3$ [$(M+H)^+$]: 434.9662, found: 434.9667.

**Example 6.** Preparation of 2,3,5,6-tetrafluoro-4-(phenylthio)benzenesulfonamide (compound **2j**).

The mixture of pentafluorobenzenesulfonamide (compound **1**) (0.28g, 1.13mmol), MeOH (10 mL), $Et_3N$ (0.158mL, 1.13mmol) and HSPh (0.116mL, 1.13mmol) was stirred at ambient temperature for 2h. MeOH was evaporated in vacuum and the resultant precipitate was filtered, washed with $H_2O$. Recrystallization was accomplished from EtOH:$H_2O$ (2:1). Yield: 0.28g, 74%, mp 139°C.

$^1H$ NMR (300 MHz, DMSO-$D_6$): 7.2-7.8(5H, m, ArH), 8.47(2H, s, $SO_2NH_2$).

$^{13}C$ NMR (75 MHz, DMSO-$D_6$): 117.7(C1, t, J($^{19}F$-$^{13}C$)= 19.6Hz), 124.5(C4, t, J($^{19}F$-$^{13}C$)= 16.3Hz), 128.7(Ar), 130.3(Ar), 130.4(Ar), 132.4(Ar), 143.5(C2, C6, ddd, $^1J$($^{19}F$-$^{13}C$)= 255Hz, $^2J$($^{19}F$-$^{13}C$)= 17Hz, $^3J$($^{19}F$-$^{13}C$)= 5Hz), 147.2(C3, C5, ddd, $^1J$($^{19}F$-$^{13}C$)= 240Hz, $^2J$($^{19}F$-$^{13}C$)= 17Hz, $^3J$($^{19}F$-$^{13}C$)= 5Hz).

$^{19}F$ NMR (282 MHz, DMSO-$D_6$): -132.59(2F, dd, $^1J$= 24.5Hz, $^2J$= 11.6Hz), -138.75(2F, dd, $^1J$= 24.5Hz, $^2J$= 10.4Hz).

HRMS calcd. for $C_{12}H_7F_4NO_2S_2$ [$(M-H)^-$]: 335.9782, found: 335.9780.

**Example 7.** Preparation of 2,3,5,6-tetrafluoro-4-(phenylsulfonyl)benzenesulfonamide (compound **2k**).

The mixture of 2,3,5,6-tetrafluoro-4-(phenylthio)benzenesulfonamide (compound **2j**) (0.2g, 0.59mmol), $CrO_3$ (0.18g, 1.8mmol), $CH_3COOH$ (10mL), $H_2O$ (0.5mL) was heated at 70ºC for 2h. The solvent was then removed in vacuum and the resultant precipitate was filtered, washed with $H_2O$. Recrystallization was accomplished from EtOH. Yield: 0.17g, 77%, mp 266-267°C.

$^1H$ NMR (300 MHz, DMSO-$D_6$): 7.77(2H, t, J= 7.8Hz, ArH), 7.87(1H, t, J= 7.5Hz, ArH), 8.09(2H, d, J= 7.8Hz, ArH), 8.59(2H, s, $SO_2NH_2$).

$^{13}C$ NMR (75 MHz, DMSO-$D_6$): 123(C1, C4, t, J($^{19}F$-$^{13}C$)= 13Hz), 128.4(Ar), 130.7(Ar), 136.2(Ar), 140.4(Ar), 143.8(C2, C6 or C3, C5, dd, $^1J$($^{19}F$-$^{13}C$)= 258Hz, $^2J$($^{19}F$-$^{13}C$)= 18Hz), 144.4(C2, C6 or C3, C5, dd, $^1J$($^{19}F$-$^{13}C$)= 258Hz, $^2J$($^{19}F$-$^{13}C$)= 18Hz).

$^{19}F$ NMR (282 MHz, DMSO-$D_6$): -136.6(4F, s).

HRMS calcd. for $C_{12}H_7F_4NO_4S_2$ [$(M-H)^-$]: 367.968, found: 367.9684.

**Example 8.** Preparation of 2,3,5,6-tetrafluoro-4-phenoxybenzenesulfonamide (compound **21**).

The mixture of pentafluorobenzenesulfonamide (compound **1**) (0.2g, 0.81mmol), sodium phenoxide trihydrate (0.145g, 0.85mmol) and DMSO (1mL) was stirred at ambient temperature for 4h. The mixture was then diluted with $H_2O$ (20mL) and the resultant precipitate was filtered, washed with $H_2O$. Recrystallization was accomplished from EtOH:$H_2O$ (1:2). Yield: 0.07g, 27%, mp 164-165°C.

$^1H$ NMR (300 MHz, DMSO-$D_6$): 7.0-7.6(5H, m, ArH), 8.44(2H, s, $SO_2NH_2$).

$^{13}C$ NMR (75 MHz, DMSO-$D_6$): 116.4(Ar), 120.4(C1, t, J($^{19}F$-$^{13}C$)= 14Hz), 125(Ar), 130.9(Ar), 136.3(C4, t, J($^{19}F$-$^{13}C$)= 14Hz), 141.9(C3, C5, d, J($^{19}F$-$^{13}C$)= 250Hz), 144.4(C2, C6, d, J($^{19}F$-$^{13}C$)= 256Hz), 157(Ar).

$^{19}F$ NMR (282 MHz, DMSO-$D_6$): -139.6(2F, d, J= 16Hz), -154(2F, d, J= 16Hz).

HRMS calcd. for $C_{12}H_7F_4NO_3S$ [$(M-H)^-$]: 320.001, found: 320.0008.

**Example 9.** Preparation of 2,3,5,6-tetrafluoro-4-[(2-phenylethyl)sulfonyl]benzenesulfonamide (compound **2q**)

The mixture of 2,3,5,6-tetrafluoro-4-[(2-phenylethyl)thio]benzenesulfonamide (**2p**) (0.1g, 0.27mmol), $CrO_3$ (0.082g, 0.82mmol), $CH_3COOH$ (10mL), $H_2O$ (0.2mL) was heated at 60ºC for 4h. The resultant precipitate was filtered, washed with $H_2O$. Yield: 0.07g, 64%, mp 248-249°C.

$^1H$ NMR (300 MHz, DMSO-$D_6$): 3.12(2H, t, J= 7.2Hz, $SO_2CH_2\underline{CH_2}$), 3.97(2H, t, J= 7.8Hz, $SO_2\underline{CH_2}CH_2$), 7.1-7.4(5H, m, ArH), 8.66(2H, s, $SO_2NH_2$).

$^{13}C$ NMR (75 MHz, DMSO-$D_6$): 28.6($SO_2CH_2\underline{CH_2}$), 58($SO_2\underline{CH_2}CH_2$), 121.5(C1 or C4, t, J($^{19}F$-$^{13}C$)= 15Hz), 127.5(Ar), 128.2(C1 or C4, t, J($^{19}F$-$^{13}C$)= 16Hz), 128.9(Ar), 129.2(Ar), 137.4(Ar), 143.5(C2, C6 or C3, C5, dd, $^1J$($^{19}F$-$^{13}C$)= 258Hz, $^2J$($^{19}F$-$^{13}C$)= 17Hz), 144.8(C2, C6 or C3, C5, dd, $^1J$($^{19}F$-$^{13}C$)= 255Hz, $^2J$($^{19}F$-$^{13}C$)= 17Hz).

$^{19}F$ NMR (282 MHz, DMSO-$D_6$): -136(2F, dd, $^1J$= 25Hz, $^2J$= 12Hz), -137.1(2F, dd, $^1J$= 25Hz, $^2J$= 12Hz).

HRMS calcd. for $C_{14}H_{11}F_4NO_4S_2$ [(M-H)$^-$]: 395.9993, found: 395.9996.

**Example 10.** Preparation of 2,3,5,6-tetrafluoro-4-morpholin-4-ylbenzenesulfonamide (compound **2r**).

The mixture of pentafluorobenzenesulfonamide (compound **1**) (0.2g, 0.809mmol), MeOH (10mL), morpholine (0.141mL, 1.62mmol) was refluxed for 8h. MeOH was evaporated in vacuum and the resultant precipitate was filtered, washed with $H_2O$. Recrystallization was accomplished from EtOH:$H_2O$ (1:1). Yield: 0.13g, 52%, mp 233-234°C.
$^1$H NMR (300 MHz, DMSO-$D_6$): 3.32(4H, br s, 2$CH_2$), 3.7(4H, br s, 2$CH_2$), 8.2(2H, s, $SO_2NH_2$).
$^{13}$C NMR (75 MHz, DMSO-$D_6$): 51.2(N($CH_2$)$_2$, t, J($^{19}$F-$^{13}$C)= 3.6Hz), 67.2(O($CH_2$)$_2$), 115.4(C1, t, J($^{19}$F-$^{13}$C)= 19Hz), 133.3(C4, t, J($^{19}$F-$^{13}$C)= 16Hz), 144.7(C3, C5, d, J($^{19}$F-$^{13}$C)= 256Hz), 146.3(C2, C6, d, J($^{19}$F-$^{13}$C)= 244Hz).
$^{19}$F NMR (282 MHz, DMSO-$D_6$): -141.6(2F, d, J= 15.8Hz), -151.2(2F, d, J= 16.6Hz).
HRMS calcd. for $C_{10}H_{10}F_4N_2O_3S$ [(M+H)$^+$]: 315.0421, found: 315.0426.

**Example 11.** Preparation of 4-[(4,6-dimethylpyrimidin-2-yl)thio]-2,3,5,6-tetrafluorobenzenesulfonamide (**2t**), 4-(1,3-benzothiazol-2-ylthio)- 2,3,5,6-tetrafluorobenzenesulfonamide (**2u**), 4-[(4,5-diphenyl-1*H*-imidazol-2-yl)thio]-2,3,5,6-tetrafluorobenzenesulfonamide (**2x**).

The mixture of pentafluorobenzenesulfonamide (compound **1**) (0.1g, 0.404mmol), $K_2CO_3$ (0.056g, 0.406mmol), DMSO (2mL) and appropriate nucleophile (0.404mmol) was stirred at ambient temperature for 5h. The mixture was then diluted with $H_2O$ (20mL) and extracted with EtAc (2×10mL). The combined organic phase was dried over $Na_2SO_4$ and evaporated in vacuum.
The compound **2t.** Recrystallization was accomplished from EtOH:$H_2O$ (2:1). Yield: 0.12g, 80%, mp 131-132°C.
$^1$H NMR (300 MHz, DMSO-$D_6$): 2.34(6H, s, 2$CH_3$), 7.15(1H, s, ArH), 8.58(2H, s, $SO_2NH_2$).
$^{13}$C NMR (75 MHz, DMSO-$D_6$): 24($CH_3$), 114.1(C1, t, J($^{19}$F-$^{13}$C)= 20Hz), 118.5(Ar), 125.5(C4, t, J($^{19}$F-$^{13}$C)= 14Hz), 143.3(C2, C6, d, J($^{19}$F-$^{13}$C)= 255Hz), 147.7(C3, C5, d, J($^{19}$F-$^{13}$C)= 248Hz), 166.3(Ar), 169(Ar).
$^{19}$F NMR (282 MHz, DMSO-$D_6$): -130.65(2F, dd, $^1$J= 25.4Hz, $^2$J= 10.7Hz), -139.25(2F, dd, $^1$J= 26.2Hz, $^2$J= 11Hz).
HRMS calcd. for $C_{12}H_9F_4N_3O_2S_2$ [(M+H)$^+$]: 368.0145, found: 368.0142.
The compound **2u.** Recrystallization was accomplished from EtOH:$H_2O$ (2:1). Yield: 0.11g, 69%, mp 171°C.
$^1$H NMR (300 MHz, DMSO-$D_6$): 7.44(1H, t, J= 8.1Hz, ArH), 7.51(1H, t, J= 8.1Hz, ArH), 7.9 (1H, d, J= 7.5Hz, ArH), 8.06(1H, d, J= 8.4Hz, ArH), 8.65(2H, s, $SO_2NH_2$).
$^{13}$C NMR (75 MHz, DMSO-$D_6$): 113.8(C1, t, J($^{19}$F-$^{13}$C)= 20Hz), 122.7(Ar), 122.9(Ar), 126.2(Ar), 126.5(C4, t, J($^{19}$F-$^{13}$C)= 16Hz), 127.5(Ar), 136(Ar), 143.5(C2, C6, dd, $^1$J($^{19}$F-$^{13}$C)= 254Hz, $^2$J($^{19}$F-$^{13}$C)= 12Hz), 147.6(C3, C5, dd, $^1$J($^{19}$F-$^{13}$C)= 250Hz, $^2$J($^{19}$F-$^{13}$C)= 16Hz), 153.1(Ar), 162.4(Ar).
$^{19}$F NMR (282 MHz, DMSO-$D_6$): -130.52(2F, dd, $^1$J= 24.8Hz, $^2$J= 11.6Hz), -137.87(2F, dd, $^1$J= 24.8Hz, $^2$J= 11.6Hz).
HRMS calcd. for $C_{13}H_6F_4N_2O_2S_3$ [(M-H)$^-$]: 392.9455, found: 392.9457.
The compound **2x.** Recrystallization was accomplished from EtOH:$H_2O$ (2:1). Yield: 0.12g, 63%, mp 221-122°C.
$^1$H NMR (300 MHz, DMSO-$D_6$): 7.2-7.5(10H, m, ArH), 8.51(2H, s, $SO_2NH_2$), 13.2(1H, br s, NH).
$^{13}$C NMR (75 MHz, DMSO-$D_6$): 116(C1, t, J($^{19}$F-$^{13}$C)= 15Hz), 124.1(C4, t, J($^{19}$F-$^{13}$C)= 16Hz), 128.3(br s, Ar), 129.3(br s, Ar), 133.9(Ar), 143.3(C2, C6, d, J($^{19}$F-$^{13}$C)= 253Hz), 146.7(C3, C5, d, J($^{19}$F-$^{13}$C)= 250Hz).
$^{13}$C NMR (75 MHz, DMSO-$D_6$, $CF_3COOH$): 115.1(C1, t, J($^{19}$F-$^{13}$C)= 15Hz), 125(C4, t, J($^{19}$F-$^{13}$C)= 16Hz), 128.6(Ar), 129.3(Ar), 129.5(Ar), 130.2(Ar), 133.4(Ar), 134.2(Ar), 143.5(C2, C6, d, J($^{19}$F-$^{13}$C)= 255Hz), 146.9(C3, C5, d, J($^{19}$F-$^{13}$C)= 255Hz).
$^{19}$F NMR (282 MHz, DMSO-$D_6$): -133.53(2F, dd, J= 24.8Hz, J= 10.2Hz), -139,13(2F, dd, J= 24.8Hz, J= 10.2Hz).
HRMS calcd. for $C_{21}H_{13}F_4N_3O_2S_2$ [(M+H)$^+$]: 480.0458, found: 480.0449.

**Example 12.** Preparation of 4-(1-adamantylamino)-2,3,5,6-tetrafluorobenzenesulfonamide (compound **2v**).

The mixture of pentafluorobenzenesulfonamide (compound **1**) (0.2g, 0.81mmol), $Et_3N$ (0.226mL, 1.62mmol), adamantanamine hydrochloride (0.15g, 0.81mmol) and DMSO (2mL) was stirred at ambient temperature for 48h. The mixture was then diluted with $H_2O$ (20mL) and extracted with EtAc (2×10mL). The combined organic phase was dried over $Na_2SO_4$ and evaporated in vacuum. The product was purified by chromatography on a column of silica gel (0.04-0.063mm) with EtAc:$CHCl_3$ (1:4), $R_f$= 0.63. Yield: 0.04g, 12%, mp 122-123°C.
$^1$H NMR (300 MHz, $CDCl_3$):1.6-1.8(6H, m, adamantane), 1.8-2(6H, m, adamantane), 2.1-2.25(3H, m, adamantane), 4.05(1H, s, NH), 5.59(2H, s, $SO_2NH_2$).
$^{13}$C NMR (75 MHz, $CDCl_3$): 30.1(adamantane), 36.1(adamantane), 43.6(adamantane), 54.8(adamantane), 110.2(C1), 130.4(C4, t, J($^{19}$F-$^{13}$C)= 14Hz), 138(C2, C6, d, J($^{19}$F-$^{13}$C)= 248Hz), 144.5(C3, C5, d, J($^{19}$F-$^{13}$C)= 251Hz).

$^{19}F$ NMR (282 MHz, $CDCl_3$): -141.53(2F, d, J= 16Hz), -152.4(2F, d, J= 17.5Hz).

HRMS calcd. for $C_{16}H_{18}F_4N_2O_2S$ $[(M-H)^-]$: 377.0952, found: 377.0952.

**Example 13.** Preparation of 2-(isopropylamino)-3,5,6-trifluoro-4-[(2-phenylethyl)thio]benzenesulfonamide (compound **3a**), 2-(benzylamino)-3,5,6-trifluoro-4-[(2-phenylethyl)thio]benzenesulfonamide (compound **3b**), 2-[(2-phenylethyl)amino]-3,5,6-trifluoro-4-[(2-phenylethyl)thio]benzenesulfonamide (compound **3c**), 2-[(1-phenylethyl)amino]-3,5,6-trifluoro-4-[(2-phenylethyl)thio]benzenesulfonamide (compound **3d**), 2-morpholin-4-yl-3,5,6-trifluoro-4-[(2-phenylethyl)thio]benzenesulfonamide (compound **3e**), 2-(cyclohexylamino)-3,5,6-trifluoro-4-[(2-phenylethyl)thio]benzenesulfonamide (compound **3f**), 2-(cycloheptylamino)-3,5,6-trifluoro-4-[(2-phenylethyl)thio]benzenesulfonamide (compound **3g**), 2-(cyclooctylamino)-3,5,6-trifluoro-4-[(2-phenylethyl)thio]benzenesulfonamide (compound **3h**), 2-(cyclododecylamino)-3,5,6-trifluoro-4-[(2-phenylethyl)thio]benzenesulfonamide (compound **3i**) 2-[(2,6-dimethoxybenzyl)amino]-3,5,6-trifluoro-4-[(2-phenylethyl)thio]benzenesulfonamide (compound **3j**), 2-[(3,4-dimethoxybenzyl)amino]-3,5,6-trifluoro-4-[(2-phenylethyl)thio]benzenesulfonamide (compound **3k**), 2-(2,3-dihydro-1*H*-inden-2-ylamino)-3,5,6-trifluoro-4-[(2-phenylethyl)thio]benzenesulfonamide (compound **3l**), 2-[(1S)-2,3-dihydro-1*H*-inden-1-ylamino]-3,5,6-trifluoro-4-[(2-phenylethyl)thio]benzenesulfonamide (compound **3m**), 2-[(1S)-1,2,3,4-tetrahydronaphthalen-1-ylamino]-3,5,6-trifluoro-4-[(2-phenylethyl)thio]benzenesulfonamide (compound **3n**), 2-{[(1S, 2R)-2-hydroxy-1,2-diphenylethyl]amino}-3,5,6-trifluoro-4-[(2-phenylethyl)thio]benzenesulfonamide (compound **3o**).

The mixture of 2,3,5,6-tetrafluoro-4-[(2-phenylethyl)thio]benzenesulfonamide (compound **2p**) (0.2g, 0.55mmol), $Et_3N$ (0.08mL, 0.57mmol), DMSO (1mL) and appropriate nucleophile (0.57mmol) was stirred at 60°C for 16h, compounds **3n**, **o** were obtained after 40h, compound **3e** was obtained after stirring at 70°C for 26h. The mixture was then diluted with $H_2O$ (20mL) and extracted with EtAc (3×10mL). The combined organic phase was dried over $MgSO_4$ and evaporated in vacuum.

The compound **3a.** The product was purified by chromatography on a column of silica gel (0.04-0.063mm) with EtAc(5%):$CHCl_3$, Rf= 0.51. Yield: 0.12g, 55%, mp 47-48°C.

$^1H$ NMR (300 MHz, $CDCl_3$): 1.23(6H, dd, $^1J$= 6.3Hz, $^2J$= 1.2Hz, $2CH_3$), 2.94(2H, t, J= 8Hz, $SCH_2\overline{CH_2}$), 3.28(2H, t, J= 8Hz, $S\overline{CH_2}CH_2$), 3.84-3.95(1H, m, CH), 5.52(2H, br s, $SO_2NH_2$), 7.2-7.4(5H, m, ArH).

$^{13}C$ NMR (75 MHz, $CDCl_3$): 24($2CH_3$), 35.4($SCH_2CH_2$, t, J($^{19}F$-$^{13}C$)= 3.7Hz), 36.8($SCH_2CH_2$), 48.4(CH, d, J($^{19}F$-$^{13}C$)= 11Hz), 117.4(C1, dd, $^1J$($^{19}F$-$^{13}C$)= 12Hz, $^2J$($^{19}F$-$^{13}C$)= 5Hz), 119.9(C4, t, J($^{19}F$-$^{13}C$)= 21Hz), 127(Ar), 128.8(Ar), 132.6(C2, d, J($^{19}F$-$^{13}C$)= 16Hz), 139.4(Ar), 142.2(C5 or C6, ddd, $^1J$($^{19}F$-$^{13}C$)= 240Hz, $^2J$($^{19}F$-$^{13}C$)= 16Hz, $^3J$($^{19}F$-$^{13}C$)= 5Hz), 145(C5 or C6, ddd, $^1J$($^{19}F$-$^{13}C$)= 247Hz, $^2J$($^{19}F$-$^{13}C$)= 16Hz, $^3J$($^{19}F$-$^{13}C$)= 5Hz), 148.6(C3, d, J($^{19}F$-$^{13}C$)= 243Hz).

$^{19}F$ NMR (282 MHz, $CDCl_3$): -125.1(C3-F, d, J= 11Hz), -143.4(C5-F, dd, $^1J$= 27Hz, $^2J$= 12Hz), -148.5(C6-F, d, J= 26Hz).

HRMS calcd. for $C_{17}H_{19}F_3N_2O_2S_2$ $[(M+H)^+]$: 405.0913, found: 405.0918.

The compound **3b.** The product was purified by chromatography on a column of silica gel (0.04-0.063mm) with EtAc:$CHCl_3$(1:14), Rf= 0.45. Yield: 0.15g, 60%, mp 94-95°C.

$^1H$ NMR (300 MHz, DMSO-$D_6$): 2.74(2H, t, J= 7.4Hz, $SCH_2CH_2$), 3.19(2H, t, J= 7.5Hz, $SCH_2CH_2$), 4.51(2H, dd, $^1J$=6.3Hz, $^2J$= 4.2Hz, $NHCH_2$), 6.81(1H, td, $^1J$= 6.4Hz, $^2J$= 1.8Hz, NH), 7.1-7.4(10H, m, ArH), 8.2(2H, s, $SO_2NH_2$).

$^{13}C$ NMR (75 MHz, DMSO-$D_6$): 35.3($SCH_2CH_2$, t, J($^{19}F$-$^{13}C$)= 3.2Hz), 36.3($SCH_2CH_2$), 50.5($NHCH_2$, d, J($^{19}F$-$^{13}C$)= 12Hz), 118(C4, t, J($^{19}F$-$^{13}C$)= 19Hz), 119.2(C1, dd, $^1J$($^{19}F$-$^{13}C$)= 12Hz, $^2J$($^{19}F$-$^{13}C$)= 4.5Hz), 127.1(Ar), 127.9(Ar), 128.1(Ar), 129(Ar), 129.2(Ar), 129.22(Ar), 132.9(C2, d, J($^{19}F$-$^{13}C$)= 14Hz), 139.8(Ar), 140.1(Ar), 141.8(C5 or C6, d, J($^{19}F$-$^{13}C$)= 234Hz), 144.8(C5 or C6, d, J($^{19}F$-$^{13}C$)= 261Hz), 148.1(C3, d, J($^{19}F$-$^{13}C$)= 242Hz).

$^{19}F$ NMR (282 MHz, DMSO-$D_6$): -121.2(C3-F, d, J= 9Hz), -138.1(C5-F, dd, $^1J$= 27Hz , $^2J$= 12Hz), -145.4(C6-F, d, J= 27Hz).

HRMS calcd. for $C_{21}H_{19}F_3N_2O_2S_2$ $[(M+H)^+]$: 453.0913, found: 453.0917.

The compound **3c.** The product was purified by chromatography on a column of silica gel (0.04-0.063mm) with EtAc(10%):$CHCl_3$, Rf= 0.53. Yield: 0.24g, 92%, 90-91°C.

$^1H$ NMR (300 MHz, $CDCl_3$): 2.9-3.0(4H, m, $SCH_2\overline{CH_2}$, $NHCH_2\overline{CH_2}$), 3.29(2H, t, J= 8Hz, $S\overline{CH_2}CH_2$), 3.6-3.75(2H, m, $NHCH_2$), 5.21(2H, s, $SO_2NH_2$), 7.2-7.4(10H, m, ArH).

$^{13}C$ NMR (75 MHz, $CDCl_3$): 35.4($SCH_2CH_2$, t, J($^{19}F$-$^{13}C$)= 4Hz), 36.8($SCH_2CH_2$), 37.1($NHCH_2CH_2$), 48.2($NHCH_2$, d, J($^{19}F$-$^{13}C$)= 11.5Hz), 116.3(C1, dd, $^1J$($^{19}F$-$^{13}C$)= 12Hz, $^2J$($^{19}F$-$^{13}C$)= 5Hz), 120(C4, t, J($^{19}F$-$^{13}C$)= 22Hz), 126.9(Ar), 127(Ar), 128.8(Ar), 128.9(Ar), 129.2(Ar), 133.1(C2, d, J($^{19}F$-$^{13}C$)= 14Hz), 139(Ar), 139.4(Ar), 141.9(C5 or C6, ddd, $^1J$($^9F$-$^{13}C$)= 242Hz, $^2J$($^{19}F$-$^{13}C$)= 16Hz, $^3J$($^{19}F$-$^{13}C$)= 5Hz), 145(C5 or C6, ddd, $^1J$($^{19}F$-$^{13}C$)= 253Hz, $^2J$($^{19}F$-$^{13}C$)= 11Hz, $^3J$($^{19}F$-$^{13}C$)= 4Hz), 148.2(C3, d, J($^{19}F$-$^{13}C$)= 242Hz).

$^{19}F$ NMR (282 MHz, $CDCl_3$): -126.6(C3-F, d, J= 11Hz), -143.6(C5-F, dd, $^1J$= 27Hz, $^2J$= 12Hz), -149.1(C6-F, d, J= 28Hz).

HRMS calcd. for $C_{22}H_{21}F_3N_2O_2S_2$ $[(M+H)^+]$: 467.1069, found: 467.1077.

The compound **3d.** The product was purified by chromatography on a column of silica gel (0.04-0.063mm) with EtAc(5%):$CHCl_3$, Rf= 0.75. Yield: 0.15g, 58%.

$^1$H NMR (300 MHz, $CDCl_3$):1.59(3H, dd, $^1$J= 7Hz, $^2$J= 1Hz, $CH_3$), 2.78(2H, td, $^1$J= 7.5Hz, $^2$J= 3Hz, $SCH_2CH_2$), 3.13(2H, t, J= 8Hz, $SCH_2CH_2$), 4.85-4.95(1H, m, CH), 5.33(2H, s, $SO_2NH_2$), 7.1-7.4(10H, m, ArH).

$^{13}$C NMR (75 MHz, $CDCl_3$): 24.6($CH_3$), 35.5($SCH_2CH_2$, t, J($^{19}$F-$^{13}$C)= 3.5Hz), 36.7($SCH_2CH_2$), 56.4(NHCH, d, J($^{19}$F-$^{13}$C)= 12Hz), 117.5(C1, dd, $^1$J($^{19}$F-$^{13}$C)= 12Hz, $^2$J($^{19}$F-$^{13}$C)= 5Hz), 119.8(C4, t, J($^{19}$F-$^{13}$C)= 19.5Hz), 126.3(Ar), 126.9(Ar), 127.7(Ar), 128.8(Ar), 128.8 (Ar), 128.9(Ar), 132.3(C2, d, J($^{19}$F-$^{13}$C)= 13Hz), 139.4(Ar), 142.4(C5 or C6, ddd, $^1$J($^9$F-$^{13}$C)= 240Hz, $^2$J($^{19}$F-$^{13}$C)= 15Hz, $^3$J($^{19}$F-$^{13}$C)= 5Hz), 144.9(C5 or C6, ddd, $^1$J($^{19}$F-$^{13}$C)= 248Hz, $^2$J($^{19}$F-$^{13}$C)= 16Hz, $^3$J($^{19}$F-$^{13}$C)= 4Hz), 148.8(C3, d, J($^{19}$F-$^{13}$C)= 244Hz).

$^{19}$F NMR (282 MHz, $CDCl_3$): -122.5(C3-F, d, J= 11Hz), -143.2(C5-F, dd, $^1$J= 27Hz , $^2$J= 12Hz), -147.4(C6-F, d, J= 26Hz).

HRMS calcd. for $C_{22}H_{21}F_3N_2O_2S_2$ [$(M+H)^+$]: 467.1069, found: 467.1069.

The compound **3e.** The product was purified by chromatography on a column of silica gel (0.04-0.063mm) with EtAc:$CHCl_3$(1:3), Rf= 0.38. Yield: 0.1g, 42%, mp 149-150°C.

$^1$H NMR (300 MHz, $CDCl_3$): 2.9-3.05(4H, m, $SCH_2CH_2$, morpholine), 3.33(2H, t, J= 8Hz, $SCH_2CH_2$), 3.48(2H, t, J= 11Hz, morpholine), 3.73(2H, t, J= 11Hz, morpholine), 4.0(2H, d, J= 11Hz, morpholine), 6.12(2H, s, $SO_2NH_2$), 7.15-7.35(5H, m, ArH).

$^{13}$C NMR (75 MHz, $CDCl_3$): 35.3($SCH_2CH_2$, t, J($^{19}$F-$^{13}$C)= 4Hz), 36.9($SCH_2CH_2$), 51.3(morpholine, d, J($^{19}$F-$^{13}$C)= 6Hz), 67.7(morpholine), 120.3(C4, t, J($^{19}$F-$^{13}$C)= 21Hz), 127.1(Ar), 128.8(Ar), 128.9(Ar), 129.5(C2, d, J($^{19}$F-$^{13}$C)= 7Hz), 131.7(C1, dd, $^1$J($^{19}$F-$^{13}$C)= 16Hz, $^2$J($^{19}$F-$^{13}$C)= 5Hz), 139.1(Ar), 143.9(C5 or C6, ddd, $^1$J($^{19}$F-$^{13}$C)= 260Hz, $^2$J($^{19}$F-$^{13}$C)= 16Hz, $^3$J($^{19}$F-$^{13}$C)= 5Hz), 149.6(C5 or C6, ddd, $^1$J($^{19}$F-$^{13}$C)= 250Hz, $^2$J($^{19}$F-$^{13}$C)= 17Hz, $^3$J($^{19}$F-$^{13}$C)= 6Hz), 157.8(C3, d, J($^{19}$F-$^{13}$C)= 251Hz).

$^{19}$F NMR (282 MHz, $CDCl_3$): -118.8(C3-F, d, J= 13Hz), -131.5(C6-F, d, J= 25Hz), - 143(C5-F, d, $^1$J= 24Hz, $^2$J= 13Hz).

HRMS calcd. for $C_{18}H_{19}F_3N_2O_3S_2$ [$(M+H)^+$]: 433.0862, found: 433.0863.

The compound **3f.** The product was purified by chromatography on a column of silica gel (0.04-0.063mm) with EtAc:$CHCl_3$(1:9), Rf= 0.63. Yield: 0.12g, 50%, mp 62-63°C.

$^1$H NMR (300 MHz, $CDCl_3$): 1.2-1.5(5H, m, cyclohexane), 1.6-1.7(1H, m, cyclohexane), 1.7-1.85(2H, m, cyclohexane), 1.9-2.05(2H, m, cyclohexane), 2.93(2H, t, J= 8Hz, $SCH_2CH_2$), 3.27(2H, t, J= 8Hz, $SCH_2CH_2$), 3.6-3.7(1H, m, CH of cyclohexane), 5.57(2H, s, $SO_2NH_2$), 6.16(1H, br s, NH), 7.2-7.4(5H, m, ArH).

$^{13}$C NMR (75 MHz, $CDCl_3$): 25(cyclohexane), 25.8(cyclohexane), 34.4(cyclohexane), 35.4($SCH_2CH_2$, t, J($^{19}$F-$^{13}$C)= 4Hz), 36.8($SCH_2CH_2$), 55.3(CH of cyclohexane, d, J($^{19}$F-$^{13}$C)= 11Hz), 117.2(C1, dd, $^1$J($^{19}$F-$^{13}$C)= 12Hz, $^2$J($^{19}$F-$^{13}$C)= 5Hz), 119.6(C4, t, J($^{19}$F-$^{13}$C)= 22Hz), 127(Ar), 128.8(Ar), 132.6(C2, d, J($^{19}$F-$^{13}$C)= 15Hz), 139.4(Ar), 142(C5 or C6, ddd, $^1$J($^9$F-$^{13}$C)= 240Hz, $^2$J($^{19}$F-$^{13}$C)= 16Hz, $^3$J($^{19}$F-$^{13}$C)= 5Hz), 145(C5 or C6, ddd, $^1$J($^{19}$F-$^{13}$C)= 248Hz, $^2$J($^{19}$F-$^{13}$C)= 16Hz, $^3$J($^{19}$F-$^{13}$C)= 4Hz), 148.5(C3, d, J($^{19}$F-$^{13}$C)= 243Hz).

$^{19}$F NMR (282 MHz, $CDCl_3$): -125.1(C3-F, d, J= 10Hz), -143.5(C5-F, dd, $^1$J= 27Hz, $^2$J= 12Hz), -149(C6-F, d, J= 27Hz).

HRMS calcd. for $C_{20}H_{23}F_3N_2O_2S_2$ [$(M+H)^+$]: 445.1226, found: 445.1235.

The compound **3g.** The product was purified by chromatography on a column of silica gel (0.04-0.063mm) with EtAc(5%):$CHCl_3$, Rf= 0.63. Yield: 0.12g, 48%.

$^1$H NMR (300 MHz, $CDCl_3$): 1.4-1.8(10H, m, cycloheptane), 1.9-2.1(2H, m, cycloheptane), 2.94(2H, t, J= 8Hz, $SCH_2CH_2$), 3.27(2H, t, J= 8Hz, $SCH_2CH_2$), 3.7-3.8(1H, m, CH of cycloheptane), 5.57(2H, s, $SO_2NH_2$), 6(1H, br s, NH), 7.1-7.4(5H, m, ArH).

$^{13}$C NMR (75 MHz, $CDCl_3$): 24.1(cycloheptane), 28.3(cycloheptane), 35.4($SCH_2CH_2$, t, J($^{19}$F-$^{13}$C)= 4Hz), 36.1(cycloheptane), 36.8($SCH_2CH_2$), 57.5(CH of cycloheptane, d, J($^{19}$F-$^{13}$C)= 10Hz), 117.2(C1, dd, $^1$J($^{19}$F-$^{13}$C)= 12Hz, $^2$J($^{19}$F-$^{13}$C)= 5Hz), 119.7(C4, t, J($^{19}$F-$^{13}$C)= 19Hz), 127(Ar), 128.8(Ar), 132.5(C2, d, J($^{19}$F-$^{13}$C)= 15Hz), 139.5(Ar), 142(C5 or C6, ddd, $^1$J($^{19}$F-$^{13}$C)= 240Hz, $^2$J($^{19}$F-$^{13}$C)= 16Hz, $^3$J($^{19}$F-$^{13}$C)= 5Hz), 145(C5 or C6, ddd, $^1$J($^{19}$F-$^{13}$C)= 250Hz, $^2$J($^{19}$F-$^{13}$C)= 16Hz, $^3$J($^{19}$F-$^{13}$C)= 4Hz), 148.5(C3, d, J($^{19}$F-$^{13}$C)= 243Hz).

$^{19}$F NMR (282 MHz, $CDCl_3$): -125.1(C3-F, d, J= 11Hz), -143.4(C5-F, dd, $^1$J= 26Hz, $^2$J= 12Hz), -148.9(C6-F, d, J= 25Hz).

HRMS calcd. for $C_{21}H_{25}F_3N_2O_2S_2$ [$(M+H)^+$]: 459.1382, found: 459.1388.

The compound **3h.** The product was purified by chromatography on a column of silica gel (0.04-0.063mm) with EtAc:$CHCl_3$(1:6), Rf= 0.8. Yield: 0.14g, 54%.

$^1$H NMR (300 MHz, $CDCl_3$): 1.4-1.8(12H, m, cyclooctane), 1.85-2(2H, m, cyclooctane), 2.94(2H, t, J= 8Hz, $SCH_2CH_2$), 3.27(2H, t, J= 8Hz, $SCH_2CH_2$), 3.75-3.9(1H, m, CH of cyclooctane), 5.45(2H, s, $SO_2NH_2$), 7.1-7.4(5H, m, ArH).

$^{13}$C NMR (75 MHz, $CDCl_3$): 23.7(cyclooctane), 25.8(cyclooctane), 27.5(cyclooctane), 33.1(cyclooctane), 35.4($SCH_2CH_2$, t, J($^{19}$F-$^{13}$C)= 4Hz), 36.8($SCH_2CH_2$), 56.3(CH of cyclooctane, d, J($^{19}$F-$^{13}$C) =10Hz), 117(C1, dd, $^1$J($^{19}$F-

$^{13}C$)= 12Hz, $^{2}J(^{19}F-^{13}C)$= 6Hz), 119.8(C4, t, $J(^{19}F-^{13}C)$= 18Hz), 127(Ar), 128.8(Ar), 132.6(C2, d, $J(^{19}F-^{13}C)$= 15Hz), 139.5(Ar), 141.8(C5 or C6, ddd, $^{1}J(^{19}F-^{13}C)$= 240Hz, $^{2}J(^{19}F-^{13}C)$= 16Hz, $^{3}J(^{19}F-^{13}C)$= 5Hz), 145.1(C5 or C6, ddd, $^{1}J(^{19}F-^{13}C)$= 245Hz, $^{2}J(^{19}F-^{13}C)$= 16Hz, $^{3}J(^{19}F-^{13}C)$= 4Hz), 148.5(C3, d, $J(^{19}F-^{13}C)$= 243Hz).

$^{19}F$ NMR (282 MHz, $CDCl_3$): -124.9(C3-F, d, J=11Hz), -143.4(C5-F, dd, $^{1}J$= 27Hz, $^{2}J$= 12Hz), -149.2(C6-F, d, J= 25Hz).

HRMS calcd. for $C_{22}H_{27}F_3N_2O_2S_2$ [$(M+H)^+$]: 473.1539, found: 473.1548

The compound **3i.** The product was purified by chromatography on a column of silica gel (0.04-0.063mm) with EtAc(5%):$CHCl_3$, Rf= 0.65. Yield: 0.12g, 41%, mp 98-99°C.

$^{1}H$ NMR (300 MHz, $CDCl_3$): 1.3-1.8(22H, m, cyclododecane), 2.94(2H, t, J= 8Hz, $SCH_2CH_2$), 3.27(2H, t, J= 8Hz, $SCH_2CH_2$), 3.84(1H, br s, CH of cyclododecane), 5.4(2H, s, $SO_2NH_2$), 7.1-7.4(5H, m, ArH).

$^{13}C$ NMR (75 MHz, $CDCl_3$): 21.3(cyclododecane), 23.4(cyclododecane), 23.5(cyclododecane), 24.3(cyclododecane), 24.6(cyclododecane), 31(cyclododecane), 35.4($SCH_2CH_2$, t, $J(^{19}F-^{13}C)$= 3.7Hz), 36.8($SCH_2CH_2$), 53.6(CH of cyclododecane, d, $J(^{19}F-^{13}C)$= 11Hz), 116.7(C1, dd, $^{1}J(^{19}F-^{13}C)$= 12Hz, $^{2}J(^{19}F-^{13}C)$= 6Hz), 119.8(C4, t, $J(^{19}F-^{13}C)$= 18Hz), 127(Ar), 128.8(Ar), 133.1(C2, d, $J(^{19}F-^{13}C)$= 15Hz), 139.4(Ar), 141.7(C5 or C6, ddd, $^{1}J(^{19}F-^{13}C)$= 237Hz, $^{2}J(^{19}F-^{13}C)$= 16Hz, $^{3}J(^{19}F-^{13}C)$= 5Hz), 145.1(C5 or C6, ddd, $^{1}J(^{19}F-^{13}C)$= 244.5Hz, $^{2}J(^{19}F-^{13}C)$= 12Hz, $^{3}J(^{19}F-^{13}C)$= 4Hz), 148.4(C3, d, $J(^{19}F-^{13}C)$= 242.6Hz).

$^{19}F$ NMR (282 MHz, $CDCl_3$): -120.1(C3-F, d, J= 11Hz), -138.6(C5-F, dd, $^{1}J$= 23Hz, $^{2}J$= 12Hz), -144.7(C6-F, d, J= 26Hz).

HRMS calcd. for $C_{26}H_{35}F_3N_2O_2S_2$ [$(M+H)^+$]: 529.2165, found:529.2164.

The compound **3j.** The product was purified by chromatography on a column of silica gel (0.04-0.063mm) with EtAc(5%):$CHCl_3$, Rf= 0.53. Yield: 0.2g, 71%, mp 118-119°C.

$^{1}H$ NMR (300 MHz, $CDCl_3$): 2.96(2H, t, J= 8Hz, $SCH_2CH_2$), 3.3(2H, t, J= 8Hz, $SCH_2CH_2$), 3.79(6H, s, $2CH_3$), 4.53(2H, d, J= 1.5Hz, $NHCH_2$), 5.25(2H, s, $SO_2NH_2$), 6.55(2H, d, J= 8.4Hz, ArH), 7.2-7.4(6H, m, ArH).

$^{13}C$ NMR (75 MHz, $CDCl_3$): 35.5($SCH_2CH_2$, t, $J(^{19}F-^{13}C)$= 4Hz), 36.8($SCH_2CH_2$), 40.3($NHCH_2$, d, $J(^{19}F-^{13}C)$= 11Hz), 56($2CH_3$), 104(Ar), 115.3(Ar), 119.1(C4, t, $J(^{19}F-^{13}C)$= 19Hz), 119.6(C1, dd, $^{1}J(^{19}F-^{13}C)$= 11Hz, $^{2}J(^{19}F-^{13}C)$= 4Hz), 127(Ar), 128.85(Ar), 128.87(Ar), 129.6(Ar), 133.6(C2, dd, $^{1}J(^{19}F-^{13}C)$= 15Hz, $^{2}J(^{19}F-^{13}C)$= 3.3Hz), 139.5(Ar), 143.3(C5 or C6, ddd, $^{1}J(^{19}F-^{13}C)$= 242Hz, $^{2}J(^{19}F-^{13}C)$= 16Hz, $^{3}J(^{19}F-^{13}C)$= 5Hz), 144.4(C5 or C6, ddd, $^{1}J(^{19}F-^{13}C)$= 251Hz, $^{2}J(^{19}F-^{13}C)$= 16Hz, $^{3}J(^{19}F-^{13}C)$= 4Hz), 150.5(C3, d, $J(^{19}F-^{13}C)$= 245Hz), 158.8(Ar).

$^{19}F$ NMR (282 MHz, $CDCl_3$): -122.4(C3-F, d, J= 12Hz), -144.2(C5-F, dd, $^{1}J$= 24Hz, $^{2}J$= 12Hz), -146.3(C6-F, d, J= 25Hz).

HRMS calcd. for $C_{23}H_{23}F_3N_2O_4S_2$ [$(M+H)^+$]: 513.1124, found: 513.1122.

The compound **3k.** The product was purified by chromatography on a column of silica gel (0.04-0.063mm) with EtAc(5%):$CHCl_3$, Rf= 0.43. Yield: 0.13g, 46%, mp 104-105°C.

$^{1}H$ NMR (300 MHz, $CDCl_3$): 2.87(2H, t, J= 8Hz, $SCH_2CH_2$), 3.22(2H, t, J= 8Hz, $SCH_2CH_2$), 3.81(3H, s, $CH_3$), 3.88(3H, s, $CH_3$), 4.35(2H, d, J= 3.3Hz, $NHCH_2$), 5.41(2H, s, $SO_2NH_2$), 6.76(1H, d, J= 8.1Hz, ArH), 6.83-6.92(2H, m, ArH), 7.14-7.36(5H, m, ArH).

$^{13}C$ NMR (75 MHz, $CDCl_3$): 35.4($SCH_2CH_2$, t, $J(^{19}F-^{13}C)$= 3.5Hz), 36.8($SCH_2CH_2$), 51($NHCH_2$, d, $J(^{19}F-^{13}C)$= 12Hz), 56.06($CH_3$), 56.13($CH_3$), 111.2(Ar), 117.6(C1, dd, $^{1}J(^{19}F-^{13}C)$= 12Hz, $^{2}J(^{19}F-^{13}C)$= 5Hz), 119.8(C4, t, $J(^{19}F-^{13}C)$= 21Hz), 120.4(Ar), 127(Ar), 128.8(Ar), 131.6(Ar), 132.8(C2, d, $J(^{19}F-^{13}C)$= 15Hz), 139.4(Ar), 142.4(C5 or C6, ddd, $^{1}J(^{19}F-^{13}C)$= 240Hz, $^{2}J(^{19}F-^{13}C)$= 16Hz, $^{3}J(^{19}F-^{13}C)$= 5Hz), 144.8(C5 or C6, ddd, $^{1}J(^{19}F-^{13}C)$= 250Hz, $^{2}J(^{19}F-^{13}C)$= 16Hz, $^{3}J(^{19}F-^{13}C)$= 4Hz), 148.6(Ar), 148.9(C3, d, $J(^{19}F-^{13}C)$= 243Hz), 149.3(Ar).

$^{19}F$ NMR (282 MHz, $CDCl_3$): -123.9(C3-F, d, J= 13Hz), -143.5(C5-F, dd, $^{1}J$= 27Hz, $^{2}J$= 12Hz), -147.7(C6-F, d, J= 26Hz).

HRMS calcd. for $C_{23}H_{23}F_3N_2O_4S_2$ [$(M-H)^-$]: 511.0979, found: 511.0982.

The compound **31.** The product was purified by chromatography on a column of silica gel (0.04-0.063mm) with EtAc(10%):$CHCl_3$, Rf= 0.55. Yield: 0.19g, 73%, mp 73-74°C.

$^{1}H$ NMR (300 MHz, $CDCl_3$): 2.85-3.05(4H, m, $SCH_2CH_2$, $CH_2$ of indane), 3.25-3.4(4H, m, $SCH_2CH_2$, $CH_2$ of indane), 4.58-4.7(1H, m, NHCH), 5.12(2H, s, $SO_2NH_2$), 7.2-7.4(9H, m, ArH).

$^{13}C$ NMR (75 MHz, $CDCl_3$): 35.4($SCH_2CH_2$, t, $J(^{19}F-^{13}C)$= 4Hz), 36.9($SCH_2CH_2$), 41.2($CH_2$ of indane), 57.8(NH-CH, d, $J(^{19}F-^{13}C)$= 11Hz), 117.2(C1, dd, $^{1}J(^{19}F-^{13}C)$= 12Hz, $^{2}J(^{19}F-^{13}C)$= 5.5Hz), 120.1(C4, t, $J(^{19}F-^{13}C)$= 22Hz), 125.2(Ar), 127(Ar), 127.2(Ar), 128.8(Ar), 132.2(C2, d, $J(^{19}F-^{13}C)$= 15Hz), 139.4(Ar), 141.1(Ar), 142.2(C5 or C6, ddd, $^{1}J(^{19}F-^{13}C)$= 239Hz, $^{2}J(^{19}F-^{13}C)$= 16Hz, $^{3}J(^{19}F-^{13}C)$= 5Hz), 145(C5 or C6, ddd, $^{1}J(^{19}F-^{13}C)$= 251Hz, $^{2}J(^{19}F-^{13}C)$= 12Hz, $^{3}J(^{19}F-^{13}C)$= 4Hz), 148.2(C3, d, $J(^{19}F-^{13}C)$= 242Hz).

$^{19}F$ NMR (282 MHz, $CDCl_3$): -126(C3-F, d, J=12Hz), -143.3(C5-F, dd, $^{1}J$= 27Hz, $^{2}J$= 12Hz), -148.3(C6-F, d, J= 25Hz).

HRMS calcd. for $C_{23}H_{21}F_3N_2O_2S_2$ [$(M+H)^+$]: 479.1069, found: 479.1077.

The compound **3m.** The product was purified by chromatography on a column of silica gel (0.04-0.063mm) with EtAc(5%):$CHCl_3$, Rf= 0.53. Yield: 0.08g, 31%, mp 101-102°C.

$^1H$ NMR (300 MHz, $CDCl_3$): 2.05(1H, sex, J= 6Hz, indane), 2.56(1H, sex, J= 7Hz, indane), 2.85-2.97(1H, m, indane, signal overlaps with signal of $SCH_2CH_2$), 2.99(2H, t, J= 8Hz, $SCH_2CH_2$), 3.07-3.19(1H, m, indane), 3.33(2H, t, J= 8Hz, $SCH_2CH_2$), 5.18(3H, br s, $SO_2NH_2$, NHCH), 6.34(1H, br s, NH), 7.18-7.4(9H, m, ArH).
$^{13}C$ NMR (75 MHz, $CDCl_3$): 30.3(indane), 35(indane), 35.5($SCH_2CH_2$, t, J($^{19}F$-$^{13}C$)= 3.8Hz), 36.9($SCH_2CH_2$), 62.3(NHCH, d, J($^{19}F$-$^{13}C$)= 11Hz), 117.6(C1, dd, $^1J$($^{19}F$-$^{13}C$)= 12Hz, $^2J$($^{19}F$-$^{13}C$)= 5Hz), 120.1(C4, t, J($^{19}F$-$^{13}C$)= 22Hz), 124.3(Ar), 125.3(Ar), 126.9(Ar), 127(Ar), 128.4(Ar), 128.8(Ar), 132.9(C2, dd, $^1J$($^{19}F$-$^{13}C$)= 15Hz, $^2J$($^{19}F$-$^{13}C$)= 3Hz), 139.4(Ar), 142.5(C5 or C6, ddd, $^1J$($^{19}F$-$^{13}C$)= 240Hz, $^2J$($^{19}F$-$^{13}C$)= 15Hz, $^3J$($^{19}F$-$^{13}C$)= 4Hz), 143.7(Ar), 144.3(Ar), 145(C5 or C6, ddd, $^1J$($^{19}F$-$^{13}C$)= 248Hz, $^2J$($^{19}F$-$^{13}C$)= 16Hz, $^3J$($^{19}F$-$^{13}C$)= 5Hz), 148.8(C3, d, J($^{19}F$-$^{13}C$)= 242Hz).
$^{19}F$ NMR (282 MHz, $CDCl_3$): -124.1(C3-F, d, J= 12Hz), -143.1(C5-F, dd, $^1J$= 26Hz, $^2J$= 12Hz), -147.9(C6-F, d, J= 25Hz).
HRMS calcd. for $C_{23}H_{21}F_3N_2O_2S_2$ [(M+H)$^+$]: 479.1069, found: 479.1063.
The compound **3n.** The product was purified by chromatography on a column of silica gel (0.04-0.063mm) with EtAc(5%):$CHCl_3$, Rf= 0.79. Yield: 0.14g, 52%, mp 123-124°C.
$^1H$ NMR (300 MHz, $CDCl_3$): 1.8-2.1(4H, m, tetrahydronaphthalene), 2.7-2.95(2H, m, tetrahydronaphthalene), 2.99(2H, t, J= 8Hz, $SCH_2CH_2$), 3.33(2H, t, J= 8Hz, $SCH_2CH_2$), 4.83(1H, br s, NHCH), 5.1(2H, s, $SO_2NH_2$), 6.26(1H, br s, NH), 7.1-7.4(9H, m, ArH).
$^{13}C$ NMR (75 MHz, $CDCl_3$): 19(tetrahydronaphthalene), 29.3(tetrahydronaphthalene), 30.3(tetrahydronaphthalene), 35.5($SCH_2CH_2$, t, J($^{19}F$-$^{13}C$)= 4Hz), 36.9($SCH_2CH_2$), 54.7(NHCH, d, J($^{19}F$-$^{13}C$)= 11Hz), 118.2(C1, dd, $^1J$($^{19}F$-$^{13}C$)= 12Hz, $^2J$($^{19}F$-$^{13}C$)= 5Hz), 120(C4, t, J($^{19}F$-$^{13}C$)= 21Hz), 126.2(Ar), 127(Ar), 127.7(Ar), 128.9(Ar), 129.1(Ar), 129.7(Ar), 132.6(C2, d, J($^{19}F$-$^{13}C$)= 15Hz), 137.57(Ar), 137.6(Ar), 139.4(Ar), 142.7(C5 or C6, ddd, $^1J$($^{19}F$-$^{13}C$)= 240Hz, $^2J$($^{19}F$-$^{13}C$)= 16Hz, $^3J$($^{19}F$-$^{13}C$)= 5Hz), 145(C5 or C6, ddd, $^1J$($^{19}F$-$^{13}C$)= 249Hz, $^2J$($^{19}F$-$^{13}C$)= 12Hz, $^3J$($^{19}F$-$^{13}C$)= 4Hz), 149.3(C3, d, J($^{19}F$-$^{13}C$)= 243Hz).
$^{19}F$ NMR (282 MHz, $CDCl_3$): -123.1(C3-F, d, J= 11Hz), -142.9(C5-F, dd, $^1J$= 27Hz, $^2J$= 12Hz), -147.4(C6-F, d, J= 26Hz).
HRMS calcd. for $C_{24}H_{23}F_3N_2O_2S_2$ [(M+H)$^+$]: 493.1226, found: 493.1222.
The compound **3o**. The product was purified by chromatography on a column of silica gel (0.04-0.063mm) with EtAc(10%):$CHCl_3$, Rf= 0.37. Yield: 0.11g, 35%.
$^1H$ NMR (300 MHz, $CDCl_3$): 2.73(2H, t, J= 7.2Hz, $SCH_2CH_2$), 3.08(2H, td, $^1J$= 7.7Hz, $^2J$= 3.3Hz, $SCH_2CH_2$), 5.01(1H, dd, $^1J$= 5.4Hz, $^2J$= 1.8Hz, CH), 5.06(1H, d, J= 5.1Hz, CH), 5.58(2H, s, $SO_2NH_2$), 7.05-7.32(15H, m, ArH).
$^{13}C$ NMR (75 MHz, $CDCl_3$): 35.5($SCH_2CH_2$, t, J($^{19}F$-$^{13}C$)= 3.4Hz), 36.6($SCH_2CH_2$), 66.1(NHCH, d, J($^{19}F$-$^{13}C$)= 11Hz), 77.3(CHOH, signal overlaps with $CDCl_3$ signal), 117.7(C1, dd, $^1J$($^{19}F$-$^{13}C$)= 12Hz, $^2J$($^{19}F$-$^{13}C$)= 5Hz), 119.8(C4, t, J($^{19}F$-$^{13}C$)= 21Hz), 126.8(Ar), 126.9(Ar), 128.2(Ar), 128.3(Ar), 128.5(Ar), 128.7(Ar), 128.8(Ar), 131.6(C2, d, J($^{19}F$-$^{13}C$)= 15Hz), 138.1(Ar), 139.3(Ar), 140.4(Ar), 142.5(C5 or C6, ddd, $^1J$($^{19}F$-$^{13}C$)= 240Hz, $^2J$($^{19}F$-$^{13}C$)= 16Hz, $^3J$($^{19}F$-$^{13}C$)= 5Hz), 144.8(C5 or C6, ddd, $^1J$($^{19}F$-$^{13}C$)= 248Hz, $^2J$($^{19}F$-$^{13}C$)= 12Hz, $^3J$($^{19}F$-$^{13}C$)= 4Hz), 148.6(C3, d, J($^{19}F$-$^{13}C$)= 243Hz).
$^{19}F$ NMR (282 MHz, $CDCl_3$): -122.6(C3-F, d, J= 12Hz), -143.2(C5-F, dd, $^1J$= 25Hz, $^2J$= 12Hz), -147.1(C6-F, d, J= 26Hz).
HRMS calcd. for $C_{28}H_{25}F_3N_2O_3S_2$ [(M+H)$^+$]: 559.1331, found: 559.1331.

**Example 14.** Preparation of 2-(cyclooctylamino)-3,5,6-trifluoro-4-[(2-hydroxyethyl)thio]benzenesulfonamide (compound **4a**), 2-(cyclododecylamino)-3,5,6-trifluoro-4-[(2-hydroxyethyl)thio]benzenesulfonamide (compound **4b**), 2-[(2,6-dimethoxybenzyl)amino]-3,5,6-trifluoro-4-[(2-hydroxyethyl)thio]benzenesulfonamide (compound **4c**), 2-[(3,4-dimethoxybenzyl)amino]-3,5,6-trifluoro-4-[(2-hydroxyethyl)thio]benzenesulfonamide (compound **4d**), 2-[(1S)-2,3-dihydro-1*H*-inden-1-ylamino]-3,5,6-trifluoro-4-[(2-hydroxyethyl)thio] benzenesulfonamide (compound **4e**), 2-[(1S)-1,2,3,4-tetrahydronaphthalen-1-ylamino]-3,5,6-trifluoro-4-[(2-hydroxyethyl)thio]benzenesulfonamide (compound **4f**), 2-{[(1S, 2R)-2-hydroxy-1,2-diphenylethyl]amino}-3,5,6-trifluoro-4-[(2-hydroxyethyl)thio]benzenesulfonamide (compound **4g**).

The mixture of 2,3,5,6-tetrafluoro-4[(2-hydroxyethyl)thio]benzenesulfonamide (compound **2c**) (0.2g, 0.66mmol), $Et_3N$ (0.095mL, 0.68mmol), DMSO (1mL) and appropriate nucleophile (0.68mmol) was stirred at 60°C for 16h. The mixture was then diluted with $H_2O$ (20mL) and extracted with EtAc (3×10mL). The combined organic phase was dried over $MgSO_4$ and evaporated in vacuum.
The compound **4a.** The product was purified by chromatography on a column of silica gel (0.04-0.063mm) with EtAc:$CHCl_3$(1:1), Rf= 0.59. Yield: 0.15g, 56%, mp 68-69°C.
$^1H$ NMR (300 MHz, $CDCl_3$): 1.4-1.75(12H, m, cyclooctane), 1.8-1.95(2H, m, cyclooctane), 2.54(1H, br s, OH), 3.14(2H, t, J= 6Hz, $SCH_2CH_2$), 3.74(2H, t, J= 6Hz, $SCH_2CH_2$), 3.75-3.85(1H, m, CH of cyclooctane, signal overlaps with signal of $SCH_2CH_2$), 5.77(2H, s, $SO_2NH_2$), 6.16(1H, br s, NH).
$^{13}C$ NMR (75 MHz, $CDCl_3$): 23.7(cyclooctane), 25.8(cyclooctane), 27.5(cyclooctane), 33(cyclooctane), 37.5($SCH_2CH_2$, br t), 56.4(CH of cyclooctane, d, J($^{19}F$-$^{13}C$)= 11Hz), 61.2($SCH_2CH_2$), 117.9(C1, dd, $^1J$($^{19}F$-$^{13}C$)= 12Hz,

$^2J(^{19}F-^{13}C)$= 5Hz), 118.3(C4, t, $J(^{19}F-^{13}C)$= 21Hz), 132.7(C2, d, $J(^{19}F-^{13}C)$= 15Hz), 142.1(C5 or C6, ddd, $^1J(^{19}F-^{13}C)$= 240Hz, $^2J(^{19}F-^{13}C)$= 16Hz, $^3J(^{19}F-^{13}C)$= 5Hz), 145.1(C5 or C6, ddd, $^1J(^{19}F-^{13}C)$= 247Hz, $^2J(^{19}F-^{13}C)$= 16Hz, $^3J(^{19}F-^{13}C)$= 4Hz), 149.1(C3, d, $J(^{19}F-^{13}C)$= 243Hz).

$^{19}F$ NMR (282 MHz, $CDCl_3$): -124.5(C3-F, d, J= 11Hz), -143(C5-F, dd, $^1J$= 27Hz, $^2J$= 12Hz), -149(C6-F, d, J= 26Hz).

HRMS calcd. for $C_{16}H_{23}F_3N_2O_3S_2$ [$(M+H)^+$]: 413.1175, found: 413.1175.

The compound **4b.** The product was purified by chromatography on a column of silica gel (0.04-0.063mm) with EtAc:$CHCl_3$(1:1), Rf= 0.65. Yield: 0.17g, 55%, mp 113-114°C.

$^1H$ NMR (300 MHz, $CDCl_3$): 1.3-1.7(22H, m, cyclododecane), 2.4(1H, br s, OH), 3.16(2H, t, J= 6Hz, $SCH_2CH_2$), 3.75(2H, t, J= 6Hz, $SCH_2CH_2$), 3.78-3.86(1H, m, CH cyclododecane, signal overlaps with signal of $SCH_2CH_2$), 5.59(2H, s, $SO_2NH_2$), 6.2(1H, br s, NH).

$^{13}C$ NMR (75 MHz, $CDCl_3$): 21.3(cyclododecane), 23.3(cyclododecane), 23.4(cyclododecane), 24.3(cyclododecane), 24.6(cyclododecane), 30.1(cyclododecane), 37.6($SCH_2CH_2$, t, $J(^{19}F-^{13}C)$= 3Hz), 53.6(CH of cyclododecane, d, $J(^{19}F-^{13}C)$= 11Hz), 61.1($SCH_2CH_2$), 117.5(C1, dd, $^1J(^{19}F-^{13}C)$= 12Hz, $^2J(^{19}F-^{13}C)$= 5Hz), 118.4(C4, t, $J(^{19}F-^{13}C)$= 20Hz), 133.3(C2, d, $J(^{19}F-^{13}C)$= 15Hz), 141.9(C5 or C6, ddd, $^1J(^{19}F-^{13}C)$= 239Hz, $^2J(^{19}F-^{13}C)$= 16Hz, $^3J(^{19}F-^{13}C)$= 5Hz), 145.1(C5 or C6, ddd, $^1J(^{19}F-^{13}C)$= 248Hz, $^2J(^{19}F-^{13}C)$= 16Hz, $^3J(^{19}F-^{13}C)$= 4Hz), 148.9(C3, d, $J(^{19}F-^{13}C)$= 243Hz).

$^{19}F$ NMR (282 MHz, $CDCl_3$): -124.5(C3-F, d, J= 11Hz), -142.9(C5-F, dd, $^1J$= 27Hz, $^2J$= 12Hz), -149.4(C6-F, d, J= 24Hz).

HRMS calcd. for $C_{20}H_{31}F_3N_2O_3S_2$ [$(M+H)^+$]: 469.1801, found: 469.1804.

The compound **4c.** The product was purified by chromatography on a column of silica gel (0.04-0.063mm) with EtAc:$CHCl_3$(1:1), Rf= 0.54. Yield: 0.1g, 34%.

$^1H$ NMR (300 MHz, $CDCl_3$): 3.15(2H, t, J= 6Hz, $SCH_2CH_2$), 3.72(2H, t, J= 6Hz, $SCH_2CH_2$), 3.79(6H, s, $2CH_3$), 4.51(2H, s, $CH_2$), 5.4(2H, br s, $SO_2NH_2$), 6.55(2H, d, J= 8.4Hz, ArH), 7.22(1H, t, J= 8.4Hz, ArH).

$^1H$ NMR (300 MHz, DMSO-$D_6$): 3.09(2H, t, J= 6Hz, $SCH_2CH_2$), 3.57(2H, t, J= 6.6Hz, $SCH_2CH_2$), 3.62(1H, s, OH), 3.75(6H, s, $2CH_3$), 4.44(2H, br s, $CH_2$), 6.5(1H, br s, NH), 6.65(2H, d, J= 8.4Hz, ArH), 7.24(1H, t, J= 8.4Hz, ArH), 7.94(2H, s, $SO_2NH_2$).

$^{13}C$ NMR (75 MHz, $CDCl_3$): 37.4($SCH_2CH_2$, br t), 40.3($NHCH_2$, d, $J(^{19}F-^{13}C)$= 11Hz), 56($CH_3$), 61.2($SCH_2CH_2$), 104(Ar), 115(Ar), 117.9(C4, t, $J(^{19}F-^{13}C)$= 19Hz), 120.3(C1, dd, $^1J(^{19}F-^{13}C)$= 11 Hz, $^2J(^{19}F-^{13}C)$= 4Hz), 129.6(Ar), 133.4(C2, d, $J(^{19}F-^{13}C)$= 16Hz), 143.7(C5 or C6, dd, $^1J(^{19}F-^{13}C)$= 240Hz, $^2J(^{19}F-^{13}C)$= 15Hz), 144.4(C5 or C6, dd, $^1J(^{19}F-^{13}C)$= 248Hz, $^2J(^{19}F-^{13}C)$= 16Hz), 150.9(C3, d, $J(^{19}F-^{13}C)$= 244Hz), 158.8(Ar).

$^{19}F$ NMR (282 MHz, $CDCl_3$): -121.9(C3-F, d, J= 12Hz), -143.7(C5-F, dd, $^1J$= 27Hz, $^2J$= 12Hz), -145.9(C6-F, d, J= 25Hz).

HRMS calcd. for $C_{17}H_{19}F_3N_2O_5S_2$ [$(M+H)^+$]: 453.076, found: 453.0752.

The compound **4d.** The product was purified by chromatography on a column of silica gel (0.04-0.063mm) with EtAc:$CHCl_3$(1:1), Rf= 0.45. Yield: 0.27g, 91%, mp 73-74°C.

$^1H$ NMR (300 MHz, $CDCl_3$): 3.08(2H, t, J= 6Hz, $SCH_2CH_2$), 3.63(2H, t, J= 6Hz, $SCH_2CH_2$), 3.84(3H, s, $CH_3$), 3.86(3H, s, $CH_3$), 4.41(2H, d, J= 3.3Hz, $CH_2$), 5.67(2H, s, $SO_2NH_2$), 6.75-6.87(3H, m, ArH).

$^{13}C$ NMR (75 MHz, $CDCl_3$): 37.4($SCH_2CH_2$, t, $J(^{19}F-^{13}C)$= 3Hz), 51($NHCH_2$, d, $J(^{19}F-^{13}C)$= 12Hz), 56.11($CH_3$), 56.18($CH_3$), 61.1($SCH_2CH_2$), 111.3(Ar), 118.3(C1, dd, $^1J(^{19}F-^{13}C)$= 11Hz, $^2J(^{19}F-^{13}C)$= 5Hz signal overlaps with signal of C4), 118.5(C4, t, $J(^{19}F-^{13}C)$= 21Hz, signal overlaps with signal of C1), 120.4(Ar), 131.5(Ar), 132.9(C2, d, $J(^{19}F-^{13}C)$= 16Hz), 142.6(C5 or C6, ddd, $^1J(^{19}F-^{13}C)$= 242Hz, $^2J(^{19}F-^{13}C)$= 15Hz, $^3J(^{19}F-^{13}C)$= 4Hz), 144.8(C5 or C6, ddd, $^1J(^{19}F-^{13}C)$= 249Hz, $^2J(^{19}F-^{13}C)$= 16Hz, $^3J(^{19}F-^{13}C)$= 5Hz), 148.6(Ar), 149.2(Ar), 149.3(C3, d, $J(^{19}F-^{13}C)$= 243Hz).

$^{19}F$ NMR (282 MHz, $CDCl_3$): -123.6(C3-F, d, J= 12Hz), -143.1(C5-F, dd, $^1J$= 25Hz, $^2J$= 12Hz), -147.6(C6-F, d, J= 26Hz).

HRMS calcd. for $C_{17}H_{19}F_3N_2O_5S_2$[$(M-H)^-$]: 451.0615, found: 451.0621.

The compound **4e.** The product was purified by chromatography on a column of silica gel (0.04-0.063mm) with EtAc:$CHCl_3$(1:1), Rf= 0.71. Yield: 0.13g, 48%.

$^1H$ NMR (300 MHz, $CDCl_3$): 2.02(1H, sex, J= 7.2Hz, indane), 2.54(1H, sex, J= 5.4Hz, indane), 2.88(1H, pent, J= 8Hz, indane), 3.04-3.16(1H, m, indane), 3.19(2H, t, J= 6Hz, $SCH_2CH_2$), 3.77(2H, t, J= 6Hz, $SCH_2CH_2$), 5.12-5.2(1H, m, NHCH), 5.35(2H, s, $SO_2NH_2$), 7.15-7.35(4H, m, ArH).

$^{13}C$ NMR (75 MHz, $CDCl_3$): 30.3(indane), 35(indane), 37.6($SCH_2CH_2$, t, $J(^{19}F-^{13}C)$= 2.5Hz), 61.3($SCH_2CH_2$), 62.2(NHCH, d, $J(^{19}F-^{13}C)$= 10Hz), 118.3(C1, dd, $^1J(^{19}F-^{13}C)$= 12Hz, $^2J(^{19}F-^{13}C)$= 5Hz), 118.8(C4, t, $J(^{19}F-^{13}C)$= 21Hz), 124.2(Ar), 125.3(Ar), 126.9(Ar), 128.4(Ar), 133(C2, d, $J(^{19}F-^{13}C)$= 15Hz), 142.7(C5 or C6, ddd, $^1J(^{19}F-^{13}C)$= 242Hz, $^2J(^{19}F-^{13}C)$= 15Hz, $^3J(^{19}F-^{13}C)$= 4Hz), 143.7(Ar), 144.2(Ar), 145.1(C5 or C6, ddd, $^1J(^{19}F-^{13}C)$= 251Hz, $^2J(^{19}F-^{13}C)$= 12Hz, $^3J(^{19}F-^{13}C)$= 4Hz), 149.2(C3, d, $J(^{19}F-^{13}C)$= 242Hz).

$^{19}F$ NMR (282 MHz, $CDCl_3$): -123.8(C3-F, d, J= 12Hz), -142.7(C5-F, dd, $^1J$= 25Hz, $^2J$= 12Hz), -147.7(C6-F, d, J= 25Hz).

HRMS calcd. for $C_{17}H_{17}F_3N_2O_3S_2$ [$(M+H)^+$]: 419.0705, found: 419.0714.

The compound **4f.** The product was purified by chromatography on a column of silica gel (0.04-0.063mm) with EtAc:$CHCl_3$(1:1), Rf= 0.66. Yield: 0.1g, 36%, mp 94-95°C.

$^1H$ NMR (300 MHz, $CDCl_3$): 1.8-2.05(4H, m, tetrahydronaphthalene), 2.4(1H, br s, OH), 2.7-3(2H, m, tetrahydronaphthalene), 3.19(2H, t, J= 6Hz, $SCH_2CH_2$), 3.78(2H, t, J= 6Hz, $SCH_2CH_2$), 4.76-4.86(1H, m, NHCH), 5.3(2H, br s, $SO_2NH_2$),6.28(1H, d, J= 9Hz, NH), 7.1-7.3(4H, m, ArH).

$^{13}C$ NMR (75 MHz, $CDCl_3$): 18.9(tetrahydronaphthalene), 29.2(tetrahydronaphthalene), 30.3(tetrahydronaphthalene), 37.6($SCH_2CH_2$, br t, J($^{19}F$-$^{13}C$)= 3Hz), 54.7(NHCH, d, J($^{19}F$-$^{13}C$)= 11Hz), 61.3($SCH_2CH_2$), 118.7(C4, t, J($^{19}F$-$^{13}C$)= 22Hz, signal overlaps with signal of C1), 118.9(C1, dd, $^1J$($^{19}F$-$^{13}C$)= 12Hz, $^2J$($^{19}F$-$^{13}C$)= 4Hz, signal overlaps with signal of C4), 126.1(Ar), 127.7(Ar), 129.1(Ar), 129.7(Ar), 132.7(C2, d, J($^{19}F$-$^{13}C$)= 14Hz), 137.48(Ar), 137.54(Ar), 142.9(C5 or C6, ddd, $^1J$($^{19}F$-$^{13}C$)= 240Hz, $^2J$($^{19}F$-$^{13}C$)= 15Hz, $^3J$($^{19}F$-$^{13}C$)= 5Hz), 145.1(C5 or C6, ddd, $^1J$($^{19}F$-$^{13}C$)= 250Hz, $^2J$($^{19}F$-$^{13}C$)= 12Hz, $^3J$($^{19}F$-$^{13}C$)= 5Hz), 149.7(C3, d, J($^{19}F$-$^{13}C$)= 243Hz).

$^{19}F$ NMR (282 MHz, $CDCl_3$): -122.7(C3-F, d, J= 11Hz), -142.5(C5-F, dd, $^1J$= 27Hz, $^2J$= 12Hz), -147.3(C6-F, d, J= 26Hz).

HRMS calcd. for $C_{18}H_{19}F_3N_2O_3S_2$ [(M-H)$^-$]: 431.0716, found: 431.0719.

The compound **4g**. The product was purified by chromatography on a column of silica gel (0.04-0.063mm) with EtAc:$CHCl_3$(1:1), Rf= 0.43. Yield: 0.13g, 39%.

$^1H$ NMR (300 MHz, $CDCl_3$): 2.2(1H, br s, OH), 2.88(2H, t, J= 6Hz, $SCH_2CH_2$), 3.1(1H, br s, OH), 3.35-3.45(2H, m, $SCH_2CH_2$), 4.97(1H, br t, J= 6Hz, CH), 5.05(1H, d, J= 5Hz, CH), 5.97(2H, br s, $SO_2NH_2$), 7-7.3(10H, m, ArH).

$^{13}C$ NMR (75 MHz, $CDCl_3$): 37.1($SCH_2CH_2$, br t, J($^{19}F$-$^{13}C$)= 2Hz), 60.9($SCH_2CH_2$), 66.1(NHCH, d, J($^{19}F$-$^{13}C$)= 12Hz), 77(CHOH, signal overlaps with $CDCl_3$ signal), 118.4(C1, dd, $^1J$($^{19}F$-$^{13}C$)= 11Hz, $^2J$($^{19}F$-$^{13}C$)= 5Hz, signal overlaps with C4 signal), 118.3(C4, t, J($^{19}F$-$^{13}C$)= 20Hz, signal overlaps with C1 signal), 126.8(Ar), 128(Ar), 128.2(Ar), 128.3(Ar), 128.4(Ar), 128.5(Ar), 131.7(C2, d, J($^{19}F$-$^{13}C$)= 15Hz), 138.1(Ar), 140.4(Ar), 142.7(C5 or C6, ddd, $^1J$($^{19}F$-$^{13}C$)= 241Hz, $^2J$($^{19}F$-$^{13}C$)= 15Hz, $^3J$($^{19}F$-$^{13}C$)= 4Hz), 144.9(C5 or C6, ddd, $^1J$($^{19}F$-$^{13}C$)= 251Hz, $^2J$($^{19}F$-$^{13}C$)= 12Hz, $^3J$($^{19}F$-$^{13}C$)= 4Hz), 148.8(C3, d, J($^{19}F$-$^{13}C$)= 243Hz).

$^{19}F$ NMR (282 MHz, $CDCl_3$): -122.2(C3-F, d, J= 11Hz), -142.9(C5-F, dd, $^1J$= 26Hz, $^2J$= 12Hz), -147.3(C6-F, d, J= 26Hz).

HRMS calcd. for $C_{22}H_{21}F_3N_2O_4S_2$ [(M+H)$^+$]: 499.0968, found: 499.0967.

**Example 15.** Preparation of 2-(cyclooctylamino)-3,5,6-trifluoro-4-(propylthio)benzenesulfonamide (compound **5**).

The mixture of 2,3,5,6-tetrafluoro-4-(propylthio)benzenesulfonamide (**2f**) (0.2g, 0.66mmol), $Et_3N$ (0.095mL, 0.68mmol), DMSO (1mL) and cyclooctylamine (0.1mL, 0.72mmol) was stirred at 60°C for 12h. The mixture was then diluted with $H_2O$ (20mL) and extracted with EtAc (3×10mL). The combined organic phase was dried over $MgSO_4$ and evaporated in vacuum.

The compound **5.** The product was purified by chromatography on a column of silica gel (0.04-0.063mm) with EtAc(5%):$CHCl_3$, Rf= 0.64. Yield: 0.16g, 59%.

$^1H$ NMR (300 MHz, $CDCl_3$): 1.03(3H, t, J= 7.2Hz, $CH_3$), 1.4-1.75(14H, m, $CH_2CH_3$, cyclooctane), 1.8-1.95(2H, m, cyclooctane), 2.98(2H, t, J= 7.2Hz, $SCH_2$), 3.7-3.85(1H, m, CH of cyclooctane), 5.62(3H, br s, NH, $SO_2NH_2$).

$^{13}C$ NMR (75 MHz, $CDCl_3$): 13.3($CH_3$), 23.5(cyclooctane), 23.7(cyclooctane), 25.8(cyclooctane), 27.5(cyclooctane), 33($CH_2$), 36.3($SCH_2$, t, J($^{19}F$-$^{13}C$)= 3.6Hz), 56.5(CH of cyclooctane, d, J($^{19}F$-$^{13}C$)= 11Hz), 117.3(C1, dd, $^1J$($^{19}F$-$^{13}C$)= 12Hz, $^2J$($^{19}F$-$^{13}C$)= 6Hz), 120(C4, t, J($^{19}F$-$^{13}C$)= 21Hz), 132.4(C2, d, J($^{19}F$-$^{13}C$)= 13Hz), 142.2(C5 or C6, ddd, $^1J$($^{19}F$-$^{13}C$)= 239Hz, $^2J$($^{19}F$-$^{13}C$)= 16Hz, $^3J$($^{19}F$-$^{13}C$)= 5Hz), 145(C5 or C6, ddd, $^1J$($^{19}F$-$^{13}C$)= 249Hz, $^2J$($^{19}F$-$^{13}C$)= 17Hz, $^3J$($^{19}F$-$^{13}C$)= 4Hz), 148.9(C3, d, J($^{19}F$-$^{13}C$)= 243Hz).

$^{19}F$ NMR (282 MHz, $CDCl_3$): -124.8(C3-F, d, J= 11Hz), -143.5(C5-F, dd, $^1J$= 27Hz, $^2J$= 12Hz), -149(C6-F, d, J= 26Hz).

HRMS calcd. for $C_{17}H_{25}F_3N_2O_2S_2$ [(M+H)$^+$]: 411.1382, found: 411.1388.

**Example 16.** Preparation of 2-(cyclooctylamino)-3,5,6-trifluoro-4-{[2-(4-hydroxyphenyl)ethyl] amino}benzenesulfonamide (compound **6**).

The mixture of 2,3,5,6-tetrafluoro-4-{[2-(4-hydroxyphenyl)ethyl]amino}benzenesulfonamide (**2o**) (0.2g, 0.55mmol), $Et_3N$ (0.085mL, 0.61mmol), DMSO (1mL) and cyclooctylamine (0.085mL, 0.61mmol) was stirred at 70°C for 28h. The mixture was then diluted with $H_2O$ (20mL) and extracted with EtAc (3×10mL). The combined organic phase was dried over $MgSO_4$ and evaporated in vacuum.

The compound **6**. The product was purified by chromatography on a column of silica gel (0.04-0.063mm) with EtAc:$CHCl_3$(1:3), Rf= 0.6. Yield: 0.13g, 50%.

$^1H$ NMR (300 MHz, $CDCl_3$): 1.4-2(14H, m, cyclooctane), 2.82(2H, t, J= 7Hz, $NHCH_2CH_2$), 3.6-3.75(3H, m, CH of cyclooctane, $NHCH_2CH_2$), 5.59(2H, s, $SO_2NH_2$), 6.1(2H, br s, 2NH), 6.79(2H, d, J= 8.4Hz, ArH), 7.04(2H, d, J= 8.4Hz,

ArH).

$^{13}C$ NMR (75 MHz, $CDCl_3$): 23.8(cyclooctane), 25.8(cyclooctane), 27.5(cyclooctane), 32.7(cyclooctane), 36.6($NHCH_2CH_2$), 46.9($NHCH_2CH_2$), 56.4(CH of cyclooctane, d, J($^{19}F$-$^{13}C$)= 10Hz), 106.3(C1, dd, $^1J$($^{19}F$-$^{13}C$)= 12Hz, $^2J$($^{19}F$-$^{13}C$)= 5Hz), 115.8(C4, signal overlaps with Ar signal), 115.83(Ar), 130.2(Ar), 130.28(Ar), 132(C2, d, J($^{19}F$-$^{13}C$)= 13Hz, signal overlaps with C5 or C6 signal), 133.8(C5 or C6, ddd, $^1J$($^{19}F$-$^{13}C$)= 239Hz, $^2J$($^{19}F$-$^{13}C$)= 18Hz, $^3J$($^{19}F$-$^{13}C$)= 6Hz), 138.5(C3, d, J($^{19}F$-$^{13}C$)= 233Hz), 146.5(C5 or C6, ddd, $^1J$($^{19}F$-$^{13}C$)= 243Hz, $^2J$($^{19}F$-$^{13}C$)= 14Hz, $^3J$($^{19}F$-$^{13}C$)= 3Hz), 154.8(Ar).

$^{19}F$ NMR (282 MHz, $CDCl_3$): -144.8(C5-F, dd, $^1J$= 23Hz, $^2J$= 9Hz), -154.1(C3-F, s), - 171.4(C6-F, d, J= 23Hz).

HRMS calcd. for $C_{22}H_{28}F_3N_3O_3S$ [$(M+H)^+$]: 472.1876, found: 472.1877.

**Example 17.** Preparation of 2-(cyclooctylamino)-3,5,6-trifluorobenzenesulfonamide (compound **8a**), 2-(cyclododecylamino)-3,5,6-trifluorobenzenesulfonamide (compound **8b**), 2-[(2,6-dimethoxybenzyl)amino]-3,5,6-trifluorobenzenesulfonamide (compound **8c**), 2-[(3,4-dimethoxybenzyl)amino]-3,5,6-trifluorobenzenesulfonamide (compound **8d**), 2-[(1S)-2,3-dihydro-1*H*-inden-1-ylamino]-3,5,6-trifluorobenzenesulfonamide (compound **8e**), 2-[(1S)-1,2,3,4-tetrahydronaphthalen-1-ylamino]-3,5,6-trifluorobenzenesulfonamide (compound **8f**).

The mixture of 2,3,5,6-tetrafluorobenzenesulfonamide (7) (0.2g, 0.87mmol), $Et_3N$ (0.124mL, 0.89mmol), DMSO (1mL) and appropriate nucleophile (0.93mmol) was stirred at 60°C for 8h, compound **8d** was obtained after 16h , compound **8f** was obtained after stirring at 70°C for 16h. The mixture was then diluted with $H_2O$ (20mL) and extracted with EtAc (3×10mL). The combined organic phase was dried over $MgSO_4$ and evaporated in vacuum.

The compound **8a**. The product was purified by chromatography on a column of silica gel (0.04-0.063mm) with EtAc:$CHCl_3$(1:20), Rf= 0.32. Yield: 0.15g, 52%, mp 117-118°C.

$^1H$ NMR (300 MHz, DMSO-$D_6$): 1.4-1.9(14H, m, cyclooctane), 3.72(1H, br s, CH of cyclooctane), 6.35(1H, br s, NH), 7.65-7.8(1H, m, ArH), 8.1(2H, s, $SO_2NH_2$).

$^{13}C$ NMR (75 MHz, DMSO-$D_6$): 23.6(cyclooctane), 25.7(cyclooctane), 27.6(cyclooctane), 32.8(cyclooctane), 55.7(CH of cyclooctane, d, J($^{19}F$-$^{13}C$)= 10Hz), 110.2(C4, t, J($^{19}F$-$^{13}C$)= 25Hz), 120.6(C1, dd, $^1J$($^{19}F$-$^{13}C$)= 12Hz, $^2J$($^{19}F$-$^{13}C$)= 5Hz), 132.9(C2, dd, $^1J$($^{19}F$-$^{13}C$)= 13Hz, $^2J$($^{19}F$-$^{13}C$)= 3Hz), 141.1(C5 or C6, dt, $^1J$($^{19}F$-$^{13}C$)= 240Hz, $^2J$($^{19}F$-$^{13}C$)= 14Hz), 145(C5 or C6, ddd, $^1J$($^{19}F$-$^{13}C$)= 248Hz, $^2J$($^{19}F$-$^{13}C$)= 14Hz, $^3J$($^{19}F$-$^{13}C$)= 5Hz), 148.3(C3, dd, $^1J$($^{19}F$-$^{13}C$)= 236Hz, $^2J$($^{19}F$-$^{13}C$)= 6Hz).

$^{19}F$ NMR (282 MHz, DMSO-$D_6$): -120.6(C3-F, t, J=13Hz), -133.35:- 133.56(C5-F or C6-F, m), -145.2(C5-F or C6-F, dd, $^1J$= 25Hz, $^2J$= 11Hz).

HRMS calcd. for $C_{14}H_{19}F_3N_2O_2S$ [$(M+H)^+$]: 337.1192, found: 337.1195.

The compound **8b**. The product was purified by chromatography on a column of silica gel (0.04-0.063mm) with EtAc:$CHCl_3$(1:20), Rf= 0.49. Yield: 0.1g, 29%, mp 113-114°C.

$^1H$ NMR (300 MHz, DMSO-$D_6$): 1.2-1.7(22H, m, cyclododecane), 3.71(1H, br s, CH of cyclododecane), 6.22(1H, d= 7.8Hz, NH), 7.64-7.78(1H, m, ArH), 8.09(2H, s, $SO_2NH_2$).

$^{13}C$ NMR (75 MHz, DMSO-$D_6$): 21.4(cyclododecane), 23.4(cyclododecane), 23.6(cyclododecane), 24.1 (cyclododecane), 24.4(cyclododecane), 31.1 (cyclododecane), 52.8(CH of cyclododecane, d, J($^{19}F$-$^{13}C$)= 11Hz), 110.2(C4, t, J($^{19}F$-$^{13}C$)= 25Hz), 120.6(C1, dd, $^1J$($^{19}F$-$^{13}C$)= 12Hz, $^2J$($^{19}F$-$^{13}C$)= 5Hz), 133.4(C2, dd, $^1J$($^{19}F$-$^{13}C$)= 12Hz, $^2J$($^{19}F$-$^{13}C$)= 2Hz), 141.1(C5 or C6, dt, $^1J$($^{19}F$-$^{13}C$)= 238Hz, $^2J$($^{19}F$-$^{13}C$)= 13Hz), 145(C5 or C6, ddd, $^1J$($^{19}F$-$^{13}C$)= 249Hz, $^2J$($^{19}F$-$^{13}C$)= 14Hz, $^3J$($^{19}F$-$^{13}C$)= 4Hz), 148.1(C3, dd, $^1J$($^{19}F$-$^{13}C$)= 245Hz, $^2J$($^{19}F$-$^{13}C$)= 9Hz).

$^{19}F$ NMR (282 MHz, DMSO-$D_6$): -125.4(C3-F, t, J= 13Hz), -138:- 138.3(C5-F or C6-F, m), -150.1(C5-F or C6-F, dd, $^1J$= 25Hz, $^2J$= 11Hz).

HRMS calcd. for $C_{18}H_{27}F_3N_2O_2S$ [$(M+H)^+$]: 393.1818, found: 393.1816.

The compound **8c.** The product was purified by chromatography on a column of silica gel (0.04-0.063mm) with EtAc(5%):$CHCl_3$, Rf= 0.4. Yield: 0.16g, 48%, mp 137-138°C.

$^1H$ NMR (300 MHz, $D_3OD$): 3.79(6H, s, $2CH_3$), 4.51(2H, d, J= 1.5Hz, $CH_2$), 4.91(2H, s, $SO_2NH_2$),6.61(2H, d, J= 8.4Hz, ArH), 7.22(1H, t, J= 8.1Hz, ArH), 7.29-7.41(1H, m, ArH).

$^1H$ NMR (300 MHz, DMSO-$D_6$): 3.75(6H, s, $2CH_3$), 4.44(2H, d, J= 2.1Hz, $CH_2$), 6.65(2H, d, J= 8.4Hz, ArH), 7.25(1H, t, J= 8.4Hz, ArH), 7.68-7.8(1H, m, ArH), 7.92(2H, s, $SO_2NH_2$).

$^1H$ NMR (300 MHz, $CDCl_3$): 3.81(6H, s, $2CH_3$), 4.56(2H, br s, $CH_2$), 4.92(2H, br s, $SO_2NH_2$),6.05(1H, br s, NH), 6.57(2H, d, J= 8.4Hz, ArH), 7.06-7.17(1H, m, ArH), 7.24(1H, t, J= 8.4Hz, ArH).

$^{13}C$ NMR (75 MHz, $D_3OD$): 39.3($CH_2$, d, J($^{19}F$-$^{13}C$)= 12Hz), 55($CH_3$), 103.6(Ar), 108.7(C4, t, J($^{19}F$-$^{13}C$)= 25Hz), 115.1(Ar), 121.4(C1, dd, $^1J$($^{19}F$-$^{13}C$)= 12Hz, $^2J$($^{19}F$-$^{13}C$)= 4Hz), 129.2(Ar), 134.3(C2, dd, $^1J$($^{19}F$-$^{13}C$)= 13Hz, $^2J$($^{19}F$-$^{13}C$)= 3Hz), 142.2(C5 or C6, d, J($^{19}F$-$^{13}C$)= 241Hz), 144.6(C5 or C6, d, J($^{19}F$-$^{13}C$)= 248Hz), 149.9(C3, d, J($^{19}F$-$^{13}C$)= 249Hz), 158.8(Ar).

$^{19}F$ NMR (282 MHz, DMSO-$D_6$): -124.1(C3-F, t, J= 13Hz), -141.5:- 141.7(C5-F or C6-F, m), -150.3(C5-F or C6-F, dd, $^1J$= 25Hz, $^2J$= 10Hz).

HRMS calcd. for $C_{15}H_{15}F_3N_2O_4S$ [(M-H)$^-$]: 375.0632, found: 375.0634.

The compound **8d.** The product was purified by chromatography on a column of silica gel (0.04-0.063mm) with EtAc(10%):$CHCl_3$, Rf= 0.29. Recrystallization was accomplished from EtOH:$H_2O$(2:1) after chromatography. Yield: 0.1g, 33%, mp 115-116°C.

$^1$H NMR (300 MHz, DMSO-$D_6$): 3.73(3H, s, $CH_3$), 3.75(3H, s, $CH_3$), 4.4(2H, dd, J= 6Hz, J= 3.6Hz, $CH_2$), 6.7(1H, td, J= 6Hz, J= 2Hz, NH), 6.82-6.98(3H, m, ArH), 7.65-7.78(1H, m, ArH), 8.14(2H, s, $SO_2NH_2$).

$^{13}$C NMR (75 MHz, DMSO-$D_6$): 50.3($CH_2$, d, J($^{19}$F-$^{13}$C)= 12Hz), 55.96($CH_3$), 56.06($CH_3$), 110.9(C4, t, J($^{19}$F-$^{13}$C)= 24Hz), 111.9(Ar), 112.2(Ar), 120.3(Ar), 120.4(C1, signal overlaps with Ar signal), 132.3(Ar), 133.5(C2, d, J($^{19}$F-$^{13}$C)= 13Hz), 141.2(C5 or C6, dt, $^1$J($^{19}$F-$^{13}$C)= 238Hz, $^2$J($^{19}$F-$^{13}$C)= 12Hz), 144.8(C5 or C6, d, J($^{19}$F-$^{13}$C)= 252Hz), 148.2(C3, d J($^{19}$F-$^{13}$C)= 240Hz), 148.6(Ar), 149.2(Ar).

$^{19}$F NMR (282 MHz, DMSO-$D_6$): -124.9(C3-F, t, J= 13Hz), -138.6:- 138.8(C5-F or C6-F, m), -149.6(C5-F or C6-F, dd, $^1$J= 25Hz, $^2$J= 10Hz).

HRMS calcd. for $C_{15}H_{15}F_3N_2O_4S$ [(M-H)$^-$]: 375.0632, found: 375.0631.

The compound **8e**. The product was purified by chromatography on a column of silica gel (0.04-0.063mm) with EtAc:$CHCl_3$(1:10), Rf= 0.38. Yield: 0.09g, 30%, mp 103-104°C.

$^1$H NMR (300 MHz, DMSO-$D_6$): 1.79-1.95(1H, m, indane), 2.38-2.5(1H, m, indane), 2.82(1H, pent, J= 8Hz, indane), 2.91-3.06(1H, m, indane), 5.1-5.2(1H, m, NHCH), 6.59(1H, dd, $^1$J= 8.6Hz, $^2$J=2Hz, NH), 7.2-7.4(4H, m, ArH), 7.75-7.9(1H, m ArH), 8.13(2H, s, $SO_2NH_2$).

$^{13}$C NMR (75 MHz, DMSO-$D_6$): 30.2(indane), 35.2(indane), 61.6(CH of indane, d, J($^{19}$F-$^{13}$C)= 11Hz), 110.5(C4, t, J($^{19}$F-$^{13}$C)= 25Hz), 120.5(C1, dd, $^1$J($^{19}$F-$^{13}$C)= 12Hz, $^2$J($^{19}$F-$^{13}$C)= 5Hz), 124.7(Ar), 125.5(Ar), 127.3(Ar), 128.6(Ar), 133.2(C2, dd, $^1$J($^{19}$F-$^{13}$C)= 13Hz, $^2$J($^{19}$F-$^{13}$C)= 3Hz), 141.4(C5 or C6, dt, $^1$J($^{19}$F-$^{13}$C)= 238Hz, $^2$J($^{19}$F-$^{13}$C)= 14Hz), 143.6(Ar), 144.7(Ar), 144.9(C5 or C6, ddd, $^1$J($^{19}$F-$^{13}$C)= 233Hz, $^2$J($^{19}$F-$^{13}$C)= 14Hz, $^3$J($^{19}$F-$^{13}$C)= 4Hz), 148.2(C3, d, J($^{19}$F-$^{13}$C)= 226Hz).

$^{19}$F NMR (282 MHz, DMSO-$D_6$): -120(C3-F, t, J= 13Hz), -133.35:- 133.55(C5-F or C6-F, m), -144.7(C5-F or C6-F, dd, $^1$J= 25Hz, $^2$J= 11Hz).

HRMS calcd. for $C_{15}H_{13}F_3N_2O_2S$ [(M-H)$^-$]: 341.0577, found: 341.0580.

The compound **8f**. The product was purified by chromatography on a column of silica gel (0.04-0.063mm) with EtAc(10%):$CHCl_3$, Rf= 0.64. Yield: 0.1g, 32%, mp 124-125°C.

$^1$H NMR (300 MHz, DMSO-$D_6$): 1.6-2(4H, m, tetrahydronaphthalene), 2.6-2.9(2H, m, tetrahydronaphthalene), 4.81(1H, br s, NHCH), 6.53(1H, d, J= 8.7Hz, NH), 7.1-7.25(3H, m, ArH), 7.41(1H, d, J= 7.5Hz, ArH), 7.73-7.85(1H, m, ArH), 8.12(2H, s, $SO_2NH_2$).

$^{13}$C NMR (75 MHz, DMSO-$D_6$): 19.3(tetrahydronaphthalene), 29.3(tetrahydronaphthalene), 30.2(tetrahydronaphthalene), 53.8(CH of tetrahydronaphthalene, d, J($^{19}$F-$^{13}$C)= 12Hz), 110.4(C4, t, J($^{19}$F-$^{13}$C)= 25Hz), 121(C1, dd, $^1$J($^{19}$F-$^{13}$C)= 12Hz, $^2$J($^{19}$F-$^{13}$C)= 5Hz), 126.6(Ar), 127.9(Ar), 129.6(Ar), 129.7(Ar), 132.7(C2, dd, $^1$J($^{19}$F-$^{13}$C)= 12Hz, $^2$J($^{19}$F-$^{13}$C)= 2Hz), 137.6(Ar), 137.9(Ar), 141.5(C5 or C6, dt, $^1$J($^{19}$F-$^{13}$C)= 239Hz, $^2$J($^{19}$F-$^{13}$C)= 13Hz), 145(C5 or C6, d, J($^{19}$F-$^{13}$C)= 247Hz), 148.4(C3, d, J($^{19}$F-$^{13}$C)= 239Hz).

$^{19}$F NMR (282 MHz, DMSO-$D_6$): -123.8(C3-F, t, J= 13Hz), -137.9:- 138.14(C5-F or C6-F, m), -149.2(C5-F or C6-F, dd, $^1$J= 25Hz, $^2$J= 10Hz).

HRMS calcd. for $C_{16}H_{15}F_3N_2O_2S$ [(M-H)$^-$]: 355.0734, found: 355.0733.

**Example 18**. Preparation of 3-(methylamino)-2,5,6-trifluoro-4-[(2-phenylethyl)sulfonyl]benzenesulfonamide (compound **9a**).

The mixture of 2,3,5,6-tetrafluoro-4-[(2-phenylethyl)sulfonyl]benzenesulfonamide (compound **2q**) (0.2g, 0.5mmol), MeOH (10mL) and methylamine (2M in methanol) (0.75mL, 1.5mmol) was refluxed for 7h. MeOH was evaporated in vacuum and the resultant precipitate was filtered, washed with $H_2O$. Recrystallization was accomplished from EtOH:$H_2O$ (2:1). Yield: 0.12g, 57%, mp 152-153°C.

$^1$H NMR (300 MHz, DMSO-$D_6$): 3.01(3H, dd, $^1$J= 7.5Hz, $^2$J= 5Hz, $CH_3$), 3.08(2H, t, J= 7.5Hz, $SO_2CH_2CH_2$), 3.89(2H, t, J= 7Hz, $SO_2CH_2CH_2$), 6.6(1H, br s, NH), 7.1-7.3(5H, m, ArH), 8.31(2H, s, $SO_2NH_2$).

$^{13}$C NMR (75 MHz, DMSO-$D_6$): 28.6($SO_2CH_2CH_2$), 34.1($CH_3$, d, J($^{19}$F-$^{13}$C)= 13Hz), 57.5($SO_2CH_2CH_2$), 114.4(C4, dd, $^1$J($^{19}$F-$^{13}$C)= 12Hz, $^2$j($^{19}$F-$^{13}$C)= 5Hz), 127.5(Ar), 128(C1, t, J($^{19}$F-$^{13}$C)= 18Hz), 129(Ar), 129.1(Ar), 137.4(C3, d, J($^{19}$F-$^{13}$C)= 12Hz), 137.5(Ar), 136.7(C5 or C6, d, J($^{19}$F-$^{13}$C)= 244Hz), 144.4(C2, d, J($^{19}$F-$^{13}$C)= 251Hz), 146.1(C5 or C6, d, J($^{19}$F-$^{13}$C)= 240Hz).

$^{19}$F NMR (282 MHz, DMSO-$D_6$): -127.5(C2-F, s), -135.9(C6-F, dd, $^1$J= 25Hz, $^2$J= 12Hz), -152.6(C5-F, dd, $^1$J= 26Hz, $^2$J= 7Hz).

HRMS calcd. for $C_{15}H_{15}F_3N_2O_4S_2$ [(M+H)$^+$]: 409.0498, found: 409.0505.

**Example 19.** Preparation of 3-(benzylamino)-2,5,6-trifluoro-4-[(2-phenylethyl)sulfonyl]benzenesulfonamide (compound **9c**).

The mixture of 2,3,5,6-tetrafluoro-4-[(2-phenylethyl)sulfonyl]benzenesulfonamide (compound **2q**) (0.3g, 0.75mmol), $Et_3N$ (0.109mL, 0.78mmol), MeOH (10mL) and benzylamine (0.085mL, 0.78mmol) was refluxed for 18h. MeOH was evaporated in vacuum and the resultant precipitate was filtered, washed with $H_2O$. Recrystallization was accomplished from EtOH. Yield: 0.21g, 57%, mp 59-61°C.

$^1$H NMR (300 MHz, DMSO-$D_6$): 3.0(2H, t, J= 8Hz, $SO_2CH_2CH_2$), 3.82(2H, t, J= 7Hz, $SO_2CH_2CH_2$), 4.54(2H, dd, J= 5.9Hz, J= 4Hz, $NHCH_2$), 7.01(1H, t, J= 6Hz, NH), 7.1-7.5(10H, m, ArH), 8.4(2H, br s, $SO_2NH_2$).

$^{13}$C NMR (75 MHz, DMSO-$D_6$): 28.5($SO_2CH_2CH_2$), 50.5($NHCH_2$, d, J($^{19}$F-$^{13}$C)= 13Hz), 57.7($SO_2CH_2CH_2$), 115.6(C4, dd, $^1$J($^{19}$F-$^{13}$C)= 13Hz, $^2$J($^{19}$F-$^{13}$C)= 5Hz), 127.5(Ar), 128.2(Ar), 128.4(Ar), 129(Ar), 129.1(Ar), 129.3(Ar), 136(C3, d, J($^{19}$F-$^{13}$C)= 14Hz), 137.5(Ar), 139.7(Ar), 139.4(C5 or C6, d, J($^{19}$F-$^{13}$C)= 244Hz), 144.9(C2, d, J($^{19}$F-$^{13}$C)= 253Hz), 146(C5 or C6, d, J($^{19}$F-$^{13}$C)= 253Hz).

$^{19}$F NMR (282 MHz, DMSO-$D_6$): -124.7(C2-F, s), -134.9(C6-F, dd, $^1$J= 25Hz, $^2$J= 12Hz), -150.4(C5-F, dd, $^1$J= 26Hz, $^2$J= 7Hz).

HRMS calcd. for $C_{21}H_{19}F_3N_2O_4S_2$ [$(M+H)^+$]: 485.0811, found: 485.0814.

**Example 20.** Preparation of 3-(*tert*-butylamino)-2,5,6-trifluoro-4-[(2-phenylethyl)sulfonyl]benzenesulfonamide (compound **9b**), 3-morpholin-4-yl-2,5,6-trifluoro-4-[(2-phenylethyl)sulfonyl]benzenesulfonamide (compound **9e**).

The mixture of 2,3,5,6-tetrafluoro-4-[(2-phenylethyl)sulfonyl]benzenesulfonamide (compound **2q**) (0.2g, 0.5mmol), DMSO 1mL) and appropriate nucleophile (1.02mmol) was stirred at ambient temperature for 4 days. The mixture was then diluted with $H_2O$ (20mL) and the resultant precipitate was filtered, washed with $H_2O$.

The compound **9b**. The product was purified by chromatography on a column of silica gel (0.04-0.063mm) with EtAc:$CHCl_3$(1:4), Rf= 0.62. Yield: 0.04g, 18%, mp 127°C.

$^1$H NMR (300 MHz, $CDCl_3$): 1.38(9H, d, J= 2Hz, $3CH_3$), 3.14(2H, t, J= 8Hz, $SO_2CH_2CH_2$), 3.65(2H, t, J= 8Hz, $SO_2CH_2CH_2$), 5.74(2H, s, $SO_2NH_2$),6.63(1H, s, NH), 7.1-7.4(5H, m, ArH).

$^{13}$C NMR (75 MHz, $CDCl_3$): 28.8($SO_2CH_2CH_2$), 30.9($CH_3$, d, J= 7Hz), 55.7($SO_2CH_2CH_2$), 58.8(NHC, d, J($^{19}$F-$^{13}$C)= 4Hz), 118.5(C4, dd, $^1$J($^{19}$F-$^{13}$C)= 12Hz, $^2$J($^{19}$F-$^{13}$C)= 6Hz), 126.3(C1, t, J($^{19}$F-$^{13}$C)= 16Hz), 127.6(Ar), 128.5(Ar), 129.2(Ar), 135.8(C3, dd, $^1$J($^{19}$F-$^{13}$C)= 18Hz, $^2$J($^{19}$F-$^{13}$C)= 3Hz), 136.6(Ar), 138.3(C5 or C6, ddd, $^1$J($^{19}$F-$^{13}$C)= 252Hz, $^2$J($^{19}$F-$^{13}$C)= 18Hz, $^3$J($^{19}$F-$^{13}$C)= 5Hz), 145.8(C2, d, J($^{19}$F-$^{13}$C)= 254Hz), 146.1(C5 or C6, ddd, $^1$J($^{19}$F-$^{13}$C)= 253Hz, $^2$J($^{19}$F-$^{13}$C)= 16Hz, $^3$J($^{19}$F-$^{13}$C)= 4Hz).

$^{19}$F NMR (282 MHz, $CDCl_3$): -122.2(C2-F, s), -137.6(C6-F, dd, $^1$J= 25Hz, $^2$J= 12Hz), - 152.9(C5-F, dd, $^1$J= 26Hz, $^2$J= 6Hz).

HRMS calcd. for $C_{18}H_{21}F_3N_2O_4S_2$ [$(M+H)^+$]: 451.0968, found: 451.0969.

The compound **9e**. Recrystallization was accomplished from EtOH. Yield: 0.11g, 46%, mp 198-199°C.

$^1$H NMR (300 MHz, DMSO-$D_6$): 2.9(2H, d, J= 11Hz, morpholine), 3.12(2H, t, J= 8Hz, $SO_2CH_2CH_2$), 3.21(2H, t, J= 11Hz, morpholine), 3.57(2H, t, J= 11Hz, morpholine), 3.79(2H, d, J= 11Hz, morpholine), 4.06(2H, t, J= 8Hz, $SO_2CH_2CH_2$), 7.2-7.4(5H, m, ArH), 8.48(2H, s, $SO_2NH_2$).

$^{13}$C NMR (75 MHz, DMSO-$D_6$): 33.3($SO_2CH_2CH_2$), 56.6(morpholine, d, J($^{19}$F-$^{13}$C)= 4Hz), 62.3($SO_2CH_2CH_2$), 71.8(morpholine), 132.2(Ar), 133.8(Ar), 134.1(Ar), 136.1(C1, t, J($^{19}$F-$^{13}$C)= 6Hz), 139.7(C4, dd, $^1$J($^{19}$F-$^{13}$C)=16Hz, $^2$J($^{19}$F-$^{13}$C)= 5Hz), 142.9(Ar), 150.7(C5, C6, dd, $^1$J($^{19}$F-$^{13}$C)= 261Hz, $^2$J($^{19}$F-$^{13}$C)= 17Hz), 159.3(C2, d, J($^{19}$F-$^{13}$C)= 259Hz).

$^{19}$F NMR (282 MHz, DMSO-$D_6$): -119.1(C2-F, d, J= 14Hz), -132.4(C5-F, d, J= 25Hz), - 136.7(C6-F, dd, $^1$J= 25Hz, $^2$J= 14Hz).

HRMS calcd. for $C_{18}H_{19}F_3N_2O_5S_2$ [$(M+H)^+$]: 465.076, found: 465.0765.

**Example 21**. Preparation of 3-[(2-phenylethyl)amino]-2,5,6-trifluoro-4-[(2-phenylethyl)sulfonyl]benzenesulfonamide (compound **9d**), 3-(cyclooctylamino)-2,5,6-trifluoro-4-[(2-phenylethyl)sulfonyl]benzenesulfonamide (compound **9f**), 3-(cyclododecylamino)-2,5,6-trifluoro-4-[(2-phenylethyl)sulfonyl]benzenesulfonamide (compound **9g**), 3-[(2,6-dimethoxybenzyl)amino]-2,5,6-trifluoro-4-[(2-phenylethyl)sulfonyl]benzenesulfonamide (compound **9h**), 3-[(3,4-dimethoxybenzyl)amino]-2,5,6-trifluoro-4-[(2-phenylethyl)sulfonyl]benzenesulfonamide (compound **9i**), 3-(2,3-dihydro-1*H*-inden-2-ylamino)-2,5,6-trifluoro-4-[(2-phenylethyl)sulfonyl]benzenesulfonamide (compound **9k**), 3-[(1S)-2,3-dihydro-1*H*-inden-1-ylamino]-2,5,6-trifluoro-4-[(2-phenylethyl)sulfonyl]benzenesulfonamide (compound **9l**), 3-[(1S)-1,2,3,4-tetrahydronaphthalen-1-ylamino]-2,5,6-trifluoro-4-[(2-phenylethyl)sulfonyl]benzenesulfonamide (compound **9m**).

The mixture of 2,3,5,6-tetrafluoro-4-[(2-phenylethyl)sulfonyl]benzenesulfonamide (compound **2q**) (0.2g, 0.5mmol), $Et_3N$ (0.071mL, 0.51mmol), DMSO (1mL) and appropriate nucleophile (0.51mmol) was stirred at ambient

temperature for 24h. The mixture was then diluted with $H_2O$ (20mL) and extracted with EtAc (3×10mL). The combined organic phase was dried over $MgSO_4$ and evaporated in vacuum.

The compound **9d**. The product was purified by chromatography on a column of silica gel (0.04-0.063mm) with EtAc:$CHCl_3$(1:6), Rf= 0.48. Yield: 0.18g, 72%, mp 141-142°C.

$^1H$ NMR (300 MHz, DMSO-$D_6$): 2.88(2H, t, J= 7Hz, $NHCH_2CH_2$), 2.98(2H, t, J= 7Hz, $SO_2CH_2CH_2$), 3.6(2H, br t, $NHCH_2CH_2$), 3.77(2H, t, J= 8Hz, $SO_2CH_2CH_2$), 6.68(1H, br s, NH), 7.1-7.4(10H, m, ArH), 8.33(2H, s, $SO_2NH_2$).

$^{13}C$ NMR (75 MHz, DMSO-$D_6$): 28.6($SO_2CH_2CH_2$), 36.8($NHCH_2CH_2$), 48.3($NHCH_2CH_2$, d, J($^{19}F$-$^{13}C$)= 13Hz), 57.5($SO_2CH_2CH_2$), 114.6(C4, d, J($^{19}F$-$^{13}C$)= 12Hz), 127.1(Ar), 127.5(Ar), 128.1(C1, t, J($^{19}F$-$^{13}C$)= 16Hz), 128.9(Ar), 129(Ar), 129.2(Ar), 129.5(Ar), 136.1(C3, d, J($^{19}F$-$^{13}C$)= 13Hz), 137.5(Ar), 139.3(Ar), 137(C5 or C6, d, J($^{19}F$-$^{13}C$)= 244Hz), 144.3(C2, d, J($^{19}F$-$^{13}C$)= 250Hz), 145.7(C5 or C6, d, J($^{19}F$-$^{13}C$)= 233Hz).

$^{19}F$ NMR (282 MHz, DMSO-$D_6$): -127(C2-F, s), -135.3(C6-F, dd, $^1J$= 27Hz, $^2J$= 12Hz), -152(C5-F, dd, $^1J$= 26Hz, $^2J$= 7Hz).

HRMS calcd. for $C_{22}H_{21}F_3N_2O_4S_2$ [$(M+H)^+$]: 499.0968, found: 499.0971.

The compound **9f**. The product was purified by chromatography on a column of silica gel (0.04-0.063mm) with EtAc:$CHCl_3$(1:9), Rf= 0.5. Yield: 0.22g, 88%, mp 90-92°C.

$^1H$ NMR (300 MHz, $CDCl_3$): 1.4-2(14H, m, cyclooctane), 3.14(2H, t, J= 8Hz, $SO_2CH_2CH_2$), 3.64(2H, t, J= 8Hz, $SO_2CH_2CH_2$), 3.85-3.95(1H, m, cyclooctane), 5.68(2H, s, $SO_2NH_2$),6.91(1H, d, J= 8.7Hz, NH), 7.1-7.4(5H, m, ArH).

$^{13}C$ NMR (75 MHz, $CDCl_3$): 23.5(cyclooctane), 25.7(cyclooctane), 27.5(cyclooctane), 28.7($SO_2CH_2CH_2$), 33.2(cyclooctane), 56.3(CH of cyclooctane, d, J($^{19}F$-$^{13}C$)= 11Hz), 58.8($SO_2CH_2CH_2$, d, J($^{19}F$-$^{13}C$)= 4Hz), 114.7(C4, dd, $^1J$($^{19}F$-$^{13}C$)= 12Hz, $^2J$($^{19}F$-$^{13}C$)= 7Hz), 126.4(C1, t, J($^{19}F$-$^{13}C$)= 16Hz), 127.6(Ar), 128.5(Ar), 129.1(Ar), 136(C3, d, J($^{19}F$-$^{13}C$)= 13Hz), 136.5(Ar), 136.7(C5 or C6, d, J($^{19}F$-$^{13}C$)= 251Hz), 145.8(C2, d, J($^{19}F$-$^{13}C$)= 252Hz), 146.2(C5 or C6, dd, $^1J$($^{19}F$-$^{13}C$)= 252Hz, $^2J$($^{19}F$-$^{13}C$)= 16Hz).

$^{19}F$ NMR (282 MHz, $CDCl_3$): -131(C2-F, s), -138.2(C6-F, dd, $^1J$= 25Hz, $^2J$= 12Hz), - 156.9(C5-F, dd, $^1J$= 26Hz, $^2J$= 7Hz).

HRMS calcd. for $C_{22}H_{27}F_3N_2O_4S_2$ [$(M+H)^+$]: 505.1437, found: 505.1439.

The compound **9g**. The product was purified by chromatography on a column of silica gel (0.04-0.063mm) with EtAc:$CHCl_3$ (1:10), Rf= 0.37. Yield: 0.13g, 46%, mp130-131°C.

$^1H$ NMR (300 MHz, DMSO-$D_6$): 1.2-1.7(22H, m, cyclododecane), 3.07(2H, t, J= 8Hz, $SO_2CH_2CH_2$), 3.8(1H, br s, CH of cyclododecane), 3.88(2H, t, J= 7Hz, $SO_2CH_2CH_2$), 6.55(1H, d, J= 8Hz, NH), 7.1-7.3(5H, m, ArH), 8.36(2H, s, $SO_2NH_2$).

$^{13}C$ NMR (75 MHz, DMSO-$D_6$): 21.2(cyclododecane), 23.3(cyclododecane), 23.4(cyclododecane), 24.5(cyclododecane), 24.7(cyclododecane), 28.5($SO_2CH_2CH_2$), 30.8(cyclododecane), 53.5(CH of cyclododecane, d, J($^{19}F$-$^{13}C$)= 12Hz), 58($SO_2CH_2CH_2$), 115.4(C4, dd, $^1J$($^{19}F$-$^{13}C$)= 13Hz, $^2J$($^{19}F$-$^{13}C$)= 4Hz), 127.5(Ar), 128.2(C1, t, J($^{19}F$-$^{13}C$)= 16Hz), 129(Ar), 135.8(C3, d, J($^{19}F$-$^{13}C$)= 16Hz), 137.6(Ar), 137.5(C5 or C6, dd, $^1J$($^{19}F$-$^{13}C$)= 246Hz, $^2J$($^{19}F$-$^{13}C$)= 17Hz), 144.7(C2, d, J($^{19}F$-$^{13}C$)= 250Hz), 146.2(C5 or C6, dd, $^1J$($^{19}F$-$^{13}C$)= 249Hz, $^2J$($^{19}F$-$^{13}C$)= 17Hz).

$^{19}F$ NMR (282 MHz, DMSO-$D_6$): -125.4(C2-F, s), -134.5(C6-F, dd, $^1J$= 27Hz, $^2J$= 12Hz), -151(C5-F, dd, $^1J$= 27Hz, $^2J$= 6Hz).

HRMS calcd. for $C_{26}H_{35}F_3N_2O_4S_2$ [$(M+H)^+$]: 561.2063, found: 561.2071.

The compound **9h.** The product was purified by chromatography on a column of silica gel (0.04-0.063mm) with EtAc:$CHCl_3$ (1:5), Rf= 0.47. Yield: 0.13g, 48%, mp 133-137°C.

$^1H$ NMR (300 MHz, DMSO-$D_6$): 2.76(2H, t, J= 8Hz, $SO_2CH_2CH_2$), 3.47(2H, t, J= 8Hz, $SO_2CH_2CH_2$), 3.74(6H, s, $2CH_3$), 4.52(2H, d, J= 5.4Hz, $NHCH_2$), 6.62(2H, d, J= 8.4Hz, ArH) 6.69(1H, br t, NH), 7.05-7.3(6H, m, ArH), 8.4(2H, s, $SO_2NH_2$).

$^{13}C$ NMR (75 MHz, DMSO-$D_6$): 28.2($SO_2CH_2CH_2$), 39.4($NHCH_2$, d, J($^{19}F$-$^{13}C$)= 13Hz, signal overlaps with signal of DMSO), 56.4($CH_3$), 57.6($SO_2CH_2CH_2$), 104.7(Ar), 114.7(Ar), 116.5(C4, dd, $^1J$($^{19}F$-$^{13}C$)= 13Hz, $^2J$($^{19}F$-$^{13}C$)= 5Hz), 127.4(Ar), 127.9(C1, t, J($^{19}F$-$^{13}C$)= 16Hz), 129(Ar), 129.1(Ar), 130.4(Ar), 136.9(C3, d, J($^{19}F$-$^{13}C$)= 13Hz), 137.5(Ar), 138.1(C5 or C6, d $^1J$($^{19}F$-$^{13}C$)= 251Hz), 145.5(C5 or C6, d, J($^{19}F$-$^{13}C$)= 253Hz), 146.1(C2, d, J($^{19}F$-$^{13}C$)= 254Hz), 158.7(Ar).

$^{19}F$ NMR (282 MHz, DMSO-$D_6$): -121.8(C2-F, dd, $^1J$= 11Hz, $^2J$= 5Hz), -135.5(C6-F, dd, $^1J$= 27Hz, $^2J$= 12Hz), -149.6(C5-F, dd, $^1J$= 27Hz, $^2J$= 5Hz).

HRMS calcd. for $C_{23}H_{23}F_3N_2O_6S_2$ [$(M-H)^-$]: 543.0877, found: 543.0881.

The compound **9i.** Recrystallization was accomplished from EtOH. Yield: 0.18g, 67%, mp 167-168°C.

$^1H$ NMR (300 MHz, DMSO-$D_6$): 2.95(2H, t, J= 7.8Hz, $SO_2CH_2CH_2$), 3.69(3H, s, $CH_3$), 3.73(3H, s, $CH_3$), 3.8(2H, t, J= 7.8Hz, $SO_2CH_2CH_2$), 4.45(2H, t, J= 4.7Hz, $NHCH_2$), 6.8-7(4H, m, ArH, NH), 7.1-7.3(5H, m, ArH), 8.38(2H, s, $SO_2NH_2$).

$^{13}C$ NMR (75 MHz, DMSO-$D_6$): 28.5($SO_2CH_2CH_2$), 50.3($NHCH_2$, d, J($^{19}F$-$^{13}C$)= 12.5Hz), 56.02($CH_3$), 56.08($CH_3$), 57.7($SO_2CH_2CH2$), 112.31(Ar), 112.34(Ar), 115.8(C4, dd, $^1J$($^{19}F$-$^{13}C$)= 13Hz, $^2J$($^{19}F$-$^{13}C$)= 5Hz), 120.8(Ar), 127.5(Ar), 128.1(C1, t, J($^{19}F$-$^{13}C$)= 16Hz), 128.97(Ar), 129.03(Ar), 131.9(Ar), 136(C3, d, J($^{19}F$-$^{13}C$)= 14Hz), 137.5(Ar),

137.7(C5 or C6, dd, $^1J(^{19}F-^{13}C)$= 249Hz, $^2J(^{19}F-^{13}C)$= 18Hz), 145.1(C2, d, $J(^{19}F-^{13}C)$= 256Hz), 146(C5 or C6, d, $J(^{19}F-^{13}C)$= 250Hz), 148.9(Ar), 149.4(Ar).

$^{19}F$ NMR (282 MHz, DMSO-$D_6$): -123.7(C2-F, s), -134.8(C6-F, dd, $^1J$= 27Hz, $^2J$= 12Hz), -150.4(C5-F, dd, $^1J$= 27Hz, $^2J$= 6Hz).

HRMS calcd. for $C_{23}H_{23}F_3N_2O_6S_2$ [(M-H)$^-$]: 543.0877, found: 543.0875.

The compound **9k.** The product was purified by chromatography on a column of silica gel (0.04-0.063mm) with EtAc:$CHCl_3$(1:4), Rf= 0.6. Recrystallization was accomplished from EtOH after chromatography. Yield: 0.12g, 45%, mp 155°C.

$^1H$ NMR (300 MHz, DMSO-$D_6$): 2.8-3(4H, m, $SO_2CH_2CH_2$ and $CH_2$ of indane), 3.26(1H, d, J= 6.3Hz, indane), 3.31(1H, d, J= 6.3Hz, indane), 3.67(2H, t, J= 8Hz, $SO_2CH_2CH_2$), 4.45-4.55(1H, m, CH of indane), 6.87(1H, d, J= 8Hz, NH), 7.1-7.3(10H, m, ArH), 8.38(2H, s, $SO_2NH_2$).

$^{13}C$ NMR (75 MHz, DMSO-$D_6$): 28.4($SO_2CH_2CH_2$), 41.1($CH_2$ of indane, signal overlaps with signal of DMSO-$D_6$), 57.58($SO_2CH_2CH_2$), 57.7(CH of indane, d, $J(^{19}F-^{13}C)$= 11Hz), 115.3(C4, dd, $^1J(^{19}F-^{13}C)$= 13Hz, $^2J(^{19}F-^{13}C)$= 5Hz), 125.4(Ar), 127.38(Ar), 127.45(Ar), 128.2(C1, t, $J(^{19}F-^{13}C)$= 16Hz), 129(Ar), 129.1(Ar), 135.4(C3, d, $J(^{19}F-^{13}C)$= 14Hz), 137.5(Ar), 137.6(C5 or C6, dd, $^1J(^{19}F-^{13}C)$= 247Hz, $^2J(^{19}F-^{13}C)$= 17Hz), 141.1(Ar), 144.4(C2, d, $J(^{19}F-^{13}C)$= 252Hz), 146.2(C5 or C6, dd, $^1J(^{19}F-^{13}C)$= 250Hz, $^2J(^{19}F-^{13}C)$= 16Hz).

$^{19}F$ NMR (282 MHz, DMSO-$D_6$): -126.1(C2-F, s), -134.7(C6-F, dd, $^1J$= 27Hz, $^2J$= 12Hz), -150.8(C5-F, dd, $^1J$= 27Hz, $^2J$= 7Hz).

HRMS calcd. for $C_{23}H_{21}F_3N_2O_4S_2$ [(M+H)$^+$]: 511.0968, found: 511.0972.

The compound **9l.** The product was purified by chromatography on a column of silica gel (0.04-0.063mm) with EtAc(5%):$CHCl_3$, Rf= 0.38. Yield: 0.17g, 66%, mp90°C.

$^1H$ NMR (300 MHz, DMSO-$D_6$): 2.0(1H, sex, J= 6Hz, indane), 2.5(1H, sex, indane, signal overlaps with signal of DMSO-$D_6$), 2.8-3.2(4H, m, $CH_2$ of indane, $SO_2CH_2CH_2$), 3.7-3.9(2H, m, $SO_2CH_2CH_2$), 5.18(1H, br s, indane), 6.89(1H, d, J= 8Hz, NH), 7.1-7.4(9H, m, ArH), 8.43(2H, s, $SO_2NH_2$).

$^{13}C$ NMR (75 MHz, DMSO-$D_6$): 28.3($SO_2CH_2CH_2$), 30.2(indane), 35.3(indane), 57.8($SO_2CH_2CH_2$), 61.7(CH of indane, d, $J(^{19}F-^{13}C)$= 11.4Hz), 115.6(C4, dd, $^1J(^{19}F-^{13}C)$=14Hz, $^2J(^{19}F-^{13}C)$= 5Hz), 124.6(Ar), 125.7(Ar), 127.4(Ar), 127.5(Ar), 128.2(C1, t, $J(^{19}F-^{13}C)$= 16Hz), 128.9(Ar), 129(Ar), 129.1(Ar), 135.7(C3, d, $J(^{19}F-^{13}C)$= 15Hz), 137.5(Ar), 137.8(C5 or C6, d, $J(^{19}F-^{13}C)$= 246Hz), 143.7(Ar), 144.2(Ar), 144.8(C2, d, $J(^{19}F-^{13}C)$= 252Hz), 146.1(C5 or C6, d, $J(^{19}F-^{13}C)$= 251Hz).

$^{19}F$ NMR (282 MHz, DMSO-$D_6$): -124.2(C2-F, s), -134.5(C6-F, dd, $^1J$= 27Hz, $^2J$= 12Hz), -150.2(C5-F, dd, $^1J$= 27Hz, $^2J$= 6Hz).

HRMS calcd. for $C_{23}H_{21}F_3N_2O_4S_2$ [(M+H)$^+$]: 511.0968, found: 511.0964.

The compound **9m.** The product was purified by chromatography on a column of silica gel (0.04-0.063mm) with EtAc:$CHCl_3$ (1:10), Rf= 0.37. Yield: 0.13g, 50%, mp 116-119°C.

$^1H$ NMR (300 MHz, DMSO-$D_6$): 1.7-2.1(4H, m, tetrahydronaphthalene), 2.6-2.9(2H, m, tetrahydronaphthalene), 2.94(2H, t, J= 7.8Hz, $SO_2CH_2CH_2$), 3.74(2H, t, J= 7.7Hz, $SO_2CH_2CH_2$), 4.8-4.9(1H, m, CH of tetrahydronaphthalene), 6.82(1H, d, J= 9Hz, NH), 7.1-7.4(9H, m, ArH), 8.41(2H, s, $SO_2NH_2$).

$^{13}C$ NMR (75 MHz, DMSO-$D_6$): 19.1(tetrahydronaphthalene), 28.3($SO_2CH_2CH_2$), 29.1(tetrahydronaphthalene), 30.5(tetrahydronaphthalene), 54.1(CH of tetrahydronaphthalene, d, $J(^{19}F-^{13}C)$= 12Hz), 58($SO_2CH_2CH_2$), 116.1(C4, dd, $^1J(^{19}F-^{13}C)$= 13Hz, $^2J(^{19}F-^{13}C)$= 5Hz), 126.8(Ar), 127.5(Ar), 128.2(Ar), 128.2(C1, t, $J(^{19}F-^{13}C)$= 18Hz, signal overlaps with signal of Ar), 129.03(Ar), 129.08(Ar), 129.5(Ar), 129.9(Ar), 135.3(C3, d, $J(^{19}F-^{13}C)$= 11Hz), 137.41(Ar), 137.48(Ar), 137.57(Ar), 138(C5 or C6, d, $J(^{19}F-^{13}C)$= 238Hz), 145.1(C2, d, $J(^{19}F-^{13}C)$= 254Hz), 146.1(C5 or C6, d, $J(^{19}F-^{13}C)$= 254Hz).

$^{19}F$ NMR (282 MHz, DMSO-$D_6$): -123.5(C2-F, s), -134.3(C6-F, dd, $^1J$= 27Hz, $^2J$= 12Hz), -149.9(C5-F, dd, $^1J$= 27Hz, $^2J$= 5Hz).

HRMS calcd. for $C_{24}H_{23}F_3N_2O_4S_2$ [(M-H)$^-$]: 523.0979, found: 523.0983.

**Example 22.** Preparation of 3-(1-adamantylamino)-2,5,6-trifluoro-4-[(2-phenylethyl)sulfonyl]benzenesulfonamide (compound **9j**), 3-{[(1S, 2R)-2-hydroxy-1,2-diphenylethyl]amino}-2,5,6-trifluoro-4-[(2-phenylethyl)sulfonyl]benzenesulfonamide (compound **9n**), 3-{[(1R, 2S)-2-hydroxy-1,2-diphenylethyl]amino}-2,5,6-trifluoro-4-[(2-phenylethyl)sulfonyl]benzenesulfonamide (compound **9o**).

The mixture of 2,3,5,6-tetrafluoro-4-[(2-phenylethyl)sulfonyl]benzenesulfonamide (compound **2q**) (0.2g, 0.5mmol), $Et_3N$ (0.071mL, 0.51mmol), DMSO (1mL) and appropriate nucleophile (0.52mmol) was stirred at ambient temperature for 3 days, compound **9j** was obtained after stirring for 5 days. The mixture was then diluted with $H_2O$ (20mL).

The compound **9j.** The resultant precipitate was filtered, washed with $H_2O$. The product was purified by chromatography on a column of silica gel (0.04-0.063mm) with EtAc:$CHCl_3$(1:4), Rf= 0.75. Yield: 0.02g, 8%, mp 155-156°C.

$^1H$ NMR (300 MHz, $CDCl_3$): 1.69(6H, br s, adamantane), 1.91(6H, br s, adamantane), 2.15(3H, br s, adamantane), 3.16(2H, t, J= 6Hz, $SO_2CH_2CH_2$), 3.67(2H, t, J= 7Hz, $SO_2CH_2CH_2$), 5.53(2H, s, $SO_2NH_2$),6.41(1H, s, NH), 7.1-7.4(5H, m, ArH).

$^{13}C$ NMR (75 MHz, $CDCl_3$): 28.7($SO_2CH_2CH_2$), 30.2(adamantane), 36.2(adamantane), 43.4(adamantane), 43.5(adamantane), 56.6($SO_2CH_2CH_2$), 58.9(adamantane, d, J($^{19}F$-$^{13}C$)= 4Hz), 119.6(C4, dd, $^1J$($^{19}F$-$^{13}C$)= 12Hz, $^2J$($^{19}F$-$^{13}C$)= 6Hz), 126(C1, t, J($^{19}F$-$^{13}C$)= 16Hz), 127.6(Ar), 128.5(Ar), 129.2(Ar), 135.3(C3, dd, $^1J$($^{19}F$-$^{13}C$)= 18Hz, $^2J$($^{19}F$-$^{13}C$)= 3Hz), 137(Ar), 139(C5 or C6, d, J($^{19}F$-$^{13}C$)= 252Hz), 146(C5 or C6, d, J($^{19}F$-$^{13}C$)= 254Hz), 146.4(C2, d, J($^{19}F$-$^{13}C$)= 253Hz).

$^{19}F$ NMR (282 MHz, $CDCl_3$): -120.3(C2-F, s), -137.6(C6-F, dd, $^1J$= 25Hz, $^2J$= 12Hz), - 152(C5-F, dd, $^1J$= 26Hz, $^2J$= 6Hz).

HRMS calcd. for $C_{24}H_{27}F_3N_2O_4S_2$ [(M+H)$^+$]: 529.1437, found: 529.1440.

The compound **9n**. The mixture was then extracted with EtAc (3×10mL). The combined organic phase was dried over $MgSO_4$ and evaporated in vacuum. Recrystallization was accomplished from EtOH:$H_2O$= 2:1. Yield: 0.12g, 40%, mp 175-176°C.

$^1H$ NMR (300 MHz, DMSO-$D_6$): 3.06(2H, t, J= 7.2Hz, $SO_2CH_2CH_2$), 3.75-3.95(2H, m, $SO_2CH_2CH_2$), 4.9-5(1H, m, CH), 5.1(1H, d, J= 4.5Hz, CH), 6(1H, br s, OH), 7.1-7.3(15H, m, ArH), 7.87(1H, d, J= 9Hz, NH), 8.3(2H, s, $SO_2NH_2$).

$^{13}C$ NMR (75 MHz, DMSO-$D_6$): 28.5($SO_2CH_2CH_2$), 58($SO_2CH_2CH_2$), 65.4(CH, d, J($^{19}F$-$^{13}C$)= 12.8Hz), 75.6(CH), 115.3(C4, dd, $^1J$($^{19}F$-$^{13}C$)= 13Hz, $^2J$($^{19}F$-$^{13}C$)= 6Hz), 127.2(Ar), 127.4(Ar), 127.8(Ar), 127.9(Ar), 128.2(Ar), 128.3(Ar), 128.93(Ar), 128.99(Ar), 129.04(Ar), 135.2(C3, d, J($^{19}F$-$^{13}C$)= 12Hz), 137.2(C5 or C6, d, J($^{19}F$-$^{13}C$)= 250Hz), 137.5(Ar), 139.7(Ar), 142.9(Ar), 144.5(C2, d, J($^{19}F$-$^{13}C$)= 254Hz), 146(C5 or C6, d, J($^{19}F$-$^{13}C$)= 249Hz).

$^{19}F$ NMR (282 MHz, DMSO-$D_6$): -123.3(C2-F, s), -134.7(C6-F, dd, $^1J$= 25Hz, $^2J$= 12Hz), -150.8(C5-F, dd, $^1J$= 26Hz, $^2J$= 7Hz).

HRMS calcd. for $C_{28}H_{25}F_3N_2O_5S_2$ [(M+H)$^+$]: 591.123, found: 591.1220.

The compound **9o**. The resultant precipitate was filtered, washed with $H_2O$. Recrystallization was accomplished from EtOH:$H_2O$= 2:1. Yield: 0.12g, 40%, mp 176-177°C.

$^1H$ NMR (300 MHz, DMSO-$D_6$): 3.06(2H, t, J= 7.2Hz, $SO_2CH_2CH_2$), 3.75-3.95(2H, m, $SO_2CH_2CH_2$), 4.9-5(1H, m, CH), 5.1(1H, t, J= 4.2Hz, CH), 6(1H, d, J= 4.2Hz, OH), 7.1-7.3(15H, m, ArH), 7.87(1H, d, J= 8Hz, NH), 8.25(2H, s, $SO_2NH_2$).

$^{13}C$ NMR (75 MHz, DMSO-$D_6$): 28.5($SO_2CH_2CH_2$), 58($SO_2CH_2CH_2$), 65.4(CH, d, J($^{19}F$-$^{13}C$)= 12.8Hz), 75.6(CH), 115.3(C4, dd, $^1J$($^{19}F$-$^{13}C$)= 13Hz, $^2J$($^{19}F$-$^{13}C$)= 6Hz), 127.2(Ar), 127.4(Ar), 127.8(Ar), 127.9(Ar), 128.2(Ar), 128.3(Ar), 128.93(Ar), 128.99(Ar), 129.04(Ar), 135.2(C3, d, J($^{19}F$-$^{13}C$)= 12Hz), 137.2(C5 or C6, d, J($^{19}F$-$^{13}C$)= 250Hz), 137.5(Ar), 139.7(Ar), 142.9(Ar), 144.5 (C2, d, J($^{19}F$-$^{13}C$)= 254Hz), 146(C5 or C6, d, J($^{19}F$-$^{13}C$)= 249Hz).

$^{19}F$ NMR (282 MHz, DMSO-$D_6$): -123.3(C2-F, s), -134.7(C6-F, dd, $^1J$= 25Hz, $^2J$= 12Hz), -150.8(C5-F, dd, $^1J$= 26Hz, $^2J$= 7Hz).

HRMS calcd. for $C_{28}H_{25}F_3N_2O_5S_2$ [(M+H)$^+$]: 591.123, found: 591.1221.

**Example 23.** Preparation of 3-(benzylamino)-2,5,6-trifluoro-4-[(2-hydroxyethyl)sulfonyl]benzenesulfonamide (compound **10a**).

The mixture of 2,3,5,6-tetrafluoro-4-[(2-hydroxyethyl)sulfonyl]benzenesulfonamide (compound **2d**) (0.25g, 0.74mmol), MeOH (10mL) and benzylamine (0.17mL, 1.56mmol) was stirred at ambient temperature for 24h. MeOH was evaporated in vacuum and the resultant precipitate was filtered, washed with $H_2O$. The product was purified by chromatography on a column of silica gel (0.04-0.063mm) with EtAc:$CHCl_3$ (1:2), Rf= 0.19. Yield: 0.11g, 35%, mp 127°C.

$^1H$ NMR (300 MHz, DMSO-$D_6$): 3.65(2H, t, J= 5.4Hz, $SO_2CH_2CH_2$), 3.83(2H, k, J= 5.4Hz, $SO_2CH_2CH_2$), 4.45-4.55(2H, m, $NHCH_2$), 5.03(1H, t, J= 5.1Hz, OH), 6.96(1H, br t, NH), 7.3-7.5(5H, m, ArH), 8.36(2H, s, $SO_2NH_2$).

$^{13}C$ NMR (75 MHz, DMSO-$D_6$): 50.7($NHCH_2$, d, J($^{19}F$-$^{13}C$)= 13Hz), 55.8($SO_2CH_2CH_2$), 60.1($SO_2CH_2CH_2$), 117.6(C4, dd, $^1J$($^{19}F$-$^{13}C$)= 13Hz, $^2J$($^{19}F$-$^{13}C$)= 5Hz), 127.9(C1 signal overlaps with signal of Ar), 128.1(Ar), 128.3(Ar), 129.3(Ar), 136.1(C3, d, J($^{19}F$-$^{13}C$)= 13Hz), 137.8(C5 or C6, d, J($^{19}F$-$^{13}C$)= 246Hz), 139.6(Ar), 144.9(C2, d, J($^{19}F$-$^{13}C$)= 252Hz), 146.2(C5 or C6, d, J($^{19}F$-$^{13}C$)= 252Hz).

$^{19}F$ NMR (282 MHz, DMSO-$D_6$): -125.1(C2-F, s), -135.3(C6-F, dd, $^1J$= 25Hz, $^2J$= 13Hz), -150.7(C5-F, dd, $^1J$= 26Hz, $^2J$= 7Hz).

HRMS calcd. for $C_{15}H_{15}F_3N_2O_5S_2$ [(M+H)$^+$]: 425.0447, found: 425.0439.

**Example 24.** Preparation of 3-(cyclooctylamino)-2,5,6-trifluoro-4-[(2-hydroxyethyl)sulfonyl]benzenesulfonamide (compound **10b**).

The mixture of 2,3,5,6-tetrafluoro-4-[(2-hydroxyethyl)sulfonyl]benzenesulfonamide (compound **2d**) (0.38g, 1.1mmol), MeOH (10mL) and cyclooctylamine (0.332mL, 2.4mmol) was refluxed for 6h. MeOH was evaporated in vacuum and the resultant oil was washed with $H_2O$. The product was purified by chromatography on a column of silica gel

(0.04-0.063mm) with EtAc:$CHCl_3$ (1:1), Rf= 0.38. Yield: 0.2g, 40%.

$^1$H NMR (300 MHz, $CDCl_3$): 1.4-2(14H, m, cyclooctane), 3.59(2H, t, J= 6Hz, $SO_2CH_2CH_2$), 3.8-3.9(1H, m, CH of cyclooctane), 4.11(2H, t, J= 6Hz, $SO_2CH_2CH_2$), 6.08(2H, s, $SO_2NH_2$), 6.74(1H, br s, NH).

$^{13}$C NMR (75 MHz, $CDCl_3$): 23.5(cyclooctane), 25.7(cyclooctane), 27.4(cyclooctane), 33.1(cyclooctane), 56.3(cyclooctane), 56.5($SO_2CH_2CH_2$), 59.8(cyclooctane), 60.8($SO_2CH_2CH_2$), 115.8(C4, dd, $^1J(^{19}F-^{13}C)$= 13Hz, $^2J(^{19}F-^{13}C)$= 6Hz), 126.5(C1, t, $J(^{19}F-^{13}C)$= 16Hz), 135.7(C3, d, $J(^{19}F-^{13}C)$= 13Hz), 137(C5 or C6, dd, $^1J(^{19}F-^{13}C)$= 246Hz, $^2J(^{19}F-^{13}C)$= 14Hz), 144.7(C2, d, $J(^{19}F-^{13}C)$= 253Hz), 146.4(C5 or C6, dd, $^1J(^{19}F-^{13}C)$= 253Hz, $^2J(^{19}F-^{13}C)$= 16Hz).

$^{19}$F NMR (282 MHz, $CDCl_3$): -125.9(C2-F, s), -134(C6-F, dd, $^1J$= 25Hz, $^2J$= 12Hz),-152.1(C5-F, dd, $^1J$= 24Hz, $^2J$= 4Hz).

HRMS calcd. for $C_{16}H_{23}F_3N_2O_5S_2$ [(M+H)$^+$]: 445.1073, found: 445.1077.

**Example 25.** Preparation of 3-(cyclododecylamino)-2,5,6-trifluoro-4-[(2-hydroxyethyl)sulfonyl]benzenesulfonamide (compound **10c**), 3-[(2,6-dimethoxybenzyl)amino]-2,5,6-trifluoro-4-[(2-hydroxyethyl)sulfonyl]benzenesulfonamide (compound **10d**), 3-[(3,4-dimethoxybenzyl)amino]-2,5,6-trifluoro-4-[(2-hydroxyethyl)sulfonyl]benzenesulfonamide (compound **10e**), 3-[(1S)-2,3-dihydro-1*H*-inden-1-ylamino]-2,5,6-trifluoro-4-[(2-hydroxyethyl)sulfonyl]benzenesulfonamide (compound **10f**), 3-[(1S)-1,2,3,4-tetrahydronaphthalen-1-ylamino]-2,5,6-trifluoro-4-[(2-hydroxyethyl)sulfonyl]benzenesulfonamide (compound **10g**), 3-{[(1S, 2R)-2-hydroxy-1,2-diphenylethyl]amino}-2,5,6-trifluoro-4-[(2-hydroxyethyl)sulfonyl]benzenesulfonamide (compound **10h**).

The mixture of 2,3,5,6-tetrafluoro-4-[(2-hydroxyethyl)sulfonyl]benzenesulfonamide (compound **2d**) (0.2g, 0.59mmol), DMSO (1mL) and appropriate nucleophile (1.2mmol) was stirred at ambient temperature for 24h. The mixture was then diluted with $H_2O$ (20mL) and extracted with EtAc (3×10mL). The combined organic phase was dried over $MgSO_4$ and evaporated in vacuum.

The compound **10c**. The product was purified by chromatography on a column of silica gel (0.04-0.063mm) with EtAc:$CHCl_3$ (1:1), Rf= 0.5. Yield: 0.26g, 88%, mp 143-144°C.

$^1$H NMR (300 MHz, DMSO-$D_6$): 1.2-1.7(22H, m, cyclododecane), 3.68(2H, t, J= 5Hz, $SO_2CH_2CH_2$), 3.8(1H, br s, CH of cyclododecane, signal overlaps with signal of $SO_2CH_2CH_2$), 3.83(2H, t, J= 5Hz, $SO_2CH_2CH_2$), 5.01(1H, t, J= 5Hz, OH), 6.55(1H, d, J= 9Hz, NH), 8.36(2H, s, $SO_2NH_2$).

$^{13}$C NMR (75 MHz, DMSO-$D_6$): 21.3(cyclododecane), 23.4(cyclododecane), 23.5(cyclododecane), 24.4(cyclododecane), 24.6(cyclododecane), 30.8(cyclododecane), 53.4(CH of cyclododecane, d, $J(^{19}F-^{13}C)$= 12Hz), 55.8($SO_2CH_2CH_2$), 60.3($SO_2CH_2CH_2$), 117.4(C4, dd, $^1J(^{19}F-^{13}C)$= 13Hz, $^2J(^{19}F-^{13}C)$= 6Hz), 127.9(C1, t, $J(^{19}F-^{13}C)$= 16Hz), 135.7(C3, d, $J(^{19}F-^{13}C)$= 13Hz), 137.4(C5 or C6, dd, $^1J(^{19}F-^{13}C)$= 246Hz, $^2J(^{19}F-^{13}C)$= 19Hz), 144.7(C2, d, $J(^{19}F-^{13}C)$= 253Hz), 146.3(C5 or C6, d, $J(^{19}F-^{13}C)$= 247Hz).

$^{19}$F NMR (282 MHz, DMSO-$D_6$): -125.4(C2-F, s), -134.8(C6-F, dd, $^1J$= 27Hz, $^2J$= 12Hz), -151.4(C5-F, dd, $^1J$= 27Hz, $^2J$= 6Hz).

HRMS calcd. for $C_{20}H_{31}F_3N_2O_5S_2$ [(M+H)$^+$]: 501.1699, found: 501.1701.

The compound **10d.** The product was purified by chromatography on a column of silica gel (0.04-0.063mm) with EtAc(60%):$CHCl_3$, Rf= 0.45. Yield: 0.15g, 52%, mp 164-165°C.

$^1$H NMR (300 MHz, DMSO-$D_6$): 3.35(2H, t, J= 6Hz, $SO_2CH_2CH_2$), 3.63(2H, br t, $SO_2CH_2CH_2$), 3.75(6H, s, $2CH_3$), 4.48(2H, d, J= 5.4Hz, $NHCH_2$), 4.93(1H, br s, OH), 6.58(1H, br t, NH), 6.66(2H, d, J= 8.4Hz, ArH), 7.26(1H, t, J= 8.4Hz, ArH), 8.42(2H, s, $SO_2NH_2$).

$^{13}$C NMR (75 MHz, DMSO-$D_6$): 39.5($NHCH_2$), 55.4($SO_2CH_2CH_2$), 56.3($2CH_3$), 59.9($SO_2CH_2CH_2$), 104.7(Ar), 114.6(Ar), 118.2(C4, dd, $^1J(^{19}F-^{13}C)$= 12Hz, $^2J(^{19}F-^{13}C)$= 5Hz), 127.7(C1, t, $J(^{19}F-^{13}C)$= 16Hz), 130.3(Ar), 136.8(C3, d, $J(^{19}F-^{13}C)$= 11Hz), 137.9(C5 or C6, d, $^1J(^{19}F-^{13}C)$= 228Hz), 146(C2, d, $J(^{19}F-^{13}C)$= 253Hz), 144.8(C5 or C6, d, $J(^{19}F-^{13}C)$= 242Hz), 158.7(Ar).

$^{19}$F NMR (282 MHz, DMSO-$D_6$): -122.1(C2-F, s), -135.9(C6-F, dd, $^1J$= 26Hz, $^2J$= 13Hz), -150.1(C5-F, dd, $^1J$= 27Hz, $^2J$= 6Hz).

HRMS calcd. for $C_{17}H_{19}F_3N_2O_7S_2$ [(M-H)$^-$]: 483.0513, found: 483.0517.

The compound **10e**. The product was purified by chromatography on a column of silica gel (0.04-0.063mm) with EtAc:$CHCl_3$ (2:1), Rf= 0.38. Recrystallization was accomplished from EtOH after chromatography. Yield: 0.1g, 29%, mp 164-165°C.

$^1$H NMR (300 MHz, DMSO-$D_6$): 3.65(2H, t, J= 6Hz, $SO_2CH_2CH_2$), 3.746(3H, s, $CH_3$), 3.755(3H, s, $CH_3$), 3.82(2H, br t, $SO_2CH_2CH_2$), 4.43(2H, br s, $NHCH_2$), 6.8-7(3H, m, ArH), 6.99(1H, s, NH), 8.38(2H, s, $SO_2NH_2$).

$^{13}$C NMR (75 MHz, DMSO-$D_6$): 50.6($NHCH_2$, d, $J(^{19}F-^{13}C)$= 12Hz), 55.8($CH_3$), 56($CH_3$), 56.1($SO_2CH_2CH_2$), 60.1($SO_2CH_2CH_2$), 112.2(Ar), 112.3(Ar), 117.7(C4, dd, $^1J(^{19}F-^{13}C)$-13Hz, $^2J(^{19}F-^{13}C)$= 5Hz), 120.7(Ar), 127.8(C1, t, $J(^{19}F-^{13}C)$= 16Hz), 131.8(Ar), 136(C3, d, $J(^{19}F-^{13}C)$= 16Hz), 137.8(C5 or C6, d, $J(^{19}F-^{13}C)$= 252Hz), 145.1(C2, d, $J(^{19}F-^{13}C)$= 253Hz), 146.1(C5 or C6, dd, $^1Je^9F-^{13}C)$= 253Hz, $^2J(^{19}F-^{13}C)$= 16Hz), 148.9(Ar), 149.5(Ar).

$^{19}$F NMR (282 MHz, DMSO-$D_6$): -124.4(C2-F, s), -135.2(C6-F, dd, $^1J$= 27Hz, $^2J$= 12Hz), -150.7(C5-F, dd, $^1J$=

27Hz, $^2J$= 6Hz).

HRMS calcd. for $C_{17}H_{19}F_3N_2O_7S_2$ [(M-H)$^-$]: 483.0513, found: 483.0515.

The compound **10f**. The product was purified by chromatography on a column of silica gel (0.04-0.063mm) with EtAc:$CHCl_3$ (1:1), Rf= 0.38. Yield: 0.16g, 60%, mp 131-132°C.

$^1H$ NMR (300 MHz, DMSO-$D_6$): 1.95(1H, sex, J= 7Hz, indane), 2.51(1H, sex, indane, signal overlaps with signal of DMSO-$D_6$), 2.8-3.1(2H, m, indane), 3.55-3.7(2H, m, $SO_2CH_2CH_2$), 3.7-3.85(2H, m, $SO_2CH_2CH_2$), 5.02(1H, t, J= 5Hz, OH), 5.1- 5.25(1H, m, CH of indane), 6.88(1H, d, J= 6Hz, NH), 7.2-7.5(4H, m, ArH), 8.43(2H, s, $SO_2NH_2$).

$^{13}C$ NMR (75 MHz, DMSO-$D_6$): 30.2(indane), 35.2(indane), 55.8($SO_2CH_2CH_2$), 60.2($SO_2CH_2CH_2$), 61.9(CH of indane, d, J($^{19}F$-$^{13}C$)= 12Hz), 117.6(C4, dd, $^1J$($^{19}F$-$^{13}C$)=13Hz, $^2J$($^{19}F$-$^{13}C$)= 5Hz), 124.7(Ar), 125.6(Ar), 127.4(Ar), 128(C1, t, J($^{19}F$-$^{13}C$)= 16Hz), 128.9(Ar), 135.6(C3, d, J($^{19}F$-$^{13}C$)= 12Hz), 137.8(C5 or C6, d, J($^{19}F$-$^{13}C$)= 253Hz), 144.4(Ar), 144.5(Ar), 144.8(C2, d, J($^{19}F$-$^{13}C$)= 251Hz), 146.3(C5 or C6, d, J($^{19}F$-$^{13}C$)= 258Hz).

$^{19}F$ NMR (282 MHz, DMSO-$D_6$): -124.6(C2-F, s), -134.8(C6-F, dd, $^1J$= 27Hz, $^2J$= 12Hz), -150.7(C5-F, dd, $^1J$= 27Hz, $^2J$= 6Hz).

HRMS calcd. for $C_{17}H_{17}F_3N_2O_5S_2$ [(M-H)$^-$]: 449.0458, found: 449.0461.

The compound **10g**. The product was purified by chromatography on a column of silica gel (0.04-0.063mm) with EtAc:$CHCl_3$ (1:1), Rf= 0.41. Yield: 0.14g, 51%, mp 103-105°C.

$^1H$ NMR (300 MHz, DMSO-D6): 1.7-2.1(4H, m, tetrahydronaphthalene), 2.6-2.9(2H, m, tetrahydronaphthalene), 3.61(2H, t, J= 5.4Hz, $SO_2CH_2CH_2$), 3.76(2H, br t, $SO_2CH_2CH_2$), 4.8-4.9(1H, m, CH of tetrahydronaphthalene), 5.01(1H, br s, OH), 6.82(1H, d, J= 9Hz, NH), 7.1-7.3(3H, m, ArH), 7.4(1H, d, J= 7.7Hz, ArH), 8.42(2H, s, $SO_2NH_2$).

$^{13}C$ NMR (75 MHz, DMSO-$D_6$): 19.3(tetrahydronaphthalene), 29.2(tetrahydronaphthalene), 30.6(tetrahydronaphthalene), 54.3(CH of tetrahydronaphthalene, d, J($^{19}F$-$^{13}C$)= 12Hz), 55.7($SO_2CH_2CH_2$), 60.3($SO_2CH_2CH_2$), 117.9(C4, dd, $^1J$($^{19}F$-$^{13}C$)= 13Hz, $^2J$($^{19}F$-$^{13}C$)= 5Hz), 126.8(Ar), 128(C1, t, J($^{19}F$-$^{13}C$)= 16Hz), 128.2(Ar), 129.5(Ar), 129.8(Ar), 135.1(C3, d, J($^{19}F$-$^{13}C$)= 15Hz), 137.48(Ar), 137.56(Ar), 137.9(C5 or C6, d, J($^{19}F$-$^{13}C$)= 245Hz), 145.1(C2, d, J($^{19}F$-$^{13}C$)= 255Hz), 146.2(C5 or C6, d, J($^{19}F$-$^{13}C$)= 254Hz).

$^{19}F$ NMR (282 MHz, DMSO-$D_6$): -123.9(C2-F, s), -134.5(C6-F, dd, $^1J$= 27Hz, $^2J$= 12Hz), -150.5(C5-F, dd, $^1J$= 27Hz, $^2J$= 6Hz).

HRMS calcd. for $C_{18}H_{19}F_3N_2O_5S_2$ [(M+H)$^+$]: 465.076, found: 465.0760.

The compound **10h**. The product was purified by chromatography on a column of silica gel (0.04-0.063mm) with EtAc:$CHCl_3$ (2:1), Rf= 0.53. Yield: 0.12g, 39%.

$^1H$ NMR (300 MHz, $CD_3OD$): 3.62(2H, k, J= 5.4Hz, $SO_2CH_2CH_2$), 4.04(2H, k, J= 5.4Hz, $SO_2CH_2CH_2$), 4.88($SO_2NH_2$, NH, OH signals overlap with signal of $H_2O$), 5.07(1H, dd, $^1J$= 5.1Hz, $^2J$= 2.1Hz, CH), 5.14(1H, d, J= 4.8Hz, CH), 7.1-7.3(10H, m, ArH).

$^{13}C$ NMR (75 MHz, $CD_3OD$): 55.5($SO_2CH_2CH_2$), 59.8($SO_2CH_2CH_2$), 65.7(CH, d, J($^{19}F$-$^{13}C$)= 12.5Hz), 76.3(CH), 116.8(C4, dd, $^1J$($^{19}F$-$^{13}C$)= 13Hz, $^2J$($^{19}F$-$^{13}C$)= 5.4Hz), 126.8(Ar), 127.33(Ar), 127.38(Ar), 127.7(Ar), 128.8(Ar), 128.5(Ar), 135(C3, d, J($^{19}F$-$^{13}C$)= 14Hz), 137.7(C5 or C6, d, J($^{19}F$-$^{13}C$)= 250Hz), 139.1(Ar), 141.7(Ar), 144.8(C2, d, J($^{19}F$-$^{13}C$)= 257Hz), 146.1(C5 or C6, d, J($^{19}F$-$^{13}C$)= 247Hz).

$^{19}F$ NMR (282 MHz, $CD_3OD$): -123.9(C2-F, s), -136.4(C6-F, dd, $^1J$= 25Hz, $^2J$= 12Hz), - 152.3(C5-F, dd, $^1J$= 24Hz, $^2J$= 6Hz).

HRMS calcd. for $C_{22}H_{21}F_3N_2O_6S_2$ [(M+H)$^+$]: 531.0866, found: 531.0865.

**Example 26**. Preparation of 3,5-bis(cyclooctylamino)-2,6-difluoro-4-[(2-phenylethyl)sulfonyl]benzenesulfonamide (compound **11**).

The mixture of 2,3,5,6-tetrafluoro-4-[(2-phenylethyl)sulfonyl]benzenesulfonamide (compound **2q**) (0.2g, 0.5mmol), $Et_3N$ (0.142mL, 1.02mmol), DMSO (1mL) cyclooctylamine (0.142mL, 1.02mmol) was stirred at 60°C for 32h. The mixture was then diluted with $H_2O$ (20mL) and extracted with EtAc (3×10mL). The combined organic phase was dried over $MgSO_4$ and evaporated in vacuum. The product was purified by chromatography on a column of silica gel (0.04-0.063mm) with EtAc (10%):$CHCl_3$, Rf= 0.72. Yield: 0.15g, 48%.

$^1H$ NMR (300 MHz, $CDCl_3$): 1.4-2(28H, m, cyclooctane), 3.05-3.15(2H, m, $SO_2CH_2CH_2$), 3.5-3.6(2H, m, $SO_2CH_2CH_2$), 3.88(2H, br s, 2×CH of cyclooctane), 5.58(2H, s, $SO_2NH_2$), 6.43(2H, br s, 2NH), 7.1-7.4(5H, m, ArH).

$^{13}C$ NMR (75 MHz,$CDCl_3$): 23.8(cyclooctane), 25.8(cyclooctane), 27.4(cyclooctane), 28.5($SO_2CH_2CH_2$), 33.5(cyclooctane), 55.9($SO_2CH_2CH_2$), 56.2(CH of cyclooctane, t, J= 6Hz), 111.1(C4, t, J($^{19}F$-$^{13}C$)= 5Hz), 126.3(C1, t, J($^{19}F$-$^{13}C$)= 16Hz), 127.4(Ar), 128.6(Ar), 129.2(Ar), 135.3(C3, dd, $^1J$($^{19}F$-$^{13}C$)= 10Hz, $^2J$($^{19}F$-$^{13}C$)= 6Hz), 137.3(Ar), 139.4(C2, dd, $^1J$($^{19}F$-$^{13}C$)= 244Hz, $^2J$($^{19}F$-$^{13}C$)= 4.5Hz).

$^{19}F$ NMR (282 MHz, $CDCl_3$): -144.1(2F, s).

HRMS calcd. for $C_{30}H_{43}F_2N_3O_4S_2$ [(M+H)$^+$]: 612.2736, found: 612.2729.

**Example 27**. Preparation of 3,5-bis[(3,4-dimethoxybenzyl)amino]-2,6-difluoro-4-[(2-hydroxyethyl)sulfonyl]benzenesulfonamide (compound **12**).

The mixture of 2,3,5,6-tetrafluoro-4-[(2-hydroxyethyl)sulfonyl]benzenesulfonamide (compound **2d**) (0.2g, 0.59mmol), DMSO (1mL) and 3,4-dimethoxybenzylamine (0.359mL, 2.38mmol) was stirred at ambient temperature for 5 days. The mixture was then diluted with $H_2O$ (20mL), the resultant precipitate was filtered, washed with $H_2O$. Recrystallization was accomplished from EtOH. Yield: 0.2g, 53%, mp 99-102°C.

$^1$H NMR (300 MHz, DMSO-$D_6$): 3.4($SO_2CH_2CH_2$, signal overlaps with signal of $H_2O$), 3.65(2H, k, J= 6Hz, $SO_2CH_2CH_2$), 3.75(12H, s, $4CH_3$), 4.4(4H, d, J= 5Hz, 2NHC$H_2$), 5.04(1H, t, J= 5.4Hz, OH), 6.38(2H, t, J= 5.7Hz, 2NH), 6.85-7.05(6H, m, ArH), 8.15(2H, s, $SO_2NH_2$).

$^{13}$C NMR (75 MHz, DMSO-$D_6$): 51.2($NHCH_2$, t, J($^{19}$F-$^{13}$C)= 6Hz), 55.5($SO_2CH_2CH_2$), 56.07($CH_3$), 56.18($CH_3$), 58.3($SO_2CH_2CH_2$), 112.4(Ar), 115.5(C4, t, J($^{19}$F-$^{13}$C)= 3Hz), 120.8(Ar), 127.9(C1, t, J($^{19}$F-$^{13}$C)= 16Hz), 132.3(Ar), 135.7(C3, dd, $^1$J($^{19}$F-$^{13}$C)= 10Hz, $^2$J($^{19}$F-$^{13}$C)= 6Hz), 141.7(C2, dd, $^1$J($^{19}$F-$^{13}$C)= 247Hz, $^2$J($^{19}$F-$^{13}$C)= 4Hz), 148.8(Ar), 149.4(Ar).

$^{19}$F NMR (282 MHz, DMSO-$D_6$): -133.47(2F, s).

HRMS calcd. for $C_{26}H_{31}F_2N_3O_9S_2$ [$(M+H)^+$]: 632.1543, found: 632.1548.

**Compound binding and inhibition measurements**

Inhibition of carbonic anhydrases is measured by determining the reduction in the velocity of catalysis. Carbonic anhydrases catalyse the reversible reaction: $CO_2 + H_2O \leftrightarrow HCO_3^- + H^+$

The inhibition of this reaction may be determined by measuring carbon dioxide consumption, bicarbonate appearance and the changes of pH (Krebs, J. F. and Fierke, C. A. (1993), J. Biol. Chem. 268, 948). All sulfonamides bind to the active center of carbonic anhydrases and diminish this reaction. Inhibition is equivalent to binding. (Chakravarty, S. and. Kannan, K. K. (1994), J. Mol. Biol. 243, 298; Lindskog, S. (1997), Pharmacol. Ther. 74, 1; Baird, T. T. J. et al. (1997), Biochemistry, 36, 2669). However, their binding and inhibitory efficiency varies greatly. Furthermore, the specificity of various sulfonamides varies greatly (Alterio, V. et al. (2012), Chem. Rev. 112, 4421). Sulfonamide binding to carbonic anhydrases is measured by a number of methods (Krishnamurthy, V. M. et al. (2008), Chem. Rev. 108, 946). Most often used methods are isothermal titration calorimetry, surface plasmon resonance, and ultracentrifugation. (Myszka, D. G. et al. (2003), J. Biomol. Tech. 14, 247). Specificity is determined by measuring binding constants with various isozymes and also by measuring intrinsic binding constants (Matulis, D. and Todd, M. J. (2004), Biocalorimetry 2).

**Example 28.** Determination of the observed binding constants by the fluorescent thermal shift assay (TSA).

Inhibitor binding to carbonic anhydrases was measured by the fluorescent thermal shift assay, which measures the binding constant of a ligand by determining the increase in the melting temperature of the protein in the presence of a ligand. TSA experiments were performed as previously described (Čapkauskaitė, E. et al. (2012), Eur. J. Med. Chem. 51, 259). TSA data were fit and analyzed as previously described (Kazlauskas, E. et al. (2012), PLoS ONE, 7, e36899).

Figure 1 shows representative binding data obtained by TSA (isozyme CA XIII). The dissociation constants for several selected characteristic compounds are listed in Table 1. A widely used CA inhibitors acetazolamide (AZM) and ethoxzolamide (EZA) were used as controls in these experiments. The data in the Table show that the number of fluorine atom substitutions and the nature of the substituent on the benzene ring significantly influences the binding affinity against CA I, CA II, CA VII, CA XII and CA XIII isoforms. The 4-substituted-2,3,5,6-tetrafluorobenzenesulfonamides (compounds **2a-x**) bind CAI, II, VII and XIII isozymes with low nanomolar and subnanomolar affinity, exhibiting especially strong binding of CA I ($K_d$ is in the range of 0.01 - 14 nM). The weakest binding is with CA XII ($K_d$ is in the range of 20 - 769 nM). Further substitution at position 2 (compounds **3a-o**, **4a-g, 5, 6**) significantly lowers affinity for all CA isozymes, expecially for CAI (the binding is in the micromolar range). Compounds **4a-g** (Table lists **4e**) demonstrate high selectivity for hCA XIII with respect to the other isoforms, CA I, II, VII and XII. Compounds **8a-f** (Table lists **8a**) do not have substituent at position 4 and fluorine atom at position 2 is substituted by a bulky group. They bind stronger to CAI then the same compounds having substituent at position 4 (compounds **4a-f**). Compounds **9a-o** having substitution at position 3 are good inhibitors of CA XIII. It seems that binding to CA XIII does not strongly depend on the size of substituent at this position because $K_d$ varies only in the narrow nanomolar range (from 1.3 nM for **9a** to 8.3 nM for **9h**). Compounds **10a-h** act as nanomolar inhibitors of CA II, CA VII, CA XII, and CA XIII.

TSA data of a selected representative compound binding to CA XIII (Figure 1). Panels on the top show thermal shift assay raw data of **2c** and **9c** binding to CA XIII. Panel on the bottom show the dependence of the protein melting temperatures $T_m$ on ligand concentrations. The curves in this panel show the simulated curves according to literature

model.

Table 1. Dissociation constants of selected compound binding to five human recombinant CA isoforms as determined by TSA (37 °C, pH 7.0).

| **Compound** | **Dissociation constants $K_d$ (nM) to CA isoforms** | | | | |
|---|---|---|---|---|---|
| | **CA I** | **CA II** | **CA VII** | **CA XII** | **CA XIII** |
| **1** | **2** | **3** | **4** | **5** | **6** |
| 2a | 8.3 | 91.0 | 465 | 769 | 140 |
| 2c | 0.11 | 6.7 | 45.5 | 222 | 8.3 |
| 2d | 0.20 | 17.0 | 7.1 | 250 | 29.0 |
| **1** | **2** | **3** | **4** | **5** | **6** |
| 2g | 0.20 | 11.0 | 5.0 | 50.0 | 6.7 |
| 2h | 0.40 | 20.0 | 10.0 | 91.0 | 20.0 |
| 2i | 0.10 | 67.0 | 147 | 200 | 14.3 |
| 2j | 0.13 | 4.0 | 11.8 | 20.0 | 1.5 |
| 2l | 0.10 | 2.5 | 1.0 | 50.0 | 1.0 |
| 2m | 0.25 | 1.25 | 1.25 | 6.67 | 0.40 |
| 2t | 2.5 | 10.0 | 10.0 | 290 | 2.5 |
| 2u | 1.1 | 1.1 | 0.22 | 200 | 0.25 |
| 2w | 0.40 | 6.7 | 13.0 | 110 | 2.5 |
| 2x | 14.0 | 6.7 | 1.7 | 670 | 2.0 |
| 3a | 3 300 | 500 | 130 | 2 900 | 67.0 |
| 3d | 1 700 | 3 300 | 4 000 | 5 000 | 40.0 |
| 3f | 25 000 | 250 | 170 | 500 | 500 |
| 31 | 5 000 | 3 300 | 4 000 | 1 700 | 100 |
| 4e | 8 300 | 2 780 | 1 100 | 1 250 | 33.3 |
| 8a | 167 | 200 | 167 | 833 | 100 |
| 9a | 67.0 | 5.9 | 8.3 | 290 | 1.3 |
| 9c | 56.0 | 6.7 | 5.0 | 40.0 | 2.5 |
| 9d | 500 | 17.0 | 4.0 | 33.0 | 6.7 |
| 9h | 58.8 | 22.2 | 667 | 167 | 8.3 |
| 9i | 213 | 22.2 | 6.7 | 250 | 3.3 |
| 9j | 500 | 50.0 | 5.0 | 17.0 | 5.6 |
| 9n | 1 700 | 33.0 | 10.0 | 250 | 5.6 |
| 9o | 770 | 91.0 | 40.0 | 400 | 6.7 |
| 10a | 200 | 83.0 | 130 | 25.0 | 14.0 |
| 10d | 200 | 16.7 | 40.0 | 66.7 | 25.0 |
| 10e | 83.3 | 25.0 | 14.3 | 66.7 | 4.3 |
| EZA | 14 | 0.71 | 0.71 | 36 | 13.0 |
| AZM | 1400 | 17.0 | 17.0 | 133 | 50.0 |

**Example 29.** Determination of the binding constants by isothermal titration calorimetry (ITC).

The heat evolved upon inhibitor binding to carbonic anhydrase isozymes was measured by isothermal titration calorimetry. ITC measurements were performed as previously described (Čapkauskaitė, E. et al. (2012), Eur. J. Med. Chem. 51, 259).

Figures 2, 3 show representative binding data obtained by ITC (isozymes CA I and CA XIII). The binding of **2d** to CA I is the case of very tight binding where ITC curve is too steep to be fitted precisely. Therefore TSA data is more reliable to determine tight binding than ITC. The observed dissociation constants for compounds **2t** and **10a** listed in Table 2 shows the negligible differences between observed $K_d$'s measured by two methods (see TSA data in Table1). Due to laborious nature of ITC measurements, only two compounds were tested by ITC and demonstrated that there is good agreement between TSA and ITC data.

Figure 2 shows ITC data of **2d** binding to CA I. Figure 3 shows ITC data of **2c** binding to CA XIII.

Table 2. Dissociation constants of selected compound binding to five human recombinant CA isoforms as determined by ITC (37 °C, pH 7.0).

| **Compound** | **Dissociation constants $K_d$ (nM) to CA isoforms** | | | | |
|---|---|---|---|---|---|
| | **CA I** | **CA II** | **CA VII** | **CA XII** | **CA XIII** |
| **2t** | 5.2 | 9.4 | 88.5 | 149 | 4.7 |
| **10a** | 130 | 139 | 405 | 12.2 | 45.2 |
| EZA | 25.0 | 0.38 | 8.8 | 23.0 | 12.0 |
| AZM | 780 | 17.7 | 83.3 | 79.2 | 64.4 |

**Example 30.** Determination of the compound inhibition constants $K_i$ by $CO_2$ hydration assay.

In addition to the binding measurements by TSA and ITC, inhibition constants were also determined by conventional hydration inhibition assay for several selected characteristic compounds and confirmed that there is good general agreement between TSA, ITC, and inhibition data.

The carbon dioxide hydration activity of recombinant human CAII was measured using Applied Photophysics SX.18MV-R stopped-flow spectrometer. Reaction velocities were measured by recording absorbance of bromothymol blue (40 μM). The reaction buffer contained 10 mM NaCl, 10 mM Hepes, pH 7.4. Saturated $CO_2$ solutions were made by bubbling gas in milli-Q water at 25°C. CAII was incubated with inhibitors for 15 min at a room temperature to form enzyme-inhibitor complex. CAII concentration was 20 nM and final DMSO concentration was less than 0.04 %. IC50 value was determined by fitting the sigmoidal curve on the data points and then $K_i$ was calculated using Cheng - Prusoff equation. The determined $K_i$ values for **2c**, **4e**, and AZM were 3.5, 500, and 10.6 nM, respectively (Figure 4 and 5). These values are systematically lower than the ones determined by TSA, but essentially within the standard deviation and the random error of both methods.

Figure 4 shows $CO_2$ hydration catalyzed by CA II and inhibition by **2c.** Figure 5 shows inhibition curves of CA II with **2c**, AZM and **4e**.

Newly synthesized sulfonamides of general formula (I)

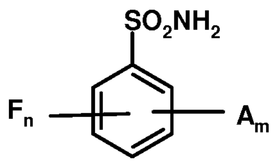

(I)

exhibit significant affinity and selectivity, often better than the existing compounds, promising to help in solving the issue of non-specific binding of clinically used inhibitors.

## Claims

1. Fluorinated benzenesulfonamides of general formula (I)

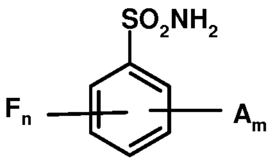

(I)

where benzenesulfonamide ring can be tetra- or penta- substituted,
where

n>1, n<5,

m≥1, (1-3 A groups are identical or different from each other, at least one A≠H, A=H no more than once),

A is H, $R^1$, OH, $OR^1$, SH, $SR^1$, $S(O)R^1$, $SO_2R^1$, $C(O)R^1$, $C(O)OR^1$, $OC(O)R^1$, $NHR^1$, $N(R^1)_2$, $C(O)NHR^1$, $C(O)N(R^1)_2$, $NHC(O)R^1$, $NR^1C(O)R^1$, $NHC(O)OR^1$, $NR^1C(O)OR^1$, $NHC(O)NH_2$, $NHC(O)NHR^1$, $NHC(O)N(R^1)_2$, $NR^1C(O)NHR^1$, $NR^1C(O)N(R^1)_2$, $SO_2NHR^1$, $SO_2N(R^1)_2$, $NR^1SO_2R^1$, $NHSO_2NHR^1$, $NHSO_2N(R^1)_2$, $NR^1SO_2NHR^1$, $NR^1SO_2N(R^1)_2$, C(O)NHNOH, $C(O)NHNOR^1$, $C(O)NHSO_2R^1$, $C(NH)NH_2$, $C(NH)NHR^1$, $C(NH)N(R^1)_2$, $NHSO_2NHR^1$, $NHSO_2N(CH_3)R^1$, $N(CH_3)SO_2N(CH_3)R^1$, Cl, Br, I, CN, $NO_2$, $N_3$, C(O)H, CHNOH, $CH(NOCH_3)$, $CF_3$, $CF_2CF_3$, $OCF_3$, $OCF_2CF_3$, C(O)OH, $C(O)NH_2$,

$R^1$ is $R^2$, $R^3$, $R^4$, $R^5$, $R^6$, $R^7$,

$R^2$ is phenyl, which is unfused or fused with benzene, heteroarene, cycloalkane or heterocycloalkane,

$R^3$ is heteroaryl, which is unfused or fused with benzene, heteroarene, cycloalkane or heterocycloalkane,

$R^4$ is cycloalkyl, cycloalkenyl, cycloalkynyl, heterocycloalkyl, heterocycloakenyl or heterocycloakynyl, each of which is unfused or fused with benzene, heteroarene,

$R^5$ is alkyl, alkenyl or alkynyl each of which is unsubstituted or substituted by one or more identical or different groups selected from $R^8$, OH, $OR^8$, SH, $SR^8$, $S(O)R^8$, $SO_2R^8$, $C(O)R^8$, $C(O)OR^8$, $OC(O)R^8$, $NHR^8$, $N(R^8)_2$, $C(O)NHR^8$, $C(O)N(R^8)_2$, $NHC(O)R^8$, $NR^8C(O)R^8$, $NHC(O)OR^8$, $NR^8C(O)OR^8$, $NHC(O)NH_2$, $NHC(O)NHR^8$, $NHC(O)N(R^8)_2$, $NR^8C(O)NHR^8$, $NR^8C(O)N(R^8)_2$, $SO_2NHR^8$, $SO_2N(R^8)_2$, $NR^8SO_2R^8$, $NHSO_2NHR^8$, $NHSO_2N(R^8)_2$, $NR^8SO_2NHR^8$, $NR^8SO_2N(R^8)_2$, C(O)NHNOH, $C(O)NHNOR^8$, $C(O)NHSO_2R^8$, $C(NH)NH_2$, $C(NH)NHR^8$, $C(NH)N(R^8)_2$, $NHSO_2NHR^8$, $NHSO_2N(CH_3)R^8$, $N(CH_3)SO_2N(CH_3)R^8$, F, Cl, Br, I, CN, $NO_2$, $N_3$, C(O)H, CHNOH, $CH(NOCH_3)$, $CF_3$, $CF_2CF_3$, $OCF_3$, $OCF_2CF_3$, C(O)OH, $C(O)NH_2$,

$R^8$ is $R^9$, $R^{10}$, $R^{11}$, $R^{12}$, $R^{13}$, $R^{14}$,

$R^9$ is phenyl, which is unfused or fused with benzene, heteroarene, cycloalkane or heterocycloalkane,

$R^{10}$ is heteroaryl, which is unfused or fused with benzene, heteroarene, cycloalkane or heterocycloalkane,

$R^{11}$ is cycloalkyl, cycloalkenyl, cycloalkynyl, heterocycloalkyl, heterocycloakenyl or heterocycloakynyl, each of which is unfused or fused with benzene, heteroarene,

$R^{12}$ is alkyl, alkenyl or alkynyl each of which is unsubstituted or substituted by one or more identical or different groups selected from

$NH_2$, $NHCH_3$, $N(CH_3)_2$, SH, SMe, $C(O)NH_2$, C(O)NHOH, $CF_3$, $CF_2CF_3$, $OCF_3$, $OCF_2CF_3$, C(O)H, C(O)OH, $C(O)OC_2H_5$, OH, $OCH_3$, $OC_2H_5$, $CH_3$, $C_2H_5$, $CH(CH_3)_2$, CN, $N_3$, $NO_2$, F, Cl, Br, I,

$R^{13}$ is phenyl which is unsubstituted or substituted by one or more identical or different groups selected from $NH_2$, $NHCH_3$, $N(CH_3)_2$, SH, SMe, $C(O)NH_2$, C(O)NHOH, $CF_3$, $CF_2CF_3$, $OCF_3$, $OCF_2CF_3$, C(O)H, C(O)OH, $C(O)OC_2H_5$, OH, $OCH_3$, $OC_2H_5$, $CH_3$, $C_2H_5$, $CH(CH_3)_2$, CN, $N_3$, $NO_2$, F, Cl, Br, I,

$R^{14}$ is heteroaryl, which is unsubstituted or substituted by one or more identical or different groups selected from

$NH_2$, $NHCH_3$, $N(CH_3)_2$, SH, SMe, $C(O)NH_2$, C(O)NHOH, $CF_3$, $CF_2CF_3$, $OCF_3$, $OCF_2CF_3$, C(O)H, C(O)OH, $C(O)OC_2H_5$, OH, $OCH_3$, $OC_2H_5$, $CH_3$, $C_2H_5$, $CH(CH_3)_2$, CN, $N_3$, $NO_2$, F, Cl, Br, I,

$R^6$ is phenyl which is unsubstituted or substituted by one or more identical or different groups selected from $R^{15}$, OH, $OR^{15}$, SH, $SR^{15}$, $S(O)R^{15}$, $SO_2R^{15}$, $C(O)R^{15}$, $C(O)OR^{15}$, $OC(O)R^{15}$, $NHR^{15}$, $N(R^{15})_2$, $C(O)NHR^{15}$, $C(O)N(R^{15})_2$, $NHC(O)R^{15}$, $NR^{15}C(O)R^{15}$, $NHC(O)OR^{15}$, $NR^{15}C(O)OR^{15}$, $NHC(O)NH_2$, $NHC(O)NHR^{15}$, $NHC(O)N(R^{15})_2$, $NR^{15}C(O)NHR^{15}$, $NR^{15}C(O)N(R^{15})_2$, $SO_2NHR^{15}$, $SO_2N(R^{15})_2$, $NR^{15}SO_2R^{15}$, $NHSO_2NHR^{15}$, $NHSO_2N(R^{15})_2$, $NR^{15}SO_2NHR^{15}$, $NR^{15}SO_2N(R^{15})_2$, C(O)NHNOH, $C(O)NHNOR^{15}$, $C(O)NHSO_2R^{15}$, $C(NH)NH_2$, $C(NH)NHR^{15}$, $C(NH)N(R^{15})_2$, $NHSO_2NHR^{15}$ $NHSO_2N(CH_3)R^{15}$, $N(CH_3)SO_2N(CH_3)R^{15}$, F, Cl, Br, I, CN, $NO_2$, $N_3$, C(O)H, CHNOH, $CH(NOCH_3)$, $CF_3$, $CF_2CF_3$, $OCF_3$, $OCF_2CF_3$, C(O)OH, $C(O)NH_2$,

$R^{15}$ is $R^{16}$, $R^{17}$, $R^{18}$, $R^{19}$, $R^{20}$, $R^{21}$,

$R^{16}$ is phenyl, which is unfused or fused with benzene, heteroarene, cycloalkane or heterocycloalkane,

$R^{17}$ is heteroaryl, which is unfused or fused with benzene, heteroarene, cycloalkane or heterocycloalkane,
$R^{18}$ is cycloalkyl, cycloalkenyl, cycloalkynyl, heterocycloalkyl, heterocycloakenyl or heterocycloakynyl, each of which is unfused or fused with benzene, heteroarene,
$R^{19}$ is alkyl, alkenyl or alkynyl each of which is unsubstituted or substituted by one or more identical or different groups selected from
$NH_2$, $NHCH_3$, $N(CH_3)_2$, SH, SMe, $C(O)NH_2$, C(O)NHOH, $CF_3$, $CF_2CF_3$, $OCF_3$, $OCF_2CF_3$, C(O)H, C(O)OH, $C(O)OC_2H_5$, OH, $OCH_3$, $OC_2H_5$, $CH_3$, $C_2H_5$, $CH(CH_3)_2$, CN, $N_3$, $NO_2$, F, Cl, Br, I,
$R^{20}$ is phenyl which is unsubstituted or substituted by one or more identical or different groups selected from $NH_2$, $NHCH_3$, $N(CH_3)_2$, SH, SMe, $C(O)NH_2$, C(O)NHOH, $CF_3$, $CF_2CF_3$, $OCF_3$, $OCF_2CF_3$, C(O)H, C(O)OH, $C(O)OC_2H_5$, OH, $OCH_3$, $OC_2H_5$, $CH_3$, $C_2H_5$, $CH(CH_3)_2$, CN, $N_3$, $NO_2$, F, Cl, Br, I,
$R^{21}$ is heteroaryl, which is unsubstituted or substituted by one or more identical or different groups selected from
$NH_2$, $NHCH_3$, $N(CH_3)_2$, SH, SMe, $C(O)NH_2$, C(O)NHOH, $CF_3$, $CF_2CF_3$, $OCF_3$, $OCF_2CF_3$, C(O)H, C(O)OH, $C(O)OC_2H_5$, OH, $OCH_3$, $OC_2H_5$, $CH_3$, $C_2H_5$, $CH(CH_3)_2$, CN, $N_3$, $NO_2$, F, Cl, Br, I,

$R^7$ is heteroaryl, which is unsubstituted or substituted by one or more identical or different groups selected from $R^{22}$, OH, $OR^{22}$, SH, $SR^{22}$, $S(O)R^{22}$, $SO_2R^{22}$, $C(O)R^{22}$, $C(O)OR^{22}$, $OC(O)R^{22}$, $NHR^{22}$, $N(R^{22})_2$, $C(O)NHR^{22}$, $C(O)N(R^{22})_2$, $NHC(O)R^{22}$, $NR^{22}C(O)R^{22}$, $NHC(O)OR^{22}$, $NR^{22}C(O)OR^{22}$, $NHC(O)NH_2$, $NHC(O)NHR^{22}$, $NHC(O)N(R^{22})_2$, $NR^{22}C(O)NHR^{22}$, $NR^{22}C(O)N(R^{22})_2$, $SO_2NHR^{22}$, $SO_2N(R^{22})_2$, $NR^{22}SO_2R^{22}$ , $NHSO_2NHR^{22}$, $NHSO_2N(R^{22})_2$, $NR^{22}SO_2NHR^{22}$, $NR^{22}SO_2N(R^{22})_2$, C(O)NHNOH, $C(O)NHNOR^{22}$, $C(O)NHSO_2R^{22}$ $C(NH)NH_2$, $C(NH)NHR^{22}$, $C(NH)N(R^{22})_2$, $NHSO_2NHR^{22}$, $NHSO_2N(CH_3)R^{22}$, $N(CH_3)SO_2N(CH_3)R^{22}$, F, Cl, Br, I, CN, $NO_2$, $N_3$, C(O)H, CHNOH, $CH(NOCH_3)$, $CF_3$, $CF_2CF_3$, $OCF_3$, $OCF_2CF_3$, C(O)OH, $C(O)NH_2$,

$R^{22}$ is $R^{23}$, $R^{24}$, $R^{25}$, $R^{26}$, $R^{27}$, $R^{28}$,
$R^{23}$ is phenyl, which is unfused or fused with benzene, heteroarene, cycloalkane or heterocycloalkane,
$R^{24}$ is heteroaryl, which is unfused or fused with benzene, heteroarene, cycloalkane or heterocycloalkane,
$R^{25}$ is cycloalkyl, cycloalkenyl, cycloalkynyl, heterocycloalkyl, heterocycloakenyl or heterocycloakynyl, each of which is unfused or fused with benzene, heteroarene,
$R^{26}$ is alkyl, alkenyl or alkynyl each of which is unsubstituted or substituted by one or more identical or different groups selected from
$NH_2$, $NHCH_3$, $N(CH_3)_2$, SH, SMe, $C(O)NH_2$, C(O)NHOH, $CF_3$, $CF_2CF_3$, $OCF_3$, $OCF_2CF_3$, C(O)H, C(O)OH, $C(O)OC_2H_5$, OH, $OCH_3$, $OC_2H_5$, $CH_3$, $C_2H_5$, $CH(CH_3)_2$, CN, $N_3$, $NO_2$, F, Cl, Br, I,
$R^{27}$ is phenyl which is unsubstituted or substituted by one or more identical or different groups selected from $NH_2$, $NHCH_3$, $N(CH_3)_2$, SH, SMe, $C(O)NH_2$, C(O)NHOH, $CF_3$, $CF_2CF_3$, $OCF_3$, $OCF_2CF_3$, C(O)H, C(O)OH, $C(O)OC_2H_5$, OH, $OCH_3$, $OC_2H_5$, $CH_3$, $C_2H_5$, $CH(CH_3)_2$, CN, $N_3$, $NO_2$, F, Cl, Br, I,
$R^{28}$ is heteroaryl, which is unsubstituted or substituted by one or more identical or different groups selected from
$NH_2$, $NHCH_3$, $N(CH_3)_2$, SH, SMe, $C(O)NH_2$, C(O)NHOH, $CF_3$, $CF_2CF_3$, $OCF_3$, $OCF_2CF_3$, C(O)H, C(O)OH, $C(O)OC_2H_5$, OH, $OCH_3$, $OC_2H_5$, $CH_3$, $C_2H_5$, $CH(CH_3)_2$, CN, $N_3$, $NO_2$, F, Cl, Br, I,

and/or pharmaceutically acceptable salts of the sulfonamides of general formula (I) thereof,
where the compounds are 4-substituted-2,3,5,6-tetrafluorobenzenesulfonamides, 2,4-disubstituted-3,5,6-trifluoro-benzenesulfonamides, 2-substituted-3,5,6-trifluorobenzensulfonamides, 3,4-disubstituted-2,5,6-trifluorobenzenesulfonamides, 3,4,5-trisubstituted-2,6-difluoro benzenesulfonamides,
with the proviso that the following compounds are excluded
2,3,5,6-tetrafluoro-4-methoxybenzenesulfonamide;
2,3,5,6-tetrafluoro-4-piperidin-1-ylbenzenesulfonamide;
2,3,5,6-tetrafluoro-4-(methylamino)benzenesulfonamide;
4-(dimethylamino)-2,3,5,6-tetrafluorobenzenesulfonamide;
4-(cyclohexylamino)-2,3,5 ,6-tetrafluorobenzenesulfonamide;
2,3,5,6-tetrafluoro-4-(isopropylamino)benzenesulfonamide;
4-(cyclopentylamino)-2,3,5 ,6-tetrafluorobenzenesulfonamide;
2,3,5,6-tetrafluoro-4-pyrrolidin-1-ylbenzenesulfonamide;
2,3,5,6-tetrafluoro-4-morpholin-4-ylbenzenesulfonamide;
2,3,5,6-tetrafluoro-4-(4-methylpiperazin-l-yl)benzenesulfonamide;
2,3,5,6-tetrafluoro-4-[(2-hydroxyethyl)amino]benzenesulfonamide;
4-(butylamino)-2,3,5,6-tetrafluorobenzenesulfonamide;
4-azepan-1-yl-2,3,5,6-tetrafluorobenzenesulfonamide;

2-(cyclopropylamino)-3,5,6-trifluorobenzenesulfonamide;
4-N-hexamethyleneimino-2,3,5,6-tetrafluorobenzenesulphonamide.

**2.** Fluorinated benzenesulfonamides according to claim 1, selected from a group comprising of:

2,3,5,6-tetrafluoro-4-hydrazinobenzenesulfonamide;
4-(2-benzylidenehydrazino)-2,3,5,6-tetrafluorobenzenesulfonamide;
2,3,5,6-tetrafluoro-4[(2-hydroxyethyl)thio]benzenesulfonamide;
2,3,5,6-tetrafluoro-4[(2-hydroxyethyl)sulfonyl]benzenesulfonamide;
2-{[4-(aminosulfonyl)-2,3,5,6-tetrafluorophenyl]sulfonyl}ethyl acetate;
2,3,5,6-tetrafluoro-4-(propylthio)benzenesulfonamide;
{[4-(aminosulfonyl)-2,3,5,6-tetrafluorophenyl]thio}acetic acid;
3-{[4-(aminosulfonyl)-2,3,5,6-tetrafluorophenyl]thio}propanoic acid;
6-{[4-(aminosulfonyl)-2,3,5,6-tetrafluorophenyl]amino}hexanoic acid;
2,3,5,6-tetrafluoro-4-(phenylthio)benzenesulfonamide;
2,3,5,6-tetrafluoro-4-(phenylsulfonyl)benzenesulfonamide;
2,3,5,6-tetrafluoro-4-phenoxybenzenesulfonamide;
4-(benzylthio)-2,3,5,6-tetrafluorobenzenesulfonamide;
4-(benzylamino)-2,3,5,6-tetrafluorobenzenesulfonamide;
2,3,5,6-tetrafluoro-4-{[2-(4-hydroxyphenyl)ethyl]amino}benzenesulfonamide;
2,3,5,6-tetrafluoro-4-[(2-phenylethyl)thio]benzenesulfonamide;
2,3,5,6-tetrafluoro-4-[(2-phenylethyl)sulfonyl]benzenesulfonamide;
2,3,5,6-tetrafluoro-4-morpholin-4-ylbenzenesulfonamide;
2,3,5,6-tetrafluoro-4-[(mesitylmethyl)thio]benzenesulfonamide;
4-[(4,6-dimethylpyrimidin-2-yl)thio]-2,3,5,6-tetrafluorobenzenesulfonamide;
4-(1,3-benzothiazol-2-ylthio)-2,3,5,6-tetrafluorobenzenesulfonamide;
4-(1-adamantylamino)-2,3,5,6-tetrafluorobenzenesulfonamide;
3-{[4-(aminosulfonyl)-2,3,5,6-tetrafluorophenyl]thio}-[1,2,3]thiadiazolo[3,4-a]benzimidazole;
4-[(4,5-diphenyl-1*H*-imidazol-2-yl)thio]-2,3,5,6-tetrafluorobenzenesulfonamide;
2-(isopropylamino)-3,5,6-trifluoro-4-[(2-phenylethyl)thio]benzenesulfonamide;
2-(benzylamino)-3,5,6-trifluoro-4-[(2-phenylethyl)thio]benzenesulfonamide;
2-[(2-phenylethyl)amino]-3,5,6-trifluoro-4-[(2-phenylethyl)thio]benzenesulfonamide;
2-[(1-phenylethyl)amino]-3,5,6-trifluoro-4-[(2-phenylethyl)thio]benzenesulfonamide;
2-morpholin-4-yl-3,5,6-trifluoro-4-[(2-phenylethyl)thio]benzenesulfonamide;
2-(cyclohexylamino)-3,5,6-trifluoro-4-[(2-phenylethyl)thio]benzenesulfonamide;
2-(cycloheptylamino)-3,5,6-trifluoro-4-[(2-phenylethyl)thio]benzenesulfonamide;
2-(cyclooctylamino)-3,5,6-trifluoro-4-[(2-phenylethyl)thio]benzenesulfonamide;
2-(cyclododecylamino)-3,5,6-trifluoro-4-[(2-phenylethyl)thio]benzenesulfonamide;
2-[(2,6-dimethoxybenzyl)amino]-3,5,6-trifluoro-4-[(2-phenylethyl)thio]benzenesulfonamide;
2-[(3,4-dimethoxybenzyl)amino]-3,5,6-trifluoro-4-[(2-phenylethyl)thio]benzenesulfonamide;
2-(2,3-dihydro-1*H*-inden-2-ylamino)-3,5,6-trifluoro-4-[(2-phenylethyl)thio]benzenesulfonamide;
2-[(1S)-2,3-dihydro-1*H*-inden-1-ylamino]-3,5,6-trifluoro-4-[(2-phenylethyl)thio]benzenesulfonamide;
2-[(1S)-1,2,3,4-tetrahydronaphthalen-1-ylamino]-3,5,6-trifluoro-4-[(2-phenylethyl)thio]benzenesulfonamide;
2-{[(1S, 2R)-2-hydroxy-1,2-diphenylethyl]amino}-3,5,6-trifluoro-4-[(2-phenylethyl)thio]benzenesulfonamide;
2-(cyclooctylamino)-3,5,6-trifluoro-4-[(2-hydroxyethyl)thio]benzenesulfonamide;
2-(cyclododecylamino)-3,5,6-trifluoro-4-[(2-hydroxyethyl)thio]benzenesulfonamide;
2-[(2,6-dimethoxybenzyl)amino]-3,5,6-trifluoro-4-[(2-hydroxyethyl)thio]benzenesulfonamide;
2-[(3,4-dimethoxybenzyl)amino]-3,5,6-trifluoro-4-[(2-hydroxyethyl)thio]benzenesulfonamide;
2-[(1S)-2,3-dihydro-1*H*-inden-1-ylamino]-3,5,6-trifluoro-4-[(2-hydroxyethyl)thio]benzenesulfonamide;
2-[(1S)-1,2,3,4-tetrahydronaphthalen-1-ylamino]-3,5,6-trifluoro-4-[(2-hydroxyethyl)thio]benzenesulfonamide;
2-{[(1S, 2R)-2-hydroxy-1,2-diphenylethyl]amino}-3,5,6-trifluoro-4-[(2-hydroxyethyl)thio]benzenesulfonamide;
2-(cyclooctylamino)-3,5,6-trifluoro-4-(propylthio)benzenesulfonamide;
2-(cyclooctylamino)-3,5,6-trifluoro-4-{[2-(4-hydroxyphenyl)ethyl]amino}benzenesulfonamide;
2-(cyclooctylamino)-3,5,6-trifluorobenzenesulfonamide;
2-(cyclododecylamino)-3,5,6-trifluorobenzenesulfonamide;
2-[(2,6-dimethoxybenzyl)amino]-3,5,6-trifluorobenzenesulfonamide;
2-[(3,4-dimethoxybenzyl)amino]-3,5,6-trifluorobenzenesulfonamide;
2-[(1S)-2,3-dihydro-1*H*-inden-1-ylamino]-3,5,6-trifluorobenzenesulfonamide;

2-[(1S)-1,2,3,4-tetrahydronaphthalen-1-ylamino]-3,5,6-trifluorobenzenesulfonamide;
3-(methylamino)-2,5,6-trifluoro-4-[(2-phenylethyl)sulfonyl]benzenesulfonamide;
3-(*tert*-butylamino)-2,5,6-trifluoro-4-[(2-phenylethyl)sulfonyl]benzenesulfonamide;
3-(benzylamino)-2,5,6-trifluoro-4-[(2-phenylethyl)sulfonyl]benzenesulfonamide;
3-[(2-phenylethyl)amino]-2,5,6-trifluoro-4-[(2-phenylethyl)sulfonyl]benzenesulfonamide;
3-morpholin-4-yl-2,5,6-trifluoro-4-[(2-phenylethyl)sulfonyl]benzenesulfonamide;
3-(cyclooctylamino)-2,5,6-trifluoro-4-[(2-phenylethyl)sulfonyl]benzenesulfonamide;
3-(cyclododecylamino)-2,5,6-trifluoro-4-[(2-phenylethyl)sulfonyl]benzenesulfonamide;
3-[(2,6-dimethoxybenzyl)amino]-2,5,6-trifluoro-4-[(2-phenylethyl)sulfonyl]benzenesulfonamide;
3-[(3,4-dimethoxybenzyl)amino]-2,5,6-trifluoro-4-[(2-phenylethyl)sulfonyl]benzenesulfonamide;
3-(1-adamantylamino)-2,5,6-trifluoro-4-[(2-phenylethyl)sulfonyl]benzenesulfonamide;
3-(2,3-dihydro-1*H*-inden-2-ylamino)-2,5,6-trifluoro-4-[(2-phenylethyl)sulfonyl]benzenesulfonamide;
3-[(1S)-2,3-dihydro-1*H*-inden-1-ylamino]-2,5,6-trifluoro-4-[(2-phenylethyl)sulfonyl]benzenesulfonamide;
3-[(1S)-1,2,3,4-tetrahydronaphthalen-1-ylamino]-2,5,6-trifluoro-4-[(2-phenylethyl)sulfonyl]benzenesulfonamide;
3-{[(1S,2R)-2-hydroxy-1,2-diphenylethyl]amino}-2,5,6-trifluoro-4-[(2-phenylethyl)sulfonyl]benzenesulfonamide;
3-{[(1R, 2S)-2-hydroxy-1,2-diphenylethyl]amino}-2,5,6-trifluoro-4-[(2-phenylethyl)sulfonyl]benzenesulfonamide;
3-(benzylamino)-2,5,6-trifluoro-4-[(2-hydroxyethyl)sulfonyl]benzenesulfonamide;
3-(cyclooctylamino)-2,5,6-trifluoro-4-[(2-hydroxyethyl)sulfonyl]benzenesulfonamide;
3-(cyclododecylamino)-2,5,6-trifluoro-4-[(2-hydroxyethyl)sulfonyl]benzenesulfonamide;
3-[(2,6-dimethoxybenzyl)amino]-2,5,6-trifluoro-4-[(2-hydroxyethyl)sulfonyl]benzenesulfonamide;
3-[(3,4-dimethoxybenzyl)amino]-2,5,6-trifluoro-4-[(2-hydroxyethyl)sulfonyl]benzenesulfonamide;
3-[(1S)-2,3-dihydro-1*H*-inden-1-ylamino]-2,5,6-trifluoro-4-[(2-hydroxyethyl) sulfonyl]benzenesulfonamide;
3-[(1S)-1,2,3,4-tetrahydronaphthalen-1-ylamino]-2,5,6-trifluoro-4-[(2-hydroxyethyl)sulfonyl]benzenesulfonamide;
3-{[(1S, 2R)-2-hydroxy-1,2-diphenylethyl]amino}-2,5,6-trifluoro-4-[(2-hydroxyethyl)sulfonyl]benzenesulfonamide;
3,5-bis(cyclooctylamino)-2,6-difluoro-4-[(2-phenylethyl)sulfonyl]benzenesulfonamide;
3,5-bis[(3,4-dimethoxybenzyl)amino]-2,6-difluoro-4-[(2-hydroxyethyl)sulfonyl]benzenesulfonamide.

3. Composition for use in control of conditions where inhibition of carbonic anhydrase is necessary, **characterized in that** it contains effective amount of sulfonamide according to any one of claims 1 to 2 and **characterized in that** the conditions are selected from intraocular hypertension (glaucoma), altitude sickness, headaches, migraine, neurological disorders, obesity and cancer, including signs and/or symptoms related to said diseases.

4. Composition according to claim 3 for use in a method for the treatment of disorders mediated by carbonic anhydrase isoforms selected from intraocular hypertension (glaucoma), altitude sickness, headaches, migraine, neurological disorders, obesity and cancer, including signs and/or symptoms related to said diseases.

**Patentansprüche**

1. Fluorierte Benzolsulfonamide einer allgemeinen Formel (I)

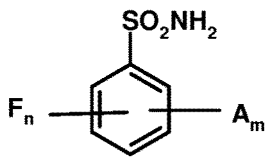

(I)

wo ein Benzolsulfonamidring tetra- oder penta-substituiert sein kann,
wo

n>1, n<5,
m≥1 (1-3 A Gruppen identisch sind oder sich voneinander unterscheiden, zumindest ein A≠H, A=H nicht mehr

als einmal),

A gleich H, $R^1$, OH, $OR^1$, SH, $SR^1$, $S(O)R^1$, $SO_2R^1$, $C(O)R^1$, $C(O)OR^1$, $OC(O)R^1$, $NHR^1$, $N(R^1)_2$, $C(O)NHR^1$, $C(O)N(R^1)_2$, $NHC(O)R^1$, $NR^1C(O)R^1$, $NHC(O)OR^1$, $NR^1C(O)OR^1$, $NHC(O)NH_2$, $NHC(O)NHR^1$, $NHC(O)N(R^1)_2$, $NR^1C(O)NHR^1$, $NR^1C(O)N(R^1)_2$, $SO_2NHR^1$, $SO_2N(R^1)_2$, $NR^1SO_2R^1$, $NHSO_2NHR^1$, $NHSO_2N(R^1)_2$, $NR^1SO_2NHR^1$, $NR^1SO_2N(R^1)_2$, C(O)NHNOH, $C(O)NHNOR^1$, $C(O)NHSO_2R^1$, $C(NH)NH_2$, $C(NH)NHR^1$, $C(NH)N(R^1)_2$, $NHSO_2NHR^1$, $NHSO_2N(CH_3)R^1$, $N(CH_3)SO_2N(CH_3)R^1$, Cl, Br, I, CN, $NO_2$, $N_3$, C(O)H, CHNOH, $CH(NOCH_3)$, $CF_3$, $CF_2CF_3$, $OCF_3$, $OCF_2CF_3$, C(O)OH, $C(O)NH_2$ ist

$R^1$ gleich $R^2$, $R^3$, $R^4$, $R^5$, $R^6$, $R^7$ ist

$R^2$ Phenyl ist, das unfusioniert oder mit Benzol, Heteroaren, Cycloalkan oder Heterocycloalkan fusioniert ist,

$R^3$ Heteroaryl ist, das unfusioniert oder mit Benzol, Heteroaren, Cycloalkan oder Heterocycloalkan fusioniert ist,

$R^4$ Cycloalkyl, Cycloalkenyl, Cycloalkinyl, Heterocycloalkyl, Heterocycloakenyl oder Heterocycloakinyl ist, von welchen jedes mit Benzol, Heteroaren unfusioniert oder fusioniert ist,

$R^5$ Alkyl, Alkenyl oder Alkinyl ist, von welchen jedes durch eine oder mehrere identische oder unterschiedliche Gruppen unsubstituiert oder substitutiert ist, ausgewählt aus $R^8$, OH, $OR^8$, SH, $SR^8$, $S(O)R^8$, $SO_2R^8$, $C(O)R^8$, $C(O)OR^8$, $OC(O)R^8$, $NHR^8$, $N(R^8)_2$, $C(O)NHR^8$, $C(O)N(R^8)_2$, $NHC(O)R^8$, $NR^8C(O)R^8$, $NHC(O)OR^8$, $NR^8C(O)OR^8$, $NHC(O)NH_2$, $NHC(O)NHR^8$, $NHC(O)N(R^8)_2$, $NR^8C(O)NHR^8$, $NR^BC(O)N(R^8)_2$, $SO_2NHR^8$, $SO_2N(R^8)_2$, $NR^8SO_2R^8$, $NHSO_2NHR^8$, $NHSO_2N(R^8)_2$, $NR^8SO_2NHR^8$, $NR^8SO_2N(R^8)_2$, C(O)NHNOH, $C(O)NHNOR^8$, $C(O)NHSO_2R^8$, $C(NH)NH_2$, $C(NH)NHR^8$, $C(NH)N(R^8)_2$, $NHSO_2NHR^8$, $NHSO_2N(CH_3)R^8$, $N(CH_3)SO_2N(CH_3)R^8$, F, Cl, Br, I, CN, $NO_2$, $N_3$, C(O)H, CHNOH, $CH(NOCH_3)$, $CF_3$, $CF_2CF_3$, $OCF_3$, $OCF_2CF_3$, C(O)OH, $C(O)NH_2$,

$R^8$ gleich $R^9$, $R^{10}$, $R^{11}$, $R^{12}$, $R^{13}$, $R^{14}$ ist,

$R^9$ Phenyl ist, das unfusioniert oder mit Benzol, Heteroaren, Cycloalkan oder Heterocycloalkan fusioniert ist,

$R^{10}$ Heteroaryl ist, das unfusioniert oder mit Benzol, Heteroaren, Cycloalkan oder Heterocycloalkan fusioniert ist,

$R^{11}$ Cycloalkyl, Cycloalkenyl, Cycloalkinyl, Heterocycloalkyl, Heterocycloakenyl oder Heterocycloakinyl ist, von welchen jedes unfusioniert oder mit Benzol, Heteroaren fusioniert ist,

$R^{12}$ Alkyl, Alkenyl oder Alkinyl ist, von welchen jedes unsubstituiert oder durch eine oder mehrere identische oder unterschiedliche Gruppen substituiert ist, ausgewählt aus

$NH_2$, $NHCH_3$, $N(CH_3)_2$, SH, SMe, $C(O)NH_2$, C(O)NHOH, $CF_3$, $CF_2CF_3$, $OCF_3$, $OCF_2CF_3$, C(O)H, C(O)OH, $C(O)OC_2H_5$, OH, $OCH_3$, $OC_2H_5$, $CH_3$, $C_2H_5$, $CH(CH_3)_2$, CN, $N_3$, $NO_2$, F, Cl, Br, I,

$R^{13}$ Phenyl ist, das unsubstituiert oder durch eine oder mehrere identische oder unterschiedliche Gruppen substituiert ist, ausgewählt aus

$NH_2$, $NHCH_3$, $N(CH_3)_2$, SH, SMe, $C(O)NH_2$, C(O)NHOH, $CF_3$, $CF_2CF_3$, $OCF_3$, $OCF_2CF_3$, C(O)H, C(O)OH, $C(O)OC_2H_5$, OH, $OCH_3$, $OC_2H_5$, $CH_3$, $C_2H_5$, $CH(CH_3)_2$, CN, $N_3$, $NO_2$, F, Cl, Br, I,

$R^{14}$ Heteroaryl ist, das unsubstituiert oder durch eine oder mehrere identische oder unterschiedliche Gruppen substituiert ist, ausgewählt aus

$NH_2$, $NHCH_3$, $N(CH_3)_2$, SH, SMe, $C(O)NH_2$, C(O)NHOH, $CF_3$, $CF_2CF_3$, $OCF_3$, $OCF_2CF_3$, C(O)H, C(O)OH, $C(O)OC_2H_5$, OH, $OCH_3$, $OC_2H_5$, $CH_3$, $C_2H_5$, $CH(CH_3)_2$, CN, $N_3$, $NO_2$, F, Cl, Br, I,

$R^6$ Phenyl ist, das unsubstituiert oder durch eine oder mehrere identische oder unterschiedliche Gruppen substituiert ist, ausgewählt aus $R^{15}$, OH, $OR^{15}$, SH, $SR^{15}$, $S(O)R^{15}$, $SO_2R^{15}$, $C(O)R^{15}$, $C(O)OR^{15}$, $OC(O)R^{15}$, $NHR^{15}$, $N(R^{15})_2$, $C(O)NHR^{15}$, $C(O)N(R^{15})_2$, $NHC(O)R^{15}$, $NR^{15}C(O)R^{15}$, $NHC(O)OR^{15}$, $NR^{15}C(O)OR^{15}$, $NHC(O)NH_2$, $NHC(O)NHR^{15}$, $NHC(O)N(R^{15})_2$, $NR^{15}C(O)NHR^{15}$, $NR^{15}C(O)N(R^{15})_2$, $SO_2NHR^{15}$, $SO_2N(R^{15})_2$, $NR^{15}SO_2R^{15}$, $NHSO_2NHR^{15}$, $NHSO_2N(R^{15})_2$, $NR^{15}SO_2NHR^{15}$, $NR^{15}SO_2N(R^{15})_2$, C(O)NHNOH, $C(O)NHNOR^{15}$, $C(O)NHSO_2R^{15}$, $C(NH)NH_2$, $C(NH)NHR^{15}$, $C(NH)N(R^{15})_2$, $NHSO_2NHR^{15}$, $NHSO_2N(CH_3)R^{15}$, $N(CH_3)SO_2N(CH_3)R^{15}$, F, Cl, Br, I, CN, $NO_2$, $N_3$, C(O)H, CHNOH, $CH(NOCH_3)$, $CF_3$, $CF_2CF_3$, $OCF_3$, $OCF_2CF_3$, C(O)OH, $C(O)NH_2$,

$R^{15}$ gleich $R^{16}$, $R^{17}$, $R^{18}$, $R^{19}$, $R^{20}$, $R^{21}$ ist,

$R^{16}$ Phenyl ist, das unfusioniert oder mit Benzol, Heteroaren, Cycloalkan oder Heterocycloalkan fusioniert ist,

$R^{17}$ Heteroaryl ist, das unfusioniert oder mit Benzol, Heteroaren, Cycloalkan oder Heterocycloalkan fusioniert ist,

$R^{18}$ Cycloalkyl, Cycloalkenyl, Cycloalkinyl, Heterocycloalkyl, Heterocycloakenyl oder Heterocycloakinyl ist, von welchen jedes unfusioniert oder mit Benzol, Heteroaren fusioniert ist,

$R^{19}$ Alkyl, Alkenyl oder Alkinyl ist, von welchen jedes unsubstituiert oder durch eine oder mehrere identische oder unterschiedliche Gruppen substituiert ist, ausgewählt aus

$NH_2$, $NHCH_3$, $N(CH_3)_2$, SH, SMe, $C(O)NH_2$, C(O)NHOH, $CF_3$, $CF_2CF_3$, $OCF_3$, $OCF_2CF_3$, C(O)H, C(O)OH, $C(O)OC_2H_5$, OH, $OCH_3$, $OC_2H_5$, $CH_3$, $C_2H_5$, $CH(CH_3)_2$, CN, $N_3$, $NO_2$, F, Cl, Br, I,

$R^{20}$ Phenyl ist, das unsubstituiert oder durch eine oder mehrere identische oder unterschiedliche Gruppen substituiert ist, ausgewählt aus

$NH_2$, $NHCH_3$, $N(CH_3)_2$, SH, SMe, $C(O)NH_2$, C(O)NHOH, $CF_3$, $CF_2CF_3$, $OCF_3$, $OCF_2CF_3$, C(O)H, C(O)OH, $C(O)OC_2H_5$, OH, $OCH_3$, $OC_2H_5$, $CH_3$, $C_2H_5$, $CH(CH_3)_2$, CN, $N_3$, $NO_2$, F, Cl, Br, I,

$R^{21}$ Heteroaryl ist, das unsubstituiert oder durch eine oder mehrere identische oder unterschiedliche Gruppen substituiert ist, ausgewählt aus
$NH_2$, $NHCH_3$, $N(CH_3)_2$, SH, SMe, $C(O)NH_2$, C(O)NHOH, $CF_3$, $CF_2CF_3$, $OCF_3$, $OCF_2CF_3$, C(O)H, C(O)OH, $C(O)OC_2H_5$, OH, $OCH_3$, $OC_2H_5$, $CH_3$, $C_2H_5$, $CH(CH_3)_2$, CN, $N_3$, $NO_2$, F, Cl, Br, I,
$R^7$ Heteroaryl ist, das unsubstituiert oder durch eine oder mehrere identische oder unterschiedliche Gruppen substituiert ist, ausgewählt aus $R^{22}$, OH, $OR^{22}$, SH, $SR^{22}$, $S(O)R^{22}$, $SO_2R^{22}$, $C(O)R^{22}$, $C(O)OR^{22}$, $OC(O)R^{22}$, $NHR^{22}$, $N(R^{22})_2$, $C(O)NHR^{22}$, $C(O)N(R^{22})_2$, $NHC(O)R^{22}$, $NR^{22}C(O)R^{22}$, $NHC(O)OR^{22}$, $NR^{22}C(O)OR^{22}$, $NHC(O)NH_2$, $NHC(O)NHR^{22}$, $NHC(O)N(R^{22})_2$, $NR^{22}C(O)NHR^{22}$, $NR^{22}C(O)N(R^{22})_2$, $SO_2NHR^{22}$, $SO_2N(R^{22})_2$, $NR^{22}SO_2R^{22}$, $NHSO_2NHR^{22}$, $NHSO_2N(R^{22})_2$, $NR^{22}SO_2NHR^{22}$, $NR^{22}SO_2N(R^{22})_2$, C(O)NHNOH, $C(O)NHNOR^{22}$, $C(O)NHSO_2R^{22}$, $C(NH)NH_2$, $C(NH)NHR^{22}$, $C(NH)N(R^{22})_2$, $NHSO_2NHR^{22}$, $NHSO_2N(CH_3)R^{22}$, $N(CH_3)SO_2N(CH_3)R^{22}$, F, Cl, Br, I, CN, $NO_2$, $N_3$, C(O)H, CHNOH, $CH(NOCH_3)$, $CF_3$, $CF_2CF_3$, $OCF_3$, $OCF_2CF_3$, C(O)OH, $C(O)NH_2$,
$R^{22}$ gleich $R^{23}$, $R^{24}$, $R^{25}$, $R^{26}$, $R^{27}$, $R^{28}$ ist,
$R^{23}$ Phenyl ist, das unfusioniert oder mit Benzol, Heteroaren, Cycloalkan oder Heterocycloalkan fusioniert ist,
$R^{24}$ Heteroaryl ist, das unfusioniert oder mit Benzol, Heteroaren, Cycloalkan oder Heterocycloalkan fusioniert ist,
$R^{25}$ Cycloalkyl, Cycloalkenyl, Cycloalkinyl, Heterocycloalkyl, Heterocycloakenyl oder Heterocycloakinyl ist, von welchen jedes unfusioniert oder mit Benzol, Heteroaren fusioniert ist,
$R^{26}$ Alkyl, Alkenyl oder Alkinyl ist, von welchen jedes unsubstituiert oder durch eine oder mehrere identische oder unterschiedliche Gruppen substituiert ist, ausgewählt aus
$NH_2$, $NHCH_3$, $N(CH_3)_2$, SH, SMe, $C(O)NH_2$, C(O)NHOH, $CF_3$, $CF_2CF_3$, $OCF_3$, $OCF_2CF_3$, C(O)H, C(O)OH, $C(O)OC_2H_5$, OH, $OCH_3$, $OC_2H_5$, $CH_3$, $C_2H_5$, $CH(CH_3)_2$, CN, $N_3$, $NO_2$, F, Cl, Br, I,
$R^{27}$ Phenyl ist, das unsubstituiert oder durch eine oder mehrere identische oder unterschiedliche Gruppen substituiert ist, ausgewählt aus
$NH_2$, $NHCH_3$, $N(CH_3)_2$, SH, SMe, $C(O)NH_2$, C(O)NHOH, $CF_3$, $CF_2CF_3$, $OCF_3$, $OCF_2CF_3$, C(O)H, C(O)OH, $C(O)OC_2H_5$, OH, $OCH_3$, $OC_2H_5$, $CH_3$, $C_2H_5$, $CH(CH_3)_2$, CN, $N_3$, $NO_2$, F, Cl, Br, I,
$R^{28}$ Heteroaryl ist, das unsubstituiert oder durch eine oder mehrere identische oder unterschiedliche Gruppen substituiert ist, ausgewählt aus
$NH_2$, $NHCH_3$, $N(CH_3)_2$, SH, SMe, $C(O)NH_2$, C(O)NHOH, $CF_3$, $CF_2CF_3$, $OCF_3$, $OCF_2CF_3$, C(O)H, C(O)OH, $C(O)OC_2H_5$, OH, $OCH_3$, $OC_2H_5$, $CH_3$, $C_2H_5$, $CH(CH_3)_2$, CN, $N_3$, $NO_2$, F, Cl, Br, I,
und/oder pharmazeutisch annehmbaren Salzen der Sulfonamide der allgemeinen Formel (I) davon,
wobei die Verbindungen 4-substituierte-2,3,5,6-Tetrafluorbenzolsulfonamide, 2,4-disubstituierte-3,5,6-Trifluorbenzolsulfonamide, 2-substituierte-3,5,6-Trifluorbenzolsulfonamide, 3,4-disubstituierte-2,5,6-Trifluorbenzolsulfonamide, 3,4,5-trisubstituierte-2,6-Difluorbenzolsulfonamide sind,
unter der Voraussetzung, dass die folgenden Verbindungen ausgeschlossen sind:

2,3,5,6-Tetrafluor-4-methoxybenzolsulfonamid;
2,3,5,6-Tetrafluor-4-piperidin-1-ylbenzolsulfonamid;
2,3,5,6-Tetrafluor-4-(methylamino)benzolsulfonamid;
4-(Dimethylamino)-2,3,5,6-tetrafluorbenzolsulfonamid;
4-(Cyclohexylamino)-2,3,5,6-tetrafluorbenzolsulfonamid;
2,3,5,6-Tetrafluor-4-(isopropylamino)benzolsulfonamid;
4-(Cyclopentylamino)-2,3,5,6-tetrafluorbenzolsulfonamid;
2,3,5,6-Tetrafluor-4-pyrrolidin-1-ylbenzolsulfonamid;
2,3,5,6-Tetrafluor-4-morpholin-4-ylbenzolsulfonamid;
2,3,5,6-Tetrafluor-4-(4-methylpiperazin-1-yl)benzolsulfonamid;
2,3,5,6-Tetrafluor-4-[(2-hydroxyethyl)amino]benzolsulfonamid;
4-(Butylamino)-2,3,5,6-tetrafluorbenzolsulfonamid;
4-Azepan-1-yl-2,3,5,6-tetrafluorbenzolsulfonamid;
2-(Cyclopropylamino)-3,5,6-tetrafluorbenzolsulfonamid;
4-N-Hexymethylenimino-2,3,5,6-tetrafluorbenzolsulfonamid.

**2.** Fluorierte Benzolsulfonamide nach Anspruch 1, ausgewählt aus einer Gruppe umfassend:

2,3,5,6-Tetrafluor-4-hydrazinobenzolsulfonamid;
4-(2-Benzylidenhydrazino)-2,3,5,6-tetrafluorbenzolsulfonamid;
2,3,5,6-Tetrafluor-4[(2-hydroxyethyl)thio]benzolsulfonamid;
2,3,5,6-Tetrafluor-4[(2-hydroxyethyl)sulfonyl]benzolsulfonamid;
2-{[4-(Aminosulfonyl)-2,3,5,6-tetrafluorphenyl]sulfonyl}ethylacetat;

2,3,5,6-Tetrafluor-4-(propylthio)benzolsulfonamid;
{[4-(Aminosulfonyl)-2,3,5,6-tetrafluorphenyl]thio}essigsäure;
3-{[4-(Aminosulfonyl)-2,3,5,6-tetrafluorphenyl]thio}propansäure;
6-{[4-(Aminosulfonyl)-2,3,5,6-tetrafluorphenyl]amino}hexansäure
2,3,5,6-Tetrafluor-4-(phenylthio)benzolsulfonamid;
2,3,5,6-Tetrafluor-4-(phenylsulfonyl)benzolsulfonamid;
2,3,5,6-Tetrafluor-4-phenoxybenzolsulfonamid;
4-(Benzylthio)-2,3,5,6-tetrafluorbenzolsulfonamid;
4-(Benzylamino)-2,3,5,6-tetrafluorbenzolsulfonamid;
2,3,5,6-Tetrafluor-4-{[2-(4-hydroxyphenyl)ethyl]amino}benzolsulfonamid;
2,3,5,6-Tetrafluor-4-[(2-phenylethyl)thio]benzolsulfonamid;
2,3,5,6-Tetrafluor-4-[(2-phenylethyl)sulfonyl]benzolsulfonamid;
2,3,5,6-Tetrafluor-4-morpholin-4-ylbenzolsulfonamid;
2,3,5,6-Tetrafluor-4-[(mesitylmethyl)thio]benzolsulfonamid;
4-[(4,6-Dimethylpyrimidin-2-yl)thio]-2,3,5,6-tetrafluorbenzolsulfonamid;
4-(1,3-Benzothiazol-2-ylthio)-2,3,5,6-tetrafluorbenzolsulfonamid;
4-(1-Adamantylamino)-2,3,5,6-tetrafluorbenzolsulfonamid;
3-1[4-(Aminosulfonyl)-2,3,5,6-tetrafluorphenyl]thiol-[1,2,3]thiadiazolo[3,4-a]benzimidazol;
4-[(4,5-Diphenyl-1*H*-imidazol-2-yl)thio]-2,3,5,6-tetrafluorbenzolsulfonamid;
2-(Isopropylamino)-3,5,6-trifluor-4-[(2-phenylethyl)thio]benzolsulfonamid;
2-(Benzylamino)-3,5,6-trifluor-4-[(2-phenylethyl)thio]benzolsulfonamid;
2-[(2-Phenylethyl)amino]-3,5,6-trifluor-4-[(2-phenylethyl)thio]benzolsulfonamid;
2-[(1-Phenylethyl)amino]-3,5,6-trifluor-4-[(2-phenylethyl)thio]benzolsulfonamid;
2-Morpholin-4-yl-3,5,6-trifluor-4-[(2-phenylethyl)thio]benzolsulfonamid;
2-(Cyclohexylamino)-3,5,6-trifluor-4-[(2-phenylethyl)thio]benzolsulfonamid;
2-(Cycloheptylamino)-3,5,6-trifluor-4-[(2-phenylethyl)thio]benzolsulfonamid;
2-(Cyclooctylamino)-3,5,6-trifluor-4-[(2-phenylethyl)thio]benzolsulfonamid;
2-(Cyclododecylamino)-3,5,6-trifluor-4-[(2-phenylethyl)thio]benzolsulfonamid;
2-[(2,6-Dimethoxybenzyl)amino]-3,5,6-trifluor-4-[(2-phenylethyl)thio]benzolsulfonamid;
2-[(3,4-Dimethoxybenzyl)amino]-3,5,6-trifluor-4-[(2-phenylethyl)thio]benzolsulfonamid;
2-(2,3-Dihydro-1*H*-inden-2-ylamino)-3,5,6-trifluor-4-[(2-phenylethyl)thio]benzolsulfonamid;
2-[(1S)-2,3-Dihydro-1*H*-inden-1-ylamino]-3,5,6-trifluor-4-[(2-phenylethyl)thio]benzolsulfonamid;
2-[(1S)-1,2,3,4-Tetrahydronapthalin-1-ylamino]-3,5,6-trifluor-4-[(2-phenylethyl)thio]benzolsulfonamid;
2-{[(15,2R)-2-Hydroxy-1,2-diphenylethyl]amino}-3,5,6-trifluor-4-[(2-phenylethyl)thio]benzolsulfonamid;
2-(Cyclooctylamino)-3,5,6-trifluor-4-[(2-hydroxyethyl)thio]benzolsulfonamid;
2-(Cyclododecylamino)-3,5,6-trifluor-4-[(2-hydroxyethyl)thio]benzolsulfonamid;
2-[(2,6-Dimethoxybenzyl)amino]-3,5,6-trifluor-4-[(2-hydroxyethyl)thio]benzolsulfonamid;
2-[(3,4-Dimethoxybenzyl)amino]-3,5,6-trifluor-4-[(2-hydroxyethyl)thio]benzolsulfonamid;
2-[(1S)-2,3-Dihydro-1*H*-inden-1-ylamino]-3,5,6-trifluor-4-[(2-hydroxyethyl)thio]benzolsulfonamid;
2-[(1S)-1,2,3,4-Tetrahydronapthalin-1-ylamino)-3,5,6-trifluor-4-[(2-hydroxyethyl)thio]benzolsulfonamid;
2-{[(1S,2R)-2-Hydroxy-1,2-diphenylethyl]amino}-3,5,6-trifluor-4-[(2-hydroxyethyl)thio]benzolsulfonamid;
2-(Cyclooctylamino)-3,5,6-trifluor-4-(propylthio)benzolsulfonamid;
2-(Cyclooctylamino)-3,5,6-trifluor-4-{[2-(4-hydroxyphenyl)ethyl]amino}benzolsulfonamid;
2-(Cyclooctylamino)-3,5,6-trifluorbenzolsulfonamid;
2-(Cyclododecylamino)-3,5,6-trifluorbenzolsulfonamid;
2-[(2,6-Dimethoxybenzyl)amino]-3,5,6-trifluorbenzolsulfonamid;
2-[(3,4-Dimethoxybenzyl)amino]-3,5,6-trifluorbenzolsulfonamid;
2-[(1S)-2,3-Dihydro-1*H*-inden-1-ylamino]-3,5,6-trifluorbenzolsulfonamid;
2-[(1S)-1,2,3,4-Tetrahydronapthalin-1-ylamino)-3,5,6-trifluorbenzolsulfonamid;
3-(Methylamino)-2,5,6-trifluor-4-[(2-phenylethyl)sulfonyl]benzolsulfonamid;
3-(*tert*-Butylamino)-2,5,6-trifluor-4-[(2-phenylethyl)sulfonyl]benzolsulfonamid;
3-(Benzylamino)-2,5,6-trifluor-4-[(2-phenylethyl)sulfonyl]benzolsulfonamid;
3-[(2-Phenylethyl)amino]-2,5,6-trifluor-4-[(2-phenylethyl)sulfonyl]benzolsulfonamid;
3-Morpholin-4-yl-2,5,6-trifluor-4-[(2-phenylethyl)sulfonyl]benzolsulfonamid;
3-(Cyclooctylamino)-2,5,6-trifluor-4-[(2-phenylethyl)sulfonyl]benzolsulfonamid;
3-(Cyclododecylamino)-2,5,6-trifluor-4-[(2-phenylethyl)sulfonyl]benzolsulfonamid;
3-[(2,6-Dimethoxybenzyl)amino]-2,5,6-trifluor-4-[(2-phenylethyl)sulfonyl]benzolsulfonamid;
3-[(3,4-Dimethoxybenzyl)amino]-2,5,6-trifluor-4-[(2-phenylethyl)sulfonyl]benzolsulfonamid;

3-(1-Adamantylamino)-2,5,6-trifluor-4-[(2-phenylethyl)sulfonyl]benzolsulfonamid;
3-(2,3-Dihydro-1*H*-inden-2-ylamino)-2,5,6-trifluor-4-[(2-phenylethyl)sulfonyl]benzolsulfonamid;
3-[(1S)-2,3-Dihydro-1*H*-inden-1-ylamino]-2,5,6-trifluor-4-[(2-phenylethyl)sulfonyl]benzolsulfonamid;
3-[(1S)-1,2,3,4-Tetrahydronapthalin-1-ylamino)-2,5,6-trifluor-4-[(2-phenylethyl)sulfonyl]benzolsulfonamid;
3-{[(1S,2R)-2-Hydroxy-1,2-diphenylethyl]amino}-2,5,6-trifluor-4-[(2-phenylethyl)sulfonyl]benzolsulfonamid;
3-{[(1R,2S)-2-Hydroxy-1,2-diphenylethyl]amino}-2,5,6-trifluor-4-[(2-phenylethyl)sulfonyl]benzolsulfonamid;
3-(Benzylamino)-2,5,6-trifluor-4-[(2-hydroxyethyl)sulfonyl]benzolsulfonamid;
3-(Cyclooctylamino)-2,5,6-trifluor-4-[(2-hydroxyethyl)sulfonyl]benzolsulfonamid;
3-(Cyclododecylamino)-2,5,6-trifluor-4-[(2-hydroxyethyl)sulfonyl]benzolsulfonamid;
3-[(2,6-Dimethoxybenzyl)amino]-2,5,6-trifluor-4-[(2-hydroxyethyl)sulfonyl]benzolsulfonamid;
3-[(3,4-Dimethoxybenzyl)amino]-2,5,6-trifluor-4-[(2-hydroxyethyl)sulfonyl]benzolsulfonamid;
3-[(1S)-2,3-Dihydro-1*H*-inden-1-ylamino]-2,5,6-trifluor-4-[(2-hydroxyethyl)sulfonyl]benzolsulfonamid;
3-[(1S)-1,2,3,4-Tetrahydronapthalin-1-ylamino)-2,5,6-trifluor-4-[(2-hydroxyethyl)sulfonyl]benzolsulfonamid;
3-{[(1S,2R)-2-Hydroxy-1,2-diphenylethyl]amino}-2,5,6-trifluor-4-[(2-hydroxyethyl)sulfonyl]benzolsulfonamid;
3,5-bis(Cyclooctylamino)-2,6-difluor-4-[(2-phenylethyl)sulfonyl]benzolsulfonamid;
3,5-bis[(3,4-Dimethoxybenzyl)amino]-2,6-difluor-4-[(2-hydroxyethyl)sulfonyl]benzolsulfonamid.

**3.** Zusammensetzung zur Verwendung bei einer Kontrolle von Erkrankungen, wo eine Hemmung von Carboanhydrase erforderlich ist, **dadurch gekennzeichnet, dass** sie eine wirksame Menge von Sulfonamid nach einem der Ansprüche 1 bis 2 enthält, und **dadurch gekennzeichnet, dass** die Erkrankungen ausgewählt sind aus intraokulärer Hypertension (Glaukom), Höhenkrankheit, Kopfschmerzen, Migräne, neurologischen Störungen, Adipositas und Krebs, Zeichen und/oder Symptomen, die mit diesen Krankheiten in Zusammenhang stehen.

**4.** Zusammensetzung nach Anspruch 3, zur Verwendung in einem Verfahren für die Behandlung von Störungen, die durch Carboanhydrase-Isoformen vermittelt werden, ausgewählt aus intraokulärer Hypertension (Glaukom), Höhenkrankheit, Kopfschmerzen, Migräne, neurologischen Störungen, Adipositas und Krebs, Zeichen und/oder Symptomen, die mit diesen Krankheiten in Zusammenhang stehen.

## Revendications

**1.** Benzènesulfonamides fluorés de formule générale (I)

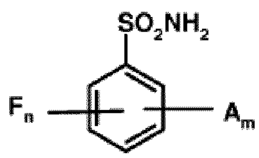

(I)

où le cycle benzènesulfonamide peut être tétra- ou penta-substitué,
où

$n > 1$, $n < 5$,
$m \geq 1$, (1 à 3 groupes A sont identiques ou différents les uns des autres, au moins un $A \neq H$, A = H pas plus d'une fois),
A est H, $R^1$, OH, $OR^1$, SH, $SR^1$, $S(O)R^1$, $SO_2R^1$, $C(O)R^1$, $C(O)OR^1$, $OC(O)R^1$, $NHR^1$, $N(R^1)_2$, $C(O)NHR^1$, $C(O)N(R^1)_2$, $NHC(O)R^1$, $NR^1C(O)R^1$, $NHC(O)OR^1$, $NR^1C(O)OR^1$, $NHC(O)NH_2$, $NHC(O)NHR^1$, $NHC(O)N(R^1)_2$, $NR^1C(O)NHR^1$, $NR^1C(O)N(R^1)_2$, $SO_2NHR^1$, $SO_2N(R^1)_2$, $NR^1SO_2R^1$, $NHSO_2NHR^1$, $NHSO_2N(R^1)_2$, $NR^1SO_2NHR^1$, $NR^1SO_2N(R^1)_2$, C(O)NHNOH, $C(O)NHNOR^1$, $C(O)NHSO_2R^1$, $C(NH)NH_2$, $C(NH)NHR^1$, $C(NH)N(R^1)_2$, $NHSO_2NHR^1$, $NHSO_2N(CH_3)R^1$, $N(CH_3)SO_2N(CH_3)R^1$, Cl, Br, I, CN, $NO_2$, $N_3$, C(O)H, CHNOH, $CH(NOCH_3)$, $CF_3$, $CF_2CF_3$, $OCF_3$, $OCF_2CF_3$, C(O)OH, $C(O)NH_2$,
$R^1$ est $R^2$, $R^3$, $R^4$, $R^5$, $R^6$, $R^7$,
$R^2$ est un phényle, qui est non fusionné ou fusionné avec un benzène, un hétéroarène, un cycloalcane ou un hétérocycloalcane,
$R^3$ est un hétéroaryle, qui est non fusionné ou fusionné avec un benzène, un hétéroarène, un cycloalcane ou un hétérocycloalcane,
$R^4$ est un cycloalkyle, un cycloalcényle, un cycloalcynyle, un hétérocycloalkyle, un hétérocycloalcényle ou un

hétérocycloalcynyle, chacun étant non fusionné ou fusionné avec un benzène, un hétéroarène,

$R^5$ est un alkyle, un alcényle ou un alcynyle, chacun étant non substitué ou substitué avec un ou plusieurs groupes identiques ou différents sélectionnés parmi $R^8$, OH, $OR^8$, SH, $SR^8$, $S(O)R^8$, $SO_2R^8$, $C(O)R^8$, $C(O)OR^8$, $OC(O)R^8$, $NHR^8$, $N(R^8)_2$, $C(O)NHR^8$, $C(O)N(R^8)_2$, $NHC(O)R^8$, $NR^8C(O)R^8$, $NHC(O)OR^8$, $NR^8C(O)OR^8$, $NHC(O)NH_2$, $NHC(O)NHR^8$, $NHC(O)N(R^8)_2$, $NR^8C(O)NHR^8$, $NR^8C(O)N(R^8)_2$, $SO_2NHR^8$, $SO_2N(R^8)_2$, $NR^8SO_2R^8$, $NHSO_2NHR^8$, $NHSO_2N(R^8)_2$, $NR^8SO_2NHR^8$, $NR^8SO_2N(R^8)_2$, C(O)NHNOH, $C(O)NHNOR^8$, $C(O)NHSO_2R^8$, $C(NH)NH_2$, $C(NH)NHR^8$, $C(NH)N(R^{15})_2$, $NHSO_2NHR^8$, $NHSO_2N(CH_3)R^8$, $N(CH_3)SO_2N(CH_3)R^8$, F, Cl, Br, I, CN, $NO_2$, $N_3$, C(O)H, CHNOH, $CH(NOCH_3)$, $CF_3$, $CF_2CF_3$, $OCF_3$, $OCF_2CF_3$, C(O)OH, $C(O)NH_2$,

$R^8$ est $R^9$, $R^{10}$, $R^{11}$, $R^{12}$, $R^{13}$, $R^{14}$,

$R^9$ est un phényle, qui est non fusionné ou fusionné avec un benzène, un hétéroarène, un cycloalcane ou un hétérocycloalcane,

$R^{10}$ est un hétéroaryle, qui est non fusionné ou fusionné avec un benzène, un hétéroarène, un cycloalcane ou un hétérocycloalcane,

$R^{11}$ est un cycloalkyle, un cycloalcényle, un cycloalcynyle, un hétérocycloalkyle, un hétérocycloalcényle ou un hétérocycloalcynyle, chacun étant non fusionné ou fusionné avec un benzène, un hétéroarène,

$R^{12}$ est un alkyle, un alcényle ou un alcynyle chacun étant non substitué ou substitué avec un ou plusieurs groupes identiques ou différents sélectionnés parmi

$NH_2$, $NHCH_3$, $N(CH_3)_2$, SH, SMe, $C(O)NH_2$, C(O)NHOH, $CF_3$, $CF_2CF_3$, $OCF_3$, $OCF_2CF_3$, C(O)H, C(O)OH, $C(O)OC_2H_5$, OH, $OCH_3$, $OC_2H_5$, $CH_3$, $C_2H_5$, $CH(CH_3)_2$, CN, $N_3$, $NO_2$, F, Cl, Br, I,

$R^{13}$ est un phényle qui est non substitué ou substitué avec un ou plusieurs groupes identiques ou différents sélectionnés parmi

$NH_2$, $NHCH_3$, $N(CH_3)_2$, SH, SMe, $C(O)NH_2$, C(O)NHOH, $CF_3$, $CF_2CF_3$, $OCF_3$, $OCF_2CF_3$, C(O)H, C(O)OH, $C(O)OC_2H_5$, OH, $OCH_3$, $OC_2H_5$, $CH_3$, $C_2H_5$, $CH(CH_3)_2$, CN, $N_3$, $NO_2$, F, Cl, Br, I,

$R^{14}$ est un hétéroaryle, qui est non substitué ou substitué avec un ou plusieurs groupes identiques ou différents sélectionnés parmi

$NH_2$, $NHCH_3$, $N(CH_3)_2$, SH, SMe, $C(O)NH_2$, C(O)NHOH, $CF_3$, $CF_2CF_3$, $OCF_3$, $OCF_2CF_3$, C(O)H, C(O)OH, $C(O)OC_2H_5$, OH, $OCH_3$, $OC_2H_5$, $CH_3$, $C_2H_5$, $CH(CH_3)_2$, CN, $N_3$, $NO_2$, F, Cl, Br, I,

$R^6$ est un phényle qui est non substitué ou substitué avec un ou plusieurs groupes identiques ou différents sélectionnés parmi $R^{15}$, OH, $OR^{15}$, SH, $SR^{15}$, $S(O)R^{15}$, $SO_2R^{15}$, $C(O)R^{15}$, $C(O)OR^{15}$, $OC(O)R^{15}$, $NHR^{15}$, $N(R^{15})_2$, $C(O)NHR^{15}$, $C(O)N(R^{15})_2$, $NHC(O)R^{15}$, $NR^{15}C(O)R^{15}$, $NHC(O)OR^{15}$, $NR^{15}C(O)OR^{15}$, $NHC(O)NH_2$, $NHC(O)NHR^{15}$, $NHC(O)N(R^{15})_2$, $NR^{15}C(O)NHR^{15}$, $NR^{15}C(O)N(R^{15})_2$, $SO_2NHR^{15}$, $SO_2N(R^{15})_2$, $NR^{15}SO_2R^{15}$, $NHSO_2NHR^{15}$, $NHSO_2N(R^{15})_2$, $NR^{15}SO_2NHR^{15}$, $NR^{15}SO_2N(R^{15})_2$, C(O)NHNOH, $C(O)NHNOR^{15}$, $C(O)NHSO_2R^{15}$, $C(NH)NH_2$, $C(NH)NHR^{15}$, $C(NH)N(R^{15})_2$, $NHSO_2NHR^{15}$, $NHSO_2N(CH_3)R^{15}$, $N(CH_3)SO_2N(CH_3)R^{15}$, F, Cl, Br, I, CN, $NO_2$, $N_3$, C(O)H, CHNOH, $CH(NOCH_3)$, $CF_3$, $CF_2CF_3$, $OCF_3$, $OCF_2CF_3$, C(O)OH, $C(O)NH_2$,

$R^{15}$ est $R^{16}$, $R^{17}$, $R^{18}$ $R^{19}$, $R^{20}$, $R^{21}$,

$R^{16}$ est un phényle, qui est non fusionné ou fusionné avec un benzène, un hétéroarène, un cycloalcane ou un hétérocycloalcane,

$R^{17}$ est un hétéroaryle, qui est non fusionné ou fusionné avec un benzène, un hétéroarène, un cycloalcane ou un hétérocycloalcane,

$R^{18}$ est un cycloalkyle, un cycloalcényle, un cycloalcynyle, un hétérocycloalkyle, un hétérocycloalcényle ou un hétérocycloalcynyle, chacun étant non fusionné ou fusionné avec un benzène, un hétéroarène,

$R^{19}$ est un alkyle, un alcényle ou un alcynyle chacun étant non substitué ou substitué avec un ou plusieurs groupes identiques ou différents sélectionnés parmi

$NH_2$, $NHCH_3$, $N(CH_3)_2$, SH, SMe, $C(O)NH_2$, C(O)NHOH, $CF_3$, $CF_2CF_3$, $OCF_3$, $OCF_2CF_3$, C(O)H, C(O)OH, $C(O)OC_2H_5$, OH, $OCH_3$, $OC_2H_5$, $CH_3$, $C_2H_5$, $CH(CH_3)_2$, CN, $N_3$, $NO_2$, F, Cl, Br, I,

$R^{20}$ est un phényle qui est non substitué ou substitué avec un ou plusieurs groupes identiques ou différents sélectionnés parmi

$NH_2$, $NHCH_3$, $N(CH_3)_2$, SH, SMe, $C(O)NH_2$, C(O)NHOH, $CF_3$, $CF_2CF_3$, $OCF_3$, $OCF_2CF_3$, C(O)H, C(O)OH, $C(O)OC_2H_5$, OH, $OCH_3$, $OC_2H_5$, $CH_3$, $C_2H_5$, $CH(CH_3)_2$, CN, $N_3$, $NO_2$, F, Cl, Br, I,

$R^{21}$ est un hétéroaryle, qui est non substitué ou substitué avec un ou plusieurs groupes identiques ou différents sélectionnés parmi

$NH_2$, $NHCH_3$, $N(CH_3)_2$, SH, SMe, $C(O)NH_2$, C(O)NHOH, $CF_3$, $CF_2CF_3$, $OCF_3$, $OCF_2CF_3$, C(O)H, C(O)OH, $C(O)OC_2H_5$, OH, $OCH_3$, $OC_2H_5$, $CH_3$, $C_2H_5$, $CH(CH_3)_2$, CN, $N_3$, $NO_2$, F, Cl, Br, I,

$R^7$ est un hétéroaryle, qui est non substitué ou substitué avec un ou plusieurs groupes identiques ou différents sélectionnés parmi $R^{22}$, OH, $OR^{22}$, SH, $SR^{22}$, $S(O)R^{22}$, $SO_2R^{22}$, $C(O)R^{22}$, $C(O)OR^{22}$, $OC(O)R^{22}$, $NHR^{22}$, $N(R^{22})_2$, $C(O)NHR^{22}$, $C(O)N(R^{22})_2$, $NHC(O)R^{22}$, $NR^{22}C(O)R^{22}$, $NHC(O)OR^{22}$, $NR^{22}C(O)OR^{22}$, $NHC(O)NH_2$,

$NHC(O)NHR^{22}$, $NHC(O)N(R^{22})_2$, $NR^{22}C(O)NHR^{22}$, $NR^{22}C(O)N(R^{22})_2$, $SO_2NHR^{22}$, $SO_2N(R^{22})_2$, $NR^{22}SO_2R^{22}$, $NHSO_2NHR^{22}$, $NHSO_2N(R^{22})_2$, $NR^{22}SO_2NHR^{22}$, $NR^{22}SO_2N(R^{22})_2$, C(O)NHNOH, $C(O)NHNOR^{22}$, $C(O)NHSO_2R^{22}$, $C(NH)NH_2$, $C(NH)NHR^{22}$, $C(NH)N(R^{22})_2$, $NHSO_2NHR^{22}$, $NHSO_2N(CH_3)R^{22}$, $N(CH_3)SO_2N(CH_3)R^{22}$, F, Cl, Br, I, CN, $NO_2$, $N_3$, C(O)H, CHNOH, $CH(NOCH_3)$, $CF_3$, $CF_2CF_3$, $OCF_3$, $OCF_2CF_3$, C(O)OH, $C(O)NH_2$,

$R^{22}$ est $R^{23}$, $R^{24}$, $R^{25}$, $R^{26}$, $R^{27}$, $R^{28}$,

$R^{23}$ est un phényle, qui est non fusionné ou fusionné avec un benzène, un hétéroarène, un cycloalcane ou un hétérocycloalcane,

$R^{24}$ est un hétéroaryle, qui est non fusionné ou fusionné avec un benzène, un hétéroarène, un cycloalcane ou un hétérocycloalcane,

$R^{25}$ est un cycloalkyle, un cycloalcényle, un cycloalcynyle, un hétérocycloalkyle, un hétérocycloalcényle ou un hétérocycloalcynyle, chacun étant non fusionné ou fusionné avec un benzène, un hétéroarène,

$R^{26}$ est un alkyle, un alcényle ou un alcynyle, chacun étant non substitué ou substitué avec un ou plusieurs groupes identiques ou différents sélectionnés parmi

$NH_2$, $NHCH_3$, $N(CH_3)_2$, SH, SMe, $C(O)NH_2$, C(O)NHOH, $CF_3$, $CF_2CF_3$, $OCF_3$, $OCF_2CF_3$, C(O)H, C(O)OH, $C(O)OC_2H_5$, OH, $OCH_3$, $OC_2H_5$, $CH_3$, $C_2H_5$, $CH(CH_3)_2$, CN, $N_3$, $NO_2$, F, Cl, Br, I,

$R^{27}$ est un phényle qui est non substitué ou substitué avec un ou plusieurs groupes identiques ou différents sélectionnés parmi

$NH_2$, $NHCH_3$, $N(CH_3)_2$, SH, SMe, $C(O)NH_2$, C(O)NHOH, $CF_3$, $CF_2CF_3$, $OCF_3$, $OCF_2CF_3$, C(O)H, C(O)OH, $C(O)OC_2H_5$, OH, $OCH_3$, $OC_2H_5$, $CH_3$, $C_2H_5$, $CH(CH_3)_2$, CN, $N_3$, $NO_2$, F, Cl, Br, I,

$R^{28}$ est un hétéroaryle, qui est non substitué ou substitué avec un ou plusieurs groupes identiques ou différents sélectionnés parmi

$NH_2$, $NHCH_3$, $N(CH_3)_2$, SH, SMe, $C(O)NH_2$, C(O)NHOH, $CF_3$, $CF_2CF_3$, $OCF_3$, $OCF_2CF_3$, C(O)H, C(O)OH, $C(O)OC_2H_5$, OH, $OCH_3$, $OC_2H_5$, $CH_3$, $C_2H_5$, $CH(CH_3)_2$, CN, $N_3$, $NO_2$, F, Cl, Br, I,

et/ou des sels pharmaceutiquement acceptables des sulfonamides de formule générale (I),

où les composés sont des 2,3,5,6-tétrafluorobenzènesulfonamides substitués en 4, des 3,5,6-trifluoro-benzènesulfonamides disubstitués en 2,4, des 3,5,6-trifluorobenzènesulfonamides substitués en 2, des 2,5,6-trifluorobenzènesulfonamides disubstitués en 3,4, des 2,6-difluorobenzènesulfonamides trisubstitués en 3,4,5,

à condition que les composés suivants soient exclus

le 2,3,5,6-tétrafluoro-4-méthoxybenzènesulfonamide ;
le 2,3,5,6-tétrafluoro-4-pipéridin-1-ylbenzènesulfonamide ;
le 2,3,5,6-tétrafluoro-4-(méthylamino)benzènesulfonamide ;
le 4-(diméthylamino)-2,3,5,6-tétrafluorobenzènesulfonamide ;
le 4-(cyclohexylamino)-2,3,5,6-tétrafluorobenzènesulfonamide ;
le 2,3,5,6-tétrafluoro-4-(isopropylamino)benzènesulfonamide ;
le 4-(cyclopentylamino)-2,3,5,6-tétrafluorobenzènesulfonamide ;
le 2,3,5,6-tétrafluoro-4-pyrrolidin-1-ylbenzènesulfonamide ;
le 2,3,5,6-tétrafluoro-4-morpholin-4-ylbenzènesulfonamide ;
le 2,3,5,6-tétrafluoro-4-(4-méthylpipérazin-1-yl)benzènesulfonamide ;
le 2,3,5,6-tétrafluoro-4-[(2-hydroxyéthyl)amino]benzènesulfonamide ;
le 4-(butylamino)-2,3,5,6-tétrafluorobenzènesulfonamide ;
le 4-azépan-1-yl-2,3,5,6-tétrafluorobenzènesulfonamide ;
le 2-(cyclopropylamino)-3,5,6-trifluorobenzènesulfonamide ;
le 4-N-hexaméthylèneimino-2,3,5,6-tétrafluorobenzènesulfonamide.

**2.** Benzènesulfonamides fluorés selon la revendication 1, sélectionnés dans un groupe comprenant :

le 2,3,5,6-tétrafluoro-4-hydrazinobenzènesulfonamide ;
le 4-(2-benzylidènehydrazino)-2,3,5,6-tétrafluorobenzènesulfonamide ;
le 2,3,5,6-tétrafluoro-4[(2-hydroxyéthyl)thio]benzènesulfonamide ;
le 2,3,5,6-tétrafluoro-4[(2-hydroxyéthyl)sulfonyl]benzènesulfonamide ;
l'acétate de 2-{[4-(aminosulfonyl)-2,3,5,6-tétrafluorophényl]sulfonyl}éthyle ;
le 2,3,5,6-tétrafluoro-4-(propylthio)benzènesulfonamide ;
l'acide {[4-(aminosulfonyl)-2,3,5,6-tétrafluorophényl]thio}acétique ;
l'acide 3-{[4-(aminosulfonyl)-2,3,5,6-tétrafluorophényl]thio}propanoïque ;
l'acide 6-{[4-(aminosulfonyl)-2,3,5,6-tétrafluorophényl]amino}hexanoïque ;
le 2,3,5,6-tétrafluoro-4-(phénylthio)benzènesulfonamide ;
le 2,3,5,6-tétrafluoro-4-(phénylsulfonyl)benzènesulfonamide ;

le 2,3,5,6-tétrafluoro-4-phénoxybenzènesulfonamide ;
le 4-(benzylthio)-2,3,5,6-tétrafluorobenzènesulfonamide ;
le 4-(benzylamino)-2,3,5,6-tétrafluorobenzènesulfonamide ;
le 2,3,5,6-tétrafluoro-4-{[2-(4-hydroxyphényl)éthyl]amino}benzènesulfonamide ;
le 2,3,5,6-tétrafluoro-4-[(2-phényléthyl)thio]benzènesulfonamide ;
le 2,3,5,6-tétrafluoro-4-[(2-phényléthyl)sulfonyl]benzènesulfonamide ;
le 2,3,5,6-tétrafluoro-4-morpholin-4-ylbenzènesulfonamide ;
le 2,3,5,6-tétrafluoro-4-[(mésitylméthyl)thio]benzènesulfonamide ;
le 4-[(4,6-diméthylpyrimidin-2-yl)thio]-2,3,5,6-tétrafluorobenzènesulfonamide ;
le 4-(1,3-benzothiazol-2-ylthio)-2,3,5,6-tétrafluorobenzènesulfonamide ;
le 4-(1-adamantylamino)-2,3,5,6-tétrafluorobenzènesulfonamide ;
le 3-{[4-(aminosulfonyl)-2,3,5,6-tétrafluorophényl]thio}-[1,2,3]thiadiazolo[3,4-a] benzimidazole ;
le 4-[(4,5-diphényl-1*H*-imidazol-2-yl)thio]-2,3,5,6-tétrafluorobenzènesulfonamide ;
le 2-(isopropylamino)-3,5,6-trifluoro-4-[(2-phényléthyl)thio]benzènesulfonamide ;
le 2-(benzylamino)-3,5,6-trifluoro-4-[(2-phényléthyl)thio]benzènesulfonamide ;
le 2-[(2-phényléthyl)amino]-3,5,6-trifluoro-4-[(2-phényléthyl)thio]benzènesulfonamide ;
le 2-[(1-phényléthyl)amino]-3,5,6-trifluoro-4-[(2-phényléthyl)thio]benzènesulfonamide ;
le 2-morpholin-4-yl-3,5,6-trifluoro-4-[(2-phényléthyl)thio]benzènesulfonamide ;
le 2-(cyclohexylamino)-3,5,6-trifluoro-4-[(2-phényléthyl)thio]benzènesulfonamide ;
le 2-(cycloheptylamino)-3,5,6-trifluoro-4-[(2-phényléthyl)thio]benzènesulfonamide ;
le 2-(cyclooctylamino)-3,5,6-trifluoro-4-[(2-phényléthyl)thio]benzènesulfonamide ;
le 2-(cyclododécylamino)-3,5,6-trifluoro-4-[(2-phényléthyl)thio]benzènesulfonamide ;
le 2-[(2,6-diméthoxybenzyl)amino]-3,5,6-trifluoro-4-[(2-phényléthyl)thio]benzènesulfonamide ;
le 2-[(3,4-diméthoxybenzyl)amino]-3,5,6-trifluoro-4-[(2-phényléthyl)thio]benzènesulfonamide ;
le 2-(2,3-dihydro-1*H*-indèn-2-ylamino)-3,5,6-trifluoro-4-[(2-phényléthyl)thio]-benzènesulfonamide ;
le 2-[(1S)-2,3-dihydro-1*H*-indèn-1-ylamino]-3,5,6-trifluoro-4-[(2-phényléthyl)thio]-benzènesulfonamide ;
le 2-[(1S)-1,2,3,4-tétrahydronaphtalèn-1-ylamino]-3,5,6-trifluoro-4-[(2-phényléthyl)thio]benzènesulfonamide ;
le 2-{[(1S,2R)-2-hydroxy-1,2-diphényléthyl]amino}-3,5,6-trifluoro-4-[(2-phényléthyl)thio]benzènesulfonamide ;
le 2-(cyclooctylamino)-3,5,6-trifluoro-4-[(2-hydroxyéthyl)thio]benzènesulfonamide ;
le 2-(cyclododécylamino)-3,5,6-trifluoro-4-[(2-hydroxyéthyl)thio]benzènesulfonamide ;
le 2-[(2,6-diméthoxybenzyl)amino]-3,5,6-trifluoro-4-[(2-hydroxyéthyl)thio]-benzènesulfonamide ;
le 2-[(3,4-diméthoxybenzyl)amino]-3,5,6-trifluoro-4-[(2-hydroxyéthyl)thio]-benzènesulfonamide ;
le 2-[(1S)-2,3-dihydro-1*H*-indèn-1-ylamino]-3,5,6-trifluoro-4-[(2-hydroxyéthyl)-thio]benzènesulfonamide ;
le 2-[(1S)-1,2,3,4-tétrahydronaphtalèn-1-ylamino)-3,5,6-trifluoro-4-[(2-hydroxyéthyl)thio]benzènesulfonamide ;
le 2-{[(1S,2R)-2-hydroxy-1,2-diphényléthyl]amino}-3,5,6-trifluoro-4-[(2-hydroxyéthyl)thio]benzènesulfonamide ;
le 2-(cyclooctylamino)-3,5,6-trifluoro-4-(propylthio)benzènesulfonamide ;
le 2-(cyclooctylamino)-3,5,6-trifluoro-4-{[2-(4-hydroxyphényl)éthyl]amino}-benzènesulfonamide ;
le 2-(cyclooctylamino)-3,5,6-trifluorobenzènesulfonamide ;
le 2-(cyclododécylamino)-3,5,6-trifluorobenzènesulfonamide ;
le 2-[(2,6-diméthoxybenzyl)amino]-3,5,6-trifluorobenzènesulfonamide ;
le 2-[(3,4-diméthoxybenzyl)amino]-3,5,6-trifluorobenzènesulfonamide ;
le 2-[(1S)-2,3-dihydro-1*H*-indèn-1-ylamino]-3,5,6-trifluorobenzènesulfonamide ;
le 2-[(1S)-1,2,3,4-tétrahydronaphtalèn-1-ylamino)-3,5,6-trifluorobenzènesulfonamide ;
le 3-(méthylamino)-2,5,6-trifluoro-4-[(2-phényléthyl)sulfonyl]benzènesulfonamide ;
le 3-(*tert*-butylamino)-2,5,6-trifluoro-4-[(2-phényléthyl)sulfonyl]benzènesulfonamide ;
le 3-(benzylamino)-2,5,6-trifluoro-4-[(2-phényléthyl)sulfonyl]benzènesulfonamide ;
le 3-[(2-phényléthyl)amino]-2,5,6-trifluoro-4-[(2-phényléthyl)sulfonyl]benzènesulfonamide ;
le 3-morpholin-4-yl-2,5,6-trifluoro-4-[(2-phényléthyl)sulfonyl]benzènesulfonamide ;
le 3-(cyclooctylamino)-2,5,6-trifluoro-4-[(2-phényléthyl)sulfonyl]benzènesulfonamide ;
le 3-(cyclododécylamino)-2,5,6-trifluoro-4-[(2-phényléthyl)sulfonyl]benzènesulfonamide ;
le 3-[(2,6-diméthoxybenzyl)amino]-2,5,6-trifluoro-4-[(2-phényléthyl)sulfonyl]-benzènesulfonamide ;
le 3-[(3,4-diméthoxybenzyl)amino]-2,5,6-trifluoro-4-[(2-phényléthyl)sulfonyl]-benzènesulfonamide ;
le 3-(1-adamantylamino)-2,5,6-trifluoro-4-[(2-phényléthyl)sulfonyl]benzène-sulfonamide ;
le 3-(2,3-dihydro-1*H*-indèn-2-ylamino)-2,5,6-trifluoro-4-[(2-phényléthyl)sulfonyl]-benzènesulfonamide ;
le 3-[(1S)-2,3-dihydro-1*H*-indèn-1-ylamino]-2,5,6-trifluoro-4-[(2-phényléthyl)-sulfonyl]benzènesulfonamide ;
le 3-[(1S)-1,2,3,4-tétrahydronaphtalèn-1-ylamino)-2,5,6-trifluoro-4-[(2-phényléthyl)sulfonyl]benzènesulfonamide ;

le 3-{[(1S,2R)-2-hydroxy-1,2-diphényléthyl]amino}-2,5,6-trifluoro-4-[(2-phényléthyl)sulfonyl]benzènesulfonamide ;
le 3-{[(1R,2S)-2-hydroxy-1,2-diphényléthyl]amino}-2,5,6-trifluoro-4-[(2-phényléthyl)sulfonyl]benzènesulfonamide ;
le 3-(benzylamino)-2,5,6-trifluoro-4-[(2-hydroxyéthyl)sulfonyl]benzènesulfonamide ;
le 3-(cyclooctylamino)-2,5,6-trifluoro-4-[(2-hydroxyéthyl)sulfonyl]benzènesulfonamide ;
le 3-(cyclododécylamino)-2,5,6-trifluoro-4-[(2-hydroxyéthyl)sulfonyl]benzènesulfonamide ;
le 3-[(2,6-diméthoxybenzyl)amino]-2,5,6-trifluoro-4-[(2-hydroxyéthyl)sulfonyl]-benzènesulfonamide ;
le 3-[(3,4-diméthoxybenzyl)amino]-2,5,6-trifluoro-4-[(2-hydroxyéthyl)sulfonyl]-benzènesulfonamide ;
le 3-[(1S)-2,3-dihydro-1*H*-indèn-1-ylamino]-2,5,6-trifluoro-4-[(2-hydroxyéthyl) sulfonyl]benzènesulfonamide ;
le 3-[(1S)-1,2,3,4-tétrahydronaphtalèn-1-ylamino]-2,5,6-trifluoro-4-[(2-hydroxyéthyl)sulfonyl]benzènesulfonamide ;
le 3-{[(1S,2R)-2-hydroxy-1,2-diphényléthyl]amino}-2,5,6-trifluoro-4-[(2-hydroxyéthyl)sulfonyl]benzènesulfonamide ;
le 3,5-bis(cyclooctylamino)-2,6-difluoro-4-[(2-phényléthyl)sulfonyl]benzènesulfonamide ;
le 3,5-bis[(3,4-diméthoxybenzyl)amino]-2,6-difluoro-4-[(2-hydroxyéthyl)sulfonyl]-benzènesulfonamide.

**3.** Composition pour une utilisation dans la maîtrise d'affections où une inhibition d'anhydrase carbonique est nécessaire, **caractérisée en ce qu'**elle contient une quantité efficace de sulfonamide selon l'une quelconque des revendications 1 à 2 et **caractérisée en ce que** les affections sont sélectionnées parmi l'hypertension intraoculaire (glaucome), la maladie de la haute altitude, les céphalées, la migraine, les troubles neurologiques, l'obésité et un cancer, incluant les signes et/ou les symptômes associés auxdites maladies.

**4.** Composition selon la revendication 3 pour une utilisation dans un procédé de traitement de troubles médiés par des isoformes d'anhydrase carbonique sélectionnés parmi l'hypertension intraoculaire (glaucome), la maladie de la haute altitude, les céphalées, la migraine, les troubles neurologiques, l'obésité et un cancer, incluant les signes et/ou les symptômes associés auxdites maladies.

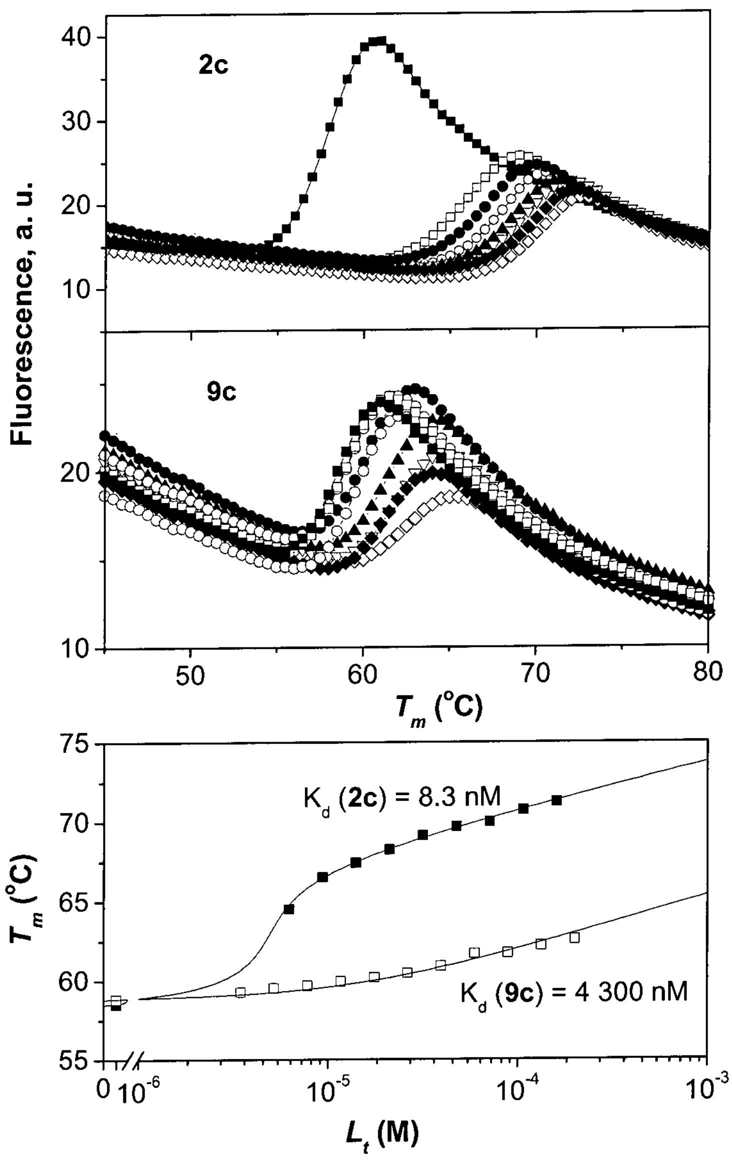
2c
9c
Fluorescence, a. u.
40
30
20
10
20
10
50
60
70
80
$T_m$ (°C)
75
70
65
60
55
$T_m$ (°C)
$K_d$ (2c) = 8.3 nM
$K_d$ (9c) = 4 300 nM
0
$10^{-6}$
$10^{-5}$
$10^{-4}$
$10^{-3}$
$L_t$ (M)

Fig. 1

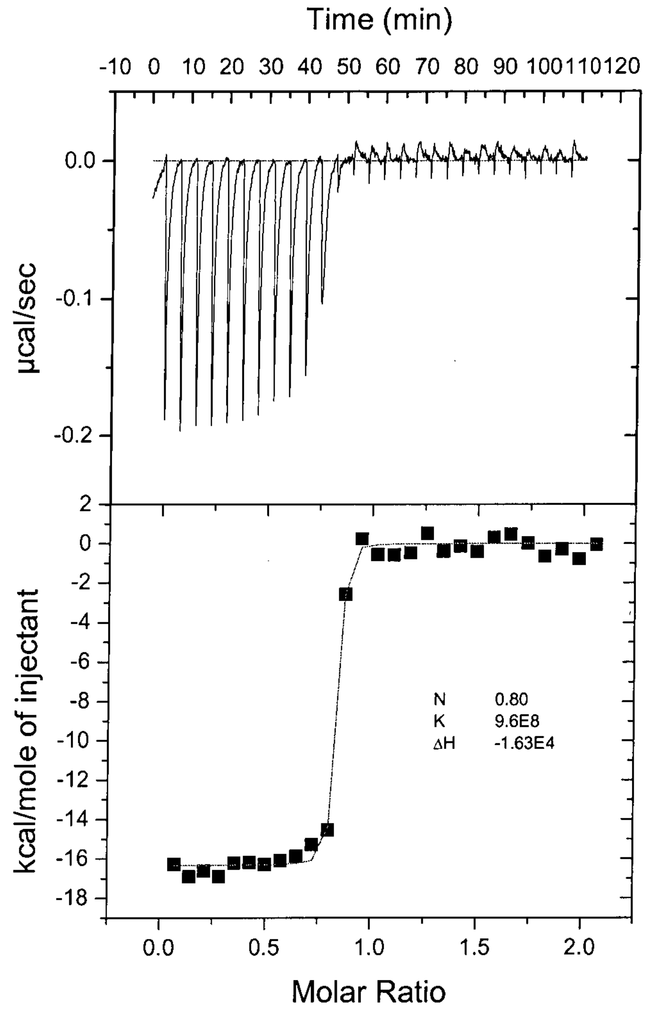
Time (min)
-10 0 10 20 30 40 50 60 70 80 90 100 110 120
µcal/sec
0.0
-0.1
-0.2
kcal/mole of injectant
2
0
-2
-4
-6
-8
-10
-12
-14
-16
-18
N 0.80
K 9.6E8
ΔH -1.63E4
0.0 0.5 1.0 1.5 2.0
Molar Ratio

**Fig. 2**

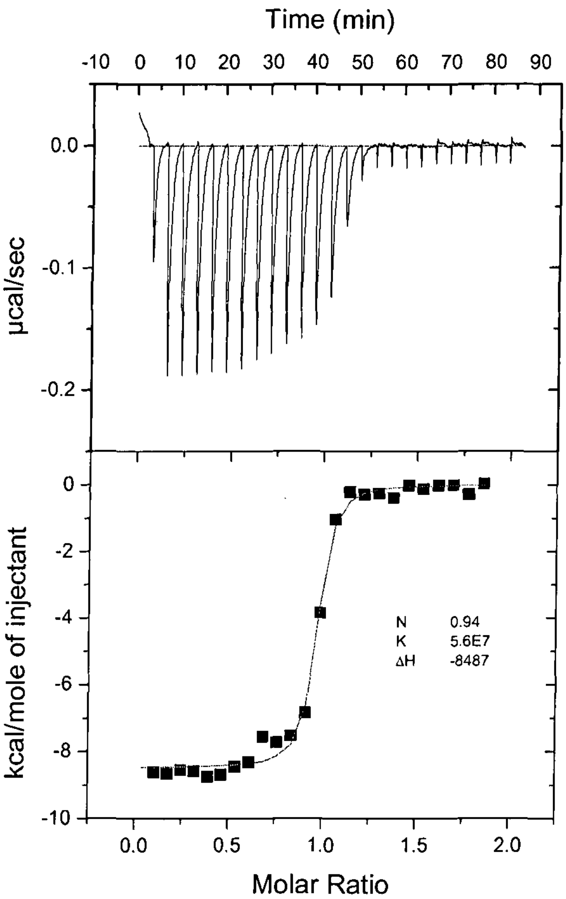
Time (min)
-10 0 10 20 30 40 50 60 70 80 90
µcal/sec
0.0
-0.1
-0.2
kcal/mole of injectant
0
-2
-4
-6
-8
-10
N 0.94
K 5.6E7
ΔH -8487
0.0 0.5 1.0 1.5 2.0
Molar Ratio

**Fig. 3**

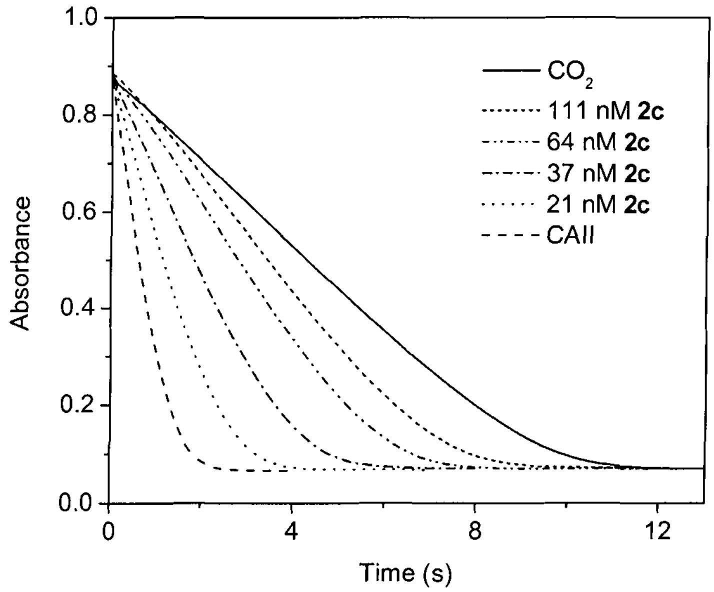
Absorbance
1.0
0.8
0.6
0.4
0.2
0.0
0 4 8 12
Time (s)
CO2
111 nM 2c
64 nM 2c
37 nM 2c
21 nM 2c
CAII

**Fig. 4**

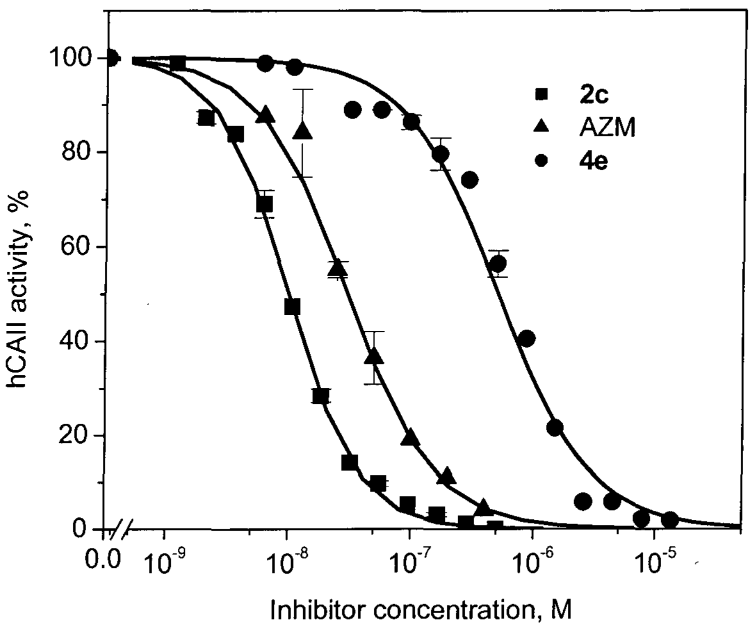
100
80
60
40
20
0
hCAII activity, %
0.0
$10^{-9}$
$10^{-8}$
$10^{-7}$
$10^{-6}$
$10^{-5}$
Inhibitor concentration, M
2c
AZM
4e

Fig. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2004048544 A
- WO 2005107470 A
- GB 1031082 A
- BE 659230
- GB 1025314 A
- BE 664831
- WO 2010004139 A
- WO 2004011460 A
- WO 2008017932 A
- US 20090163586 A
- WO 2009118292 A
- WO 2007110337 A
- WO 2006099972 A
- US 20080146637 A
- WO 2004054974 A
- CN 101418001
- US 20110124628 A
- WO 2010093845 A
- WO 2011071565 A
- WO 2011029842 A
- WO 2008017381 A

### Non-patent literature cited in the description


- **TEMPERINI, C. et al.** Drug Design of Zinc-Enzyme Inhibitors: Functional, Structural, and Disease Applications. Wiley, 2009, 473
- **GAO, B.B. et al.** *Nat. Med.,* 2007, vol. 13, 181
- **BASNYAT, B. et al.** *High Alt. Med. Biol.,* 2003, vol. 4, 45
- **HEN, N. et al.** *J. Med. Chem.,* 2011, vol. 54, 3977
- **DE SIMONE, G. et al.** *Curr. Pharm. Des.,* 2008, vol. 14, 655
- **GULER, O. O. et al.** *Curr. Med. Chem.,* 2010, vol. 17, 1516
- **SUPURAN, C. T. et al.** *Eur. J. Med. Chem.,* 2000, vol. 35, 867
- **GULER, O.O. et al.** *Curr. Med. Chem.,* 2010, vol. 17, 1516
- **DE SIMONE, G. et al.** *Biochim. Biophys. Acta,* 2010, vol. 1804, 404
- **BATTKE, C. et al.** *Cancer Immunol. Immunother,* 2011, vol. 60, 649
- **LEHTONEN, I. et al.** *J. Biol. Chem.,* 2004, vol. 279, 2791
- **MERLIN, C. et al.** *J. Bacteriol.,* 2003, vol. 185, 6415
- **PASTOREKOVA, S. et al.** *J. Enzyme Inhib. Med. Chem.,* 2004, vol. 19, 199
- **OLANDER, J. et al.** *JACS,* 1973, vol. 95, 1616
- **KRISHNAMURTHY, V. M. et al.** *Chem. Asian J.,* 2007, vol. 2, 94
- **BRADSHAW, L. R. A.** *Journal of Chromatography,* 1969, vol. 44, 422
- **ROBSON, P. et al.** *J. Chem. Soc.,* 1963, 3692
- **VERNIER, W.** *Biorg. Med. Chem.,* 2010, vol. 18, 3307
- **TULLIO, P.** *J. Med. Chem.,* 2005, vol. 48, 4990
- **KREBS, J. F. ; FIERKE, C. A.** *J. Biol. Chem.,* 1993, vol. 268, 948
- **CHAKRAVARTY, S. ; KANNAN, K. K.** *J. Mol. Biol.,* 1994, vol. 243, 298
- **LINDSKOG, S.** *Pharmacol. Ther.,* 1997, vol. 74, 1
- **BAIRD, T. T. J. et al.** *Biochemistry,* 1997, vol. 36, 2669
- **ALTERIO, V. et al.** *Chem. Rev.,* 2012, vol. 112, 4421
- **KRISHNAMURTHY, V. M. et al.** *Chem. Rev.,* 2008, vol. 108, 946
- **MYSZKA, D. G. et al.** *J. Biomol. Tech.,* 2003, vol. 14, 247
- **MATULIS, D. ; TODD, M. J.** *Biocalorimetry,* 2004, vol. 2
- **ČAPKAUSKAITĖ, E. et al.** *Eur. J. Med. Chem.,* 2012, vol. 51, 259
- **KAZLAUSKAS, E. et al.** *PLoS ONE,* 2012, vol. 7, e36899